(19) Europäisches Patentamt

European Patent Office

Office européen des brevets

(11) **EP 1 688 439 A1**

(12) **EUROPEAN PATENT APPLICATION**
published in accordance with Art. 158(3) EPC

(43) Date of publication:
09.08.2006 Bulletin 2006/32

(51) Int Cl.:
*C07K 19/00* (2006.01)   *C07K 16/00* (2006.01)
*C12N 15/62* (2006.01)   *C12N 5/10* (2006.01)
*C12P 21/08* (2006.01)   *A61K 38/17* (2006.01)
*A61P 35/00* (2006.01)   *A61P 37/00* (2006.01)
*A61P 29/00* (2006.01)

(21) Application number: 04773776.2

(22) Date of filing: 08.10.2004

(86) International application number:
PCT/JP2004/015325

(87) International publication number:
WO 2005/035586 (21.04.2005 Gazette 2005/16)

(84) Designated Contracting States:
AT BE BG CH CY CZ DE DK EE ES FI FR GB GR
HU IE IT LI LU MC NL PL PT RO SE SI SK TR

(30) Priority: 08.10.2003 JP 2003350158

(71) Applicant: Kyowa Hakko Kogyo Co., Ltd
Tokyo 100-8185 (JP)

(72) Inventors:
• SHITARA, Kenya,
c/o Kyowa Hakko Kogyo Co.,Ltd.
Tokyo 100-8185 (JP)
• HOSAKA, Emi,
c/o Kyowa Hakko Kogyo Co.,Ltd.
Tokyo 100-8185 (JP)
• NATSUME, Akito,
c/o Kyowa Hakko Kogyo Co.,Ltd.
Tokyo 100-8185 (JP)

• WAKITANI, Masako,
c/o Kyowa Hakko Kogyo Co.,Ltd.
Tokyo 100-8185 (JP)
• UCHIDA, Kazuhisa,
c/o Kyowa Hakko Kogyo Co.,Ltd.
Tokyo 100-8185 (JP)
• SATO, Mitsuo,
c/o Kyowa Hakko Kogyo Co.,Ltd.
Tokyo 100-8185 (JP)
• OHNUKI, Naoko,
c/o Kyowa Hakko Kogyo Co.,Ltd.
Tokyo 100-8185 (JP)
• NAKAMURA, Kazuyasu,
c/o Kyowa Hakko Kogyo Co.,Ltd.
Tokyo 100-8185 (JP)

(74) Representative: Vossius & Partner
Siebertstrasse 4
81675 München (DE)

(54) **FUSED PROTEIN COMPOSITION**

(57)    A fusion protein composition of an antibody Fc region which is useful as a medicament in which effector function is improved is desired.

The present invention provides a fusion protein composition comprising a fusion protein molecule of an antibody Fc region having complex type N-glycoside-linked sugar chains in the Fc region, wherein the complex type N-glycoside-linked sugar chains have a structure in which fucose is not bound to N-acetylglucosamine in the reducing end in the sugar chains; a transformant producing the fusion protein composition; a process for producing the fusion protein composition; and a medicament comprising the fusion protein composition.

EP 1 688 439 A1

**Description**

<u>Technical Field</u>

The present invention relates to a fusion protein composition comprising a fusion protein molecule of a binding protein and an antibody Fc region having complex type N-glycoside-linked sugar chains, wherein the complex type N-glycoside-linked sugar chains have a structure in which fucose is not bound to N-acetylglucosamine in the reducing end in the sugar chains; a transformant producing the composition; a process for producing the composition; and a medicament comprising the composition..

<u>Background Art</u>

An antibody induces a cytotoxic activity of an effector cell such as a natural killer cell or activated macrophage (antibody-dependent cell-mediated cytotoxicity; hereinafter referred to as "ADCC activity"), by specifically binding with its antigen and via a cell membrane expression type Fc receptor which is a receptor specific for the Fc region and expressed in the effector cell.

Regarding the antibody, in recent years, in the treatment of non-Hodgkin's lymphoma patients by Rituxan and the treatment of breast cancer patients by Herceptin, when the therapeutic antibody induces high antibody-dependent cell-mediated cytotoxicity (hereinafter referred to as "ADCC activity") in effector cells of the patients, higher therapeutic effects can be obtained [*Blood,* 99, 754 (2002); *J. Clin. Oncol.,* 21, 3940 (2003); *Clin. Cancer Res.,* 10, 5650 (2004)].

A variable region of the antibody is a domain related to the specific binding with its antigen. On the other hand, a constant region of the antibody is a domain which carry the stabilization of the antibody molecule and effector function of the antibody. Particularly, an antibody Fc region of IgG class (a region in and after the hinge region of antibody heavy chain) shows ADCC activity via FcγIIIa which is a member of the Fc receptors on effector cells. Accordingly, similar to the case of antibody molecules, a fusion protein comprising a protein molecule having binding activity with a specific molecule (hereinafter referred to as "binding protein") can bind with the specific molecule and also have ADCC activity via the Fc region.

Until now, fusion proteins of various binding proteins with Fc region have been prepared , and their ADCC activity has been examined [*Proc. Natl. Acad. Sci. USA,* 90, 7995 (1993)]. For example, it is known that, as a molecule having a similar formation with an antibody, an Fc fusion protein of a single-chain antibody (hereinafter referred to as "scFv") which is a protein molecule containing antibody heavy chain variable region (hereinafter referred to as "VH") and light chain variable region (hereinafter referred to as "VL") has the ADCC activity. For example, an Fc fusion protein with scFv (hereinafter referred to as "scFv-Fc") [*J. Immunol. Methods,* 261, 199 (2002)] prepared from a mouse monoclonal antibody for TAG-72 (tumor-associated glycoprotein-72) (hereinafter referred to as "anti-TAG-72 antibody") known as a cancer cell surface antigen has the ADCC activity for TAG-72-positive cells. In addition, an scFv-Fc obtained by carrying out panning of a phage library of antibodies prepared from melanoma patients, for melanoma cell, showed an ADCC activity specific for the melanoma cell [*Proc. Natl. Acad Sci. USA,* 96, 1627 (1999)].

The scFv to be fused is not limited to one. A molecule in which another scFv is linked to the N-terminal of scFv-Fc is called scFv$_2$-Fc and it has both of two kinds of binding specificities due to specificity of the antibody as the origin of scFv. In such molecule, it is known that its binding activity to the antigen is decreased [*Mol. Immunol.,* 37, 1123 (2000)], but its ADCC activity has not been confirmed. In recent years, improvement of effect of therapeutic agent by simultaneous action upon several kinds of target molecules has been considered promising, such as the case of co-use therapy of medicaments, and a bispecific antibody which specifically binds to two kinds of target molecules is known also in the field of medicaments comprising antibody. Since such a bispecific antibody, which targets two kinds of molecules by one preparation, additive effect and synergistic effect of the effects of therapeutic agent are expected.

In addition, a cytokine, a chemokine, an apoptosis induced signal molecule, a co-stimulation molecule, a growth factor, a differentiation inducing factor and the like are known as binding proteins other than scFv. Fusion proteins of receptors of the binding proteins with Fc have been reported in large numbers, and their examples include Etanercept (USP 5605690) which is a fusion protein of a soluble type tumor necrosis factor-α receptor II (hereinafter referred to as "sTNFR II") with Fc, Alefacept (USP 5914111) which is a fusion protein of lymphocyte function-associated antigen 3 expressed on antigen presenting cells (hereinafter referred to as "LFA-3") with Fc, a fusion protein with cytotoxic T lymphocyte-associated antigen-4 (CTLA-4) with Fc [*J. Exp. Med.,* 181, 1869 (1995)], a fusion protein of interleukin-15 with antibody Fc [*J. Immunol.,* 160, 5742 (1998)], a fusion protein of factor VII with Fc [*Proc. Natl. Acad Sci. USA,* 98, 12180 (2001)], a fusion protein of interleukin-10 with Fc [*J. Immunol.,* 154, 5590 (1995)], a fusion protein of interleukin-2 with Fc [*J. Immunol.,* 146, 915 (1991)], a fusion protein of CD40 with Fc [*Surgery,* 132, 149 (1002)], a fusion protein of 0X40 with Fc [*J. Leu. Biol.,* 72, 522 (2002)] and the like. Among them, Etanercept and Alefacept are already used as medicines. However, the binding activity of a fusion protein to its target molecule is generally low in comparison with the binding activity of an antibody to its antigen [*Proc. Natl. Acad. Sci. USA,* 90, 7995 (1993), *J. Pharmacol. Exp. Ther.,*

301, 418 (2002)], so that fusion proteins which can be used in the authentic forms as medicines are limited.

The ADCC activity is induced via the interaction between Fc region of a human IgG1 subclass antibody and Fcγ receptor (hereinafter referred to as "FcγR"). Regarding the binding of an antibody with FcγR, importance of a sugar chain linking to the hinge region and the second domain of C region (hereinafter referred to as "Cγ2 domain") of the antibody is suggested [*Chemical* Immunology, 65, 88 (1997)].

It is known that there is diversity regarding the addition of galactose to the non-reducing end in a complex type N-glycoside-linked sugar chain bound to the Fc region of an IgG antibody molecule and the addition of fucose to N-acetylglucosamine at its reducing end [*Biochemistry,* 36, 130 (1997)]. In particular, it is reported that the addition of fucose to the N-acetylglucosamine at the reducing end in the sugar chain causes significant decrease of the ADCC activity of the antibody [WO00/61739, *J. Biol. Chem.,* 278, 3466 (2003)].

The fusion protein is prepared by recombinant DNA techniques using animal cells such as Chinese hamster ovary tissue-derived CHO cells as host cells, and it is considered that the sugar chain structure of the expressed fusion protein differs depending upon host cells.

For example, it is known that it is possible to increase the ratio of sugar chains having a structure in which fucose is not bound to N-acetylglucosamine in the reducing end in the complex type N-glycoside-linked sugar chains bound to the Fc region of antibody molecules in an antibody composition by decreasing or deleting the activity of α1,6-fucosyltransferase (FUT8), GDP-mannose 4,6-dehydratase (GMD) or GDP-4-keto-6-deoxy-D-mannose 3,5-epimerase (Fx) of antibody-producing cells (WO02/31140).

## Disclosure of the Invention

An object of the present invention is to provide a fusion protein composition comprising a fusion protein of a binding protein and an antibody Fc region having complex type N-glycoside-linked sugar chains, wherein the complex type N-glycoside-linked sugar chains have a structure in which fucose is not bound to N-acetylglucosamine in the reducing end in the sugar chains; a transformant producing the fusion protein composition; a process for producing the fusion protein composition; a medicament comprising the fusion protein composition; and the like. Since the fusion protein of the present invention has a high cytotoxicity, it is useful in a treatment to decrease the number of cells which are targets of the treatment from the patient's body. By using the fusion protein having high cytotoxicity in a treatment, combined use with chemotherapy, a radioisotope label and the like becomes unnecessary, so that it is expected to decrease side effects on patients. In addition, alleviation of burden on a patient can be expected by decreasing the dose of a therapeutic agent to the patient.

The present invention relates to the following (1) to (39).

(1) A pharmaceutical fusion protein composition comprising a fusion protein molecule of a binding protein and an antibody Fc region having complex type N-glycoside-linked sugar chains, wherein the complex type N-glycoside-linked sugar chains have a structure in which fucose is not bound to N-acetylglucosamine in the reducing end in the sugar chains.

(2) The fusion protein composition according to (1), wherein the complex type N-glycoside-linked sugar chains are sugar chains in which 1-position of fucose is not bound to 6-position of N-acetylglucosamine in the reducing end through α-bond in the sugar chains.

(3) The fusion protein composition according to (1) or (2), wherein the antibody Fc region is an IgG class of a human antibody.

(4) The fusion protein composition according to (3), wherein the antibody Fc region is an IgG1 class of a human antibody.

(5) The fusion protein composition according to (4), wherein the antibody fusion protein composition comprises an IgG1 class heavy chain constant region domain 2 (CH$_2$) of a human antibody.

(6) The fusion protein composition according to (5), wherein the fusion protein composition comprises a hinge region, a heavy chain constant region domain 2 (CH$_2$) and a heavy chain constant region domain 3 (CH$_3$) of a human IgG1 class antibody.

(7) The fusion protein composition according to any one of (1) to (6), wherein the binding protein comprises at least one protein selected from the group consisting of a binding fragment of an antibody, a soluble receptor and a ligand protein.

(8) The fusion protein composition according to (7), wherein the binding fragment of an antibody comprises at least one polypeptide comprising an antibody heavy chain variable region (VH) and an antibody light chain variable region (VL).

(9) The fusion protein composition according to (8), wherein the polypeptide comprising an antibody heavy chain variable region (VH) and an antibody light chain variable region (VL) is a single-chain antibody.

(10) The fusion protein composition according to (7), wherein the binding fragment of an antibody is a single-chain antibody.

(11) The fusion protein composition according to (7), wherein the binding fragment of an antibody comprises a polypeptide comprising two kinds of antibody heavy chain variable regions (VH) and two kinds of antibody light chain variable regions (VL).

(12) The fusion protein composition according to (11), wherein the polypeptide comprising antibody heavy chain variable regions (VH) and light chain variable regions (VL) is a single-chain antibody.

(13) The fusion protein composition according to (7), wherein the binding fragment of an antibody is a bispecific single-chain antibody.

(14) The fusion protein composition according to (7), wherein the soluble receptor is a soluble TNF (tumor necrosis-factor) receptor II.

(15) The fusion protein composition according to (15), wherein the soluble receptor comprises the amino acid sequence represented by SEQ ID NO:64.

(16) The fusion protein composition according to (14) or (15), wherein the fusion protein is produced by FERM BP-8499.

(17) The fusion protein composition according to (7), wherein the ligand protein is LFA-3 (leukocyte function antigen-3).

(18) The fusion protein composition according to (16), wherein the ligand protein comprises the amino acid sequence represented by SEQ ID NO:65.

(19) The fusion protein composition according to (17) or (18), wherein the fusion protein is produced by FERM BP-8500.

(20) The fusion protein composition according to any one of (1) to (19), wherein the fusion protein composition is a dimer.

(21) A transformant obtainable by introducing a DNA encoding the fusion protein according to any one of (1) to (20) into a host cell.

(22) The transformant according to (21), wherein the host cell is a cell in which a genome is modified so that an enzyme relating to synthesis of an intracellular sugar nucleotide, GDP-fucose or an enzyme relating to a modification of a sugar chain in which 1-position of fucose is bound to 6-position of N-acetylglucosamine in the reducing end through α-bond in the complex type N-glycoside-linked sugar chain is inactivated.

(23) The transformant according to (22), wherein the host cell is a cell in which all of alleles on a genome encoding an enzyme relating to synthesis of an intracellular sugar nucleotide, GDP-fucose or an enzyme relating to a modification of a sugar chain in which 1-position of fucose is bound to 6-position of N-acetylglucosamine in the reducing end through α-bond in the complex type N-glycoside-linked sugar chain are knocked out.

(24) The transformant according to (22) or (23), wherein the enzyme relating to synthesis of an intracellular sugar nucleotide, GDP-fucose, is an enzyme selected from the group consisting of GDP-mannose 4,6-dehydratase (GMD) and GDP-4-keto-6-deoxy-D-mannose 3,5-epimerase (Fx).

(25) The transformant according to (24), wherein the GDP-mannose 4,6-dehydratase is a protein encoded by a DNA selected from the following (a) or (b):

(a) a DNA comprising the nucleotide sequence represented by SEQ ID NO: 1;
(b) a DNA which hybridizes with the DNA consisting of the nucleotide sequence represented by SEQ ID NO:1 under stringent conditions and which encodes a protein having GDP-mannose 4,6-dehydratase activity.

(26) The transformant according to (24), wherein the GDP-mannose 4,6-dehydratase is a protein selected from the group consisting of the following (a), (b) and (c):

(a) a protein comprising the amino acid sequence represented by SEQ ID NO:2;
(b) a protein consisting of an amino acid sequence wherein one or more amino acid(s) is/are deleted, substituted, inserted and/or added in the amino acid sequence represented by SEQ ID NO:2 and having GDP-mannose 4,6-dehydratase activity;
(c) a protein consisting of an amino acid sequence which has 80% or more homology to the amino acid sequence represented by SEQ ID NO:2 and having GDP-mannose 4,6-dehydratase activity.

(27) The transformant according to (24), wherein the GDP-4-keto-6-deoxy-D-mannose 3,5-epimerase is a protein encoded by a DNA selected from the following (a) or (b):

(a) a DNA comprising the nucleotide sequence represented by SEQ ID NO:3;
(b) a DNA which hybridizes with a DNA consisting of the nucleotide sequence represented by SEQ ID NO:3 under stringent conditions and which encodes a protein having GDP-4-keto-6-deoxy-D-mannose 3,5-epimerase activity.

(28) The transformant according to (24), wherein the GDP-4-keto-6-deoxy-D-mannose 3,5-epimerase is a protein selected from the group consisting of the following (a) to (c):

(a) a protein comprising the amino acid sequence represented by SEQ ID NO:4;
(b) a protein consisting of an amino acid sequence wherein one or more amino acid(s) is/are deleted, substituted, inserted and/or added in the amino acid sequence represented by SEQ ID NO:4 and having GDP-4-keto-6-deoxy-D-mannose 3,5-epimerase activity;
(c) a protein consisting of an amino acid sequence which has 80% or more homology to the amino acid sequence represented by SEQ ID NO:4 and having GDP-4-keto-6-deoxy-D-mannose 3,5-epimerase activity.

(29) The transformant according to (22) or (23), wherein the enzyme relating to a modification of a sugar chain in which 1-position of fucose is bound to 6-position of N-acetylglucosamine in the reducing end through α-bond in the complex type N-glycoside-linked sugar chain is α1,6-fucosyltransferase.
(30) The transformant according to (29), wherein the α1,6-fucosyltransferase is a protein encoded by a DNA selected from the group consisting of the following (a) to (d):

(a) a DNA comprising the nucleotide sequence represented by SEQ ID NO:5;
(b) a DNA comprising the nucleotide sequence represented by SEQ ID NO:6;
(c) a DNA which hybridizes with a DNA consisting of the nucleotide sequence represented by SEQ ID NO:5 under stringent conditions and which encodes a protein having α1,6-fucosyltransferase activity;
(d) a DNA which hybridizes with a DNA consisting of the nucleotide sequence represented by SEQ ID NO:6 under stringent conditions and which encodes a protein having α1,6-fucosyltransferase activity.

(31) The transformant according to (29), wherein the α1,6-fucosyltransferase is a protein selected from the group consisting of the following (a) to (f):

(a) a protein comprising the amino acid sequence represented by SEQ ID NO:7;
(b) a protein comprising the amino acid sequence represented by SEQ ID NO:8;
(c) a protein consisting of an amino acid sequence wherein one or more amino acid(s) is/are deleted, substituted, inserted and/or added in the amino acid sequence represented by SEQ ID NO:7 and having α1,6-fucosyltransferase activity;
(d) a protein consisting of an amino acid sequence wherein one or more amino acid(s) is/are deleted, substituted, inserted and/or added in the amino acid sequence represented by SEQ ID NO:8 and having α1,6-fucosyltransferase activity;
(e) a protein consisting of an amino acid sequence which has 80% or more homology to the amino acid sequence represented by SEQ ID NO:7 and having α1,6-fucosyltransferase activity;
(f) a protein consisting of an amino acid sequence which has 80% or more homology to the amino acid sequence represented by SEQ ID NO:8 and having α1,6-fucosyltransferase activity.

(32) The transformant according to any one of (21) to (31), wherein the host cell is a cell selected from the group consisting of the following (a) to (h):

(a) a CHO cell derived from Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-Ag14 cell;
(e) a BHK cell derived from Syrian hamster kidney tissue;
(f) a human leukemia cell line Namalwa cell;
(g) an embryonic stem cell;
(h) a fertilized egg cell.

(33) The transformant according to any one of (21) to (32), wherein the transformant is FERM BP-8499.
(34) The transformant according to any one of (21) to (32), wherein the transformant is FERM BP-8500.
(35) A process for producing the fusion protein composition according to any one of (1) to (20), which comprises culturing the transformant according to any one of (21) to (34) in a medium to form and accumulate the fusion protein composition in the culture, and recovering and purifying the antibody composition from the culture.

(36) The antibody fusion protein composition according to any one of (1) to (20), which is obtainable by the process according to (35).

(37) A medicament comprising the fusion protein composition according to any one of (1) to (20) and (36) as an active ingredient.

(38) An agent for preventing or treating tumor, inflammatory diseases or autoimmune diseases, comprising the fusion protein composition as an active ingredient according to any one of (1) to (20) and (36).

(39) The agent for preventing or treating the diseases according to the above (38), wherein the tumor is blood tumor or cancer.

The present invention is described below in detail. This application is based on the priority of Japanese patent application No. 2003-350158 filed on October 8, 2003, and the contents of the specification and the drawings in the patent application are incorporated hereinto.

The fusion protein composition comprising a binding protein and an antibody Fc region having complex type N-glycoside-linked sugar chains, wherein the complex type N-glycoside-linked sugar chains have a structure in which fucose is not bound to N-acetylglucosamine in the reducing end in the sugar chains according to the present invention includes a fusion protein composition comprising a binding protein and an antibody Fc region having complex type N-glycoside-linked sugar chains, wherein the complex type N-glycoside-linked sugar chains are sugar chains in which 1-position of fucose is not bound to 6-position of N-acetylglucosamine in the reducing end through α-bond in the sugar chains.

The antibody Fc region in the present invention may be an Fc region derived from an antibody of an antibody subclass having antibody effector activity. In view of utilization as pharmaceuticals, it is preferably an antibody Fc region derived from a human IgG class, and more preferably an antibody Fc region derived from a human IgG1 class. The antibody Fc region derived from an IgG class antibody includes a polypeptide chain comprising heavy chain constant region domain 2 (hereinafter referred to as "$CH_2$") and domain 3 (hereinafter referred to as "$CH_3$").

The antibody effector activity includes antibody-dependent cell-mediated cytotoxic activity (hereinafter referred to as "ADCC activity"), complement-dependent cytotoxic activity (hereinafter referred to as "CDC activity") and the like. The ADCC activity is a cytotoxic activity expressed by binding an antibody Fc region to an Fcγ receptor which is an antibody Fc receptor. The CDC activity is a cytotoxic activity expressed by binding an antibody Fc region to a competent component.

The antibody Fc region is bound to N-glycoside-linked sugar chains. Therefore, one sugar chain is bound to one polypeptide chain of the Fc fusion protein.

The N-glycoside-linked sugar chains include complex type sugar chains having one or several of parallel galactose-N-acetylglucosamine (hereinafter referred to as Gal-GlcNAc) side chains in the non-reducing end of the core structure and having sialic acid, bisecting N-acetylglucosamine or the like in the non-reducing end of Gal-GlcNAc.

In the present invention, the complex type N-glycoside-linked sugar chain is represented by the following chemical formula 1.

## Chemical formula 1:

In the present invention, the sugar chain to which fucose is not bound includes a sugar chain represented by the above chemical formula in which fucose is not bound to N-acetylglucosamine in the reducing end. The sugar chain in the non-reducing end may have any structure.

Accordingly, the antibody composition of the present invention comprises an antibody molecule having the same sugar chain structure or antibody molecules having different sugar chain structures, so long as the antibody composition has the above sugar chain structure.

The expression "fucose is not bound to the N-acetylglucosamine in the reducing end in the sugar chains" as used herein means that fucose is not substantially bound thereto. The "fusion protein composition in which fucose is not substantially bound" specifically refers to a fusion protein composition in which fucose is not substantially detected, i.e., the content of fucose is below the detection limit, when subjected to the sugar chain analysis described in 4 below. The fusion protein composition of the present invention in which fucose is not bound to the N-acetylglucosamine in the reducing end in the sugar chains has high ADCC activity.

The ratio of a fusion protein molecule having sugar chains in which fucose is not bound to the N-acetylglucosamine in the reducing end in a fusion protein composition comprising a fusion protein molecule having complex type N-glycoside-linked sugar chains in the Fc region can be determined by releasing the sugar chains from the fusion protein molecule by known methods such as hydrazinolysis and enzyme digestion [*Seibutsukagaku Jikkenho (Biochemical Experimentation Methods) 23 - Totanpakushitsu Tosa Kenkyuho (Methods of Studies on Glycoprotein Sugar Chains)*, Gakkai Shuppan Center, edited by Reiko Takahashi (1989)], labeling the released sugar chains with a fluorescent substance or radioisotope, and separating the labeled sugar chains by chromatography. Alternatively, the released sugar chains may be analyzed by the HPAED-PAD method [*J. Liq. Chromatogr.,* 6, 1577 (1983)] to determine the ratio.

As the binding protein used in the present invention, a protein capable of specifically binding to a specific intravital substance can be cited. The specific intravital substance includes intravital substances such as proteinous macromolecules, sugar chains and cell membrane constituting components, which are expressed disease-specifically on the surface of lesion cells of a disease. Specifically, as the receptors which are expressed in various tumor cells, gangliosides, membrane anchor type-antibodies, membrane anchor type enzymes and the like can be exemplified.

The ability of a binding protein to specifically bind means that the binding protein can bind to a specific substance, and means that 1 kind of binding protein binds to generally several kinds of substances, preferably to 2 or 3 kinds of substances, more preferably 1 kind of substance.

As the binding protein, it may contain a region which binds to a specific intravital substance in the living body, and it may be a partial region or whole of the binding protein. In addition, two regions or more of the same binding protein can also fused with the antibody Fc region.

Furthermore, in the present invention, the same or different binding proteins can also be fused with the antibody Fc region, and the number of the binding proteins to be fused may be one or more.

As the binding protein of the present invention, a binding fragment of an antibody, a ligand of a receptor, a soluble receptor, a nucleic acid binding protein, a protein which binds to a cell membrane, a protein which binds to a lipid, a protein which binds to a fatty acid binding protein, a protein which binds to a sugar or sugar chain, a dominant negative form of an enzyme substrate, a dominant negative form of an enzyme and the like can be specifically exemplified. Among them, an antibody, a binding fragment of an antibody, a ligand of a receptor, a soluble receptor and the like are preferably used.

As the antibody to be used in the present invention, a monoclonal antibody which binds to a lesion cell of a disease or a intravital substance expressing on the surface of a lesion cell of a disease or on the surface of the lesion cells can be cited. The monoclonal antibody can be obtained by the known method described in *Antibodies: A Laboratory Manual,* Cold Spring Harbor Laboratory, Chapter 14 (1998), by a method in which an antibody producing cell prepared from a living body is made into an established cell line through its immortalization, and by a phage display method in which an antibody and an antibody gene are selected by panning from an artificial antibody phage library.

In the present invention, a binding fragment of an antibody can be used as the binding protein. The binding fragment of an antibody can be prepared from the above-described monoclonal antibody by a method in which it is digested using a digestive enzyme, or by protein engineering techniques in which a gene encoding the monoclonal antibody is prepared from a hybridoma cell which produces the antibody.

The binding fragment of an antibody includes a binding fragment which comprises a part or a whole of the variable region of an antibody and retains binding activity to the antigen corresponding to the antibody. If necessary, the binding fragment which contains a part of the variable region of an antibody can be designed in such a manner that it can be expressed in a desired form, and an appropriate amino acid sequence can also be inserted for the purpose of connecting with the antibody Fc region. In the case of an antibody derived from a non-human animal, it is possible to lower immunogenicity by substituting the amino acid sequence of a framework with a human antibody-derived sequence. The binding fragment of the antibody includes Fab, F(ab')$_2$, Fab', scFv (single-chain antibody), scFv multimer, diabody, dsFv, a peptide comprising CDR and the like.

An Fab fragment is one of the fragments obtained by treatment of IgG with the protease, papain (cleavage at amino acid residue 224 of H chain). It is an antibody fragment with a molecular weight of approximately 50,000 having antigen-binding activity and composed of the N-terminal half of H chain and the whole of L chain linked by a disulfide bond.

The Fab fragment can be obtained by treating the antibody molecule with the protease, papain. Alternatively, the Fab fragment may be produced by inserting DNA encoding the Fab fragment of the antibody molecule into an expression vector for prokaryote or eukaryote, and introducing the vector into a prokaryote or eukaryote to induce expression.

An F(ab')$_2$ fragment is one of the fragments obtained by treatment of IgG with the proteolytic enzyme, pepsin (cleavage at amino acid residue 234 of H chain). It is an antibody fragment with a molecular weight of approximately 100,000 having antigen-binding activity, which is slightly larger than the Fab fragments linked together by a disulfide bond at the hinge region.

The F(ab')$_2$ fragment can be obtained by treating the antibody molecule with the protease, pepsin. Alternatively, the F(ab')$_2$ fragment may be prepared by binding Fab' fragments described below by a thioether bond or a disulfide bond.

An Fab' fragment is an antibody fragment with a molecular weight of approximately 50,000 having antigen-binding activity, which is obtained by cleaving the disulfide bond at the hinge region of the above F(ab')$_2$ fragment.

The Fab' fragment can be obtained by treating the F(ab')$_2$ fragment with a reducing agent, dithiothreitol. Alternatively, the Fab' fragment may be produced by inserting DNA encoding the Fab' fragment of the antibody into an expression vector for prokaryote or eukaryote, and introducing the vector into a prokaryote or eukaryote to induce expression.

An scFv fragment is a polypeptide linked in the order of VH-P-VL or VL-P-VH in which one VH and one VL are linked via an appropriate peptide linker (hereinafter referred to as P) and which has antigen-binding activity.

The scFv fragment can be produced by obtaining cDNAs encoding the VH and VL of the antibody molecule, constructing DNA encoding the scFv fragment, inserting the DNA into an expression vector for prokaryote or eukaryote, and introducing the expression vector into a prokaryote or eukaryote to induce expression.

A multimer of scFv is a polypeptide chains linked as scFv-(P-scFv-)$_N$ using plural scFv and an appropriate peptide linker, and has binding activity against respective antigen corresponding to each of scFv. Examples include scFv$_2$ (bispecific single-chain antibody) comprising two kinds of scFv in the same polypeptide chain and the like. A combination of respective scFv contained in the multimer of scFv may be any combination, such as a combination of plural scFv which are the same and a combination of several kinds of scFv.

The multimer of scFv can be produced by protein engineering techniques similar to the above scFv fragment.

A diabody is an antibody fragment which is a dimer of scFv showing bivalent antigen binding activity, which may be either monospecific or bispecific.

The diabody can be produced by obtaining cDNAs encoding the VH and VL of the antibody molecule, constructing DNA encoding scFv fragments with P having an amino acid sequence of 12 or less amino acid residues, inserting the DNA into an expression vector for prokaryote or eukaryote, and introducing the expression vector into a prokaryote or eukaryote to induce expression.

A dsFv fragment is an antibody fragment wherein polypeptides in which one amino acid residue of each of VH and VL is substituted with a cysteine residue are linked by a disulfide bond between the cysteine residues. The amino acid residue to be substituted with a cysteine residue can be selected based on antibody tertiary structure prediction according to the method proposed by Reiter, *et al.* [*Protein Engineering, 7*, 697 (1994)].

The dsFv fragment of the present invention can be produced by obtaining cDNAs encoding the VH and VL of the antibody molecule, constructing DNA encoding the dsFv fragment, inserting the DNA into an expression vector for prokaryote or eukaryote, and introducing the expression vector into a prokaryote or eukaryote to induce expression.

A peptide comprising CDR comprises one or more region CDR of VH or VL. A peptide comprising plural CDRs can be prepared by binding CDRs directly or via an appropriate peptide linker.

The peptide comprising CDR can be produced by constructing DNA encoding CDR of VH and VL of the antibody molecule, inserting the DNA into an expression vector for prokaryote or eukaryote, and introducing the expression vector into a prokaryote or eukaryote to induce expression.

The peptide comprising CDR can also be produced by chemical synthesis methods such as the Fmoc method (fluorenylmethyloxycarbonyl method) and the tBoc method (t-butyloxycarbonyl method).

The binding fragment of an antibody used in the fusion protein composition of the present invention is preferably scFv. The scFv may be scFv derived from one kind of an antibody or scFv$_2$ which is obtained by expressing two kinds of scFv derived from two kinds of antibodies as one polypeptide chain and has two kinds of binding specificities in one polypeptide chain. The scFv may be any scFv, so long as it is produced by any antibody, and examples include scFv comprising CDR1, CDR2 and CDR3 of antibody VH consisting of the amino acid sequences represented by SEQ ID NOs:9,10 and 11, respectively, and CDR1, CDR2 and CDR3 of antibody VL consisting of the amino acid sequences represented by SEQ ID NOs:12, 13 and 14, respectively, of a mouse monoclonal antibody against TAC-72 which is known to be a surface antigen of a cancer cell; scFv comprising antibody VH consisting of the amino acid sequence represented by SEQ ID NO: 15 and antibody VL consisting of the amino acid sequence represented by SEQ ID NO:16; scFv comprising the amino acid sequence represented by SEQ ID NO:17; and the like. Furthermore, examples include scFv comprising CDR1, CDR2 and CDR3 of antibody VH consisting of the amino acid sequences represented by SEQ ID NOs:66, 67 and 68, respectively, and CDR1, CDR2 and CDR3 of antibody VL consisting of the amino acid sequences represented by SEQ ID NOs:69, 70 and 71, respectively, of a mouse monoclonal antibody against MUC1 which is known to be a surface antigen of a cancer cell; scFv comprising antibody VH consisting of the amino acid sequence represented by SEQ ID NO:72 and antibody VL consisting of the amino acid sequence represented by SEQ ID NO:73; scFv comprising the amino acid sequence represented by SEQ ID NO:74; and the like.

Any scFv$_2$ may be used, so long as it is a combination of any scFv. The scFv may be the same or different. Examples include scFv$_2$ consisting of scFv comprising CDR1, CDR2 and CDR3 of antibody VH consisting of the amino acid sequences represented by SEQ ID NOs:9, 10 and 11, respectively, and CDR1, CDR2 and CDR3 of antibody VL consisting of the amino acid sequences represented by SEQ ID NOs:12, 13 and 14, respectively; scFv$_2$ consisting of scFv comprising CDR1, CDR2 and CDR3 of antibody VH consisting of the amino acid sequences represented by SEQ ID NOs:66, 67 and 68, respectively, and CDR1, CDR2 and CDR3 of antibody VL consisting of the amino acid sequences represented by SEQ ID NOs:69, 70 and 71, respectively; scFv$_2$ consisting of scFv comprising CDR1, CDR2 and CDR3 of antibody VH consisting of the amino acid sequences represented by SEQ ID NOs:9, 10 and 11, respectively, and CDR1, CDR2 and CDR3 of antibody VL consisting of the amino acid sequences represented by SEQ ID NOs:12, 13 and 14, respectively, and scFv comprising CDR1, CDR2 and CDR3 of antibody VH consisting of the amino acid sequences represented by SEQ ID NOs:66, 67 and 68, respectively, and CDR1, CDR2 and CDR3 of antibody VL consisting of the amino acid sequences represented by SEQ ID NOs:69, 70 and 71, respectively; scFv$_2$ consisting of scFv comprising antibody VH consisting of the amino acid sequence represented by SEQ ID NO: 15 and antibody VL consisting of the amino acid sequence represented by SEQ ID NO:16; scFv$_2$ consisting of scFv comprising antibody VH consisting of the amino acid sequence represented by SEQ ID NO:72 and antibody VL consisting of the amino acid sequence represented by SEQ ID NO:73; scFv$_2$ consisting of scFv comprising antibody VH consisting of the amino acid sequence represented by SEQ ID NO:15 and antibody VL consisting of the amino acid sequence represented by SEQ ID NO:16, and scFv$_2$ consisting of scFv comprising antibody VH consisting of the amino acid sequence represented by SEQ ID NO:72 and antibody VL consisting of the amino acid sequence represented by SEQ ID NO:73; scFv$_2$ consisting of the amino acid sequence represented by SEQ ID NO:75; scFv$_2$ consisting of the amino acid sequence represented by SEQ ID NO:76; and the like.

In the present invention, the soluble receptor may be any substance, so long as it is a receptor which can bind to a ligand expressing on the cell surface, and a receptor which can prepare such a ligand binding region of receptor by a protein engineering technique in the form of retaining the binding activity for the ligand, and the like are also included therein. Examples include a soluble form TNF (tumor necrosis factor) II receptor, a soluble receptor comprising the amino acid sequence represented by SEQ ID NO:64 and the like.

In the present invention, the ligand protein of receptor includes a ligand protein of a receptor expressing on the cell surface in the human body. The ligand protein may have influence upon the activity of the corresponding specific receptor, so long as it can bind to the receptor. For example, a signal transduction pathway in which the receptor is related may be activated by the binding of the ligand protein, the signal transduction may be inactivated, or it may have no influence upon the signal transduction system. The ligand in which a signal transduction mediating to the receptor is activated by the binding of the ligand includes a wild type ligand, a peptide having agonist activity and the like. The ligand protein which inactivates a receptor-mediated signal transduction by the binding of the ligand includes a dominant negative form of a wild type ligand, a peptide having antagonist activity and the like.

Specifically, the ligand of the receptor of the present invention includes LFA-3 (leukocyte function antigen-3), a ligand protein comprising the amino acid sequence represented by SEQ ID NO:65 and the like.

The antibody Fc region of the present invention may contain at least one antibody heavy chain constant region domain 2 (hereinafter referred to as "CH$_2$") which is directly related in its binding with the FcγIIIa receptor.

The fusion protein composition of the present invention includes, for example, the following (a) to (f). The fusion protein composition of the present invention may form a monomer, a homo-dimer and a hetero-dimer. In the following, it is preferable that CH$_2$ and antibody heavy chain constant region domain 3 (hereinafter referred to as "CH$_3$") belong to a human IgG1 class.

(a) binding protein - CH$_2$;
(b) binding protein - CH$_2$ - CH$_3$;
(c) CH$_2$ - binding protein;
(d) CH$_2$ - CH$_3$ - binding protein;
(e) binding protein - CH$_2$ - binding protein; and
(f) binding protein - CH$_2$ - CH$_3$ - binding protein.

In the above-described linked polypeptide chains of (a) to (f), the binding protein may comprise one or more binding proteins. In addition, in the case of a fusion protein in which two binding protein polypeptide chains are contained in one polypeptide chain, the intravital substance to which the two binding proteins can be bound may be the same or different.

In the present invention, the fusion protein may be those in which respective proteins of the above (a) to (f) are bound as the elements. In this case, respective elements may be the same or different or may be a repetition of the same order. The above respective elements (a) to (f) may be linked directly or may be linked via a linker such as a hinge sequence derived from antibody constant region. In addition, one or more amino acids may be added, deleted and/or substituted in the amino acid residues of respective elements, in such a degree that the binding specificity of the binding protein and the effector activity of the antibody Fc region are not caused to change.

The transformant of the present invention includes any transformant which is obtained by introducing a DNA encoding a fusion protein molecule into a host cell and which produces the fusion protein composition of the present invention. Examples of such transformants include those obtained by introducing DNA encoding a fusion protein molecule into host cells such as the following (a) or (b):

(a) a cell in which genome is modified so as to have deleted activity of an enzyme relating to the synthesis of an intracellular sugar nucleotide, GDP-fucose;
(b) a cell in which genome is modified so as to have deleted activity of an enzyme relating to the modification of a sugar chain in which 1-position of fucose is bound to 6-position of N-acetylglucosamine in the reducing end through α-bond in a complex type N-glycoside-linked sugar chain.

Specifically, the "modification of genome so as to have deleted activity of an enzyme" refers to introduction of mutation into an expression regulation region of a gene encoding the enzyme so as to delete the expression of the enzyme or introduction of mutation in the amino acid sequence of a gene encoding the enzyme so as to inactivate the enzyme. The "introduction of mutation" refers to carrying out modification of the nucleotide sequence on the genome such as deletion, substitution, insertion and/or addition in the nucleotide sequence. Complete inhibition of the expression or activity of the thus modified genomic gene is referred to as " knock out of the genomic gene".

Examples of the enzymes relating to the synthesis of the intracellular sugar nucleotide GDP-fucose include GDP-mannose 4,6-dehydratase (GMD), GDP-4-keto-6-deoxy-D-mannose 3,5-epimerase (Fx) and the like.

Examples of the GDP-mannose 4,6-dehydratase include proteins encoded by the DNAs of the following (a) and (b):

(a) a DNA consisting of the nucleotide sequence represented by SEQ ID NO: 1;
(b) a DNA which hybridizes with DNA consisting of the nucleotide sequence represented by SEQ ID NO: 1 under stringent conditions and which encodes a protein having GDP-mannose 4,6-dehydratase activity.

Examples of the GDP-mannose 4,6-dehydratase also include proteins of the following (a) to (c):

(a) a protein consisting of the amino acid sequence represented by SEQ ID NO:2;
(b) a protein consisting of an amino acid sequence wherein one or more amino acid residue(s) is/are deleted, substituted, inserted and/or added in the amino acid sequence represented by SEQ ID NO:2 and having GDP-mannose 4,6-dehydratase activity;
(c) a protein consisting of an amino acid sequence which has 80% or more homology to the amino acid sequence represented by SEQ ID NO:2 and having GDP-mannose 4,6-dehydratase activity.

Examples of the GDP-4-keto-6-deoxy-D-mannose 3,5-epimerase include proteins encoded by the DNAs of the following (a) and (b):

(a) a DNA consisting of the nucleotide sequence represented by SEQ ID NO:3;
(b) a DNA which hybridizes with DNA consisting of the nucleotide sequence represented by SEQ ID NO:3 under stringent conditions and which encodes a protein having GDP-4-keto-6-deoxy-D-mannose 3,5-epimerase activity.

Examples of the GDP-4-keto-6-deoxy-D-mannose 3,5-epimerase also include proteins of the following (a) to (c):

(a) a protein consisting of the amino acid sequence represented by SEQ ID NO:4;
(b) a protein consisting of an amino acid sequence wherein one or more amino acid residue(s) is/are deleted, substituted, inserted and/or added in the amino acid sequence represented by SEQ ID NO:4 and having GDP-4-keto-6-deoxy-D-mannose 3,5-epimerase activity;
(c) a protein consisting of an amino acid sequence which has 80% or more homology to the amino acid sequence represented by SEQ ID NO:4 and having GDP-4-keto-6-deoxy-D-mannose 3,5-epimerase activity.

An example of the enzyme relating to the modification of a sugar chain in which 1-position of fucose is bound to 6-position of N-acetylglucosamine in the reducing end through α-bond in a complex type N-glycoside-linked sugar chain includes α1,6-fucosyltransferase.

In the present invention, examples of the α1,6-fucosyltransferase include proteins encoded by the DNAs of the following (a) to (d):

(a) a DNA comprising the nucleotide sequence represented by SEQ ID NO:5;

(b) a DNA comprising the nucleotide sequence represented by SEQ ID NO:6;

(c) a DNA which hybridizes with DNA consisting of the nucleotide sequence represented by SEQ ID NO:5 under stringent conditions and which encodes a protein having an $\alpha$1,6-fucosyltransferase activity;

(d) a DNA which hybridizes with DNA consisting of the nucleotide sequence represented by SEQ ID NO:6 under stringent conditions and which encodes a protein having an $\alpha$1,6-fucosyltransferase activity, or

(e) a protein comprising the amino acid sequence represented by SEQ ID NO:7;

(f) a protein comprising the amino acid sequence represented by SEQ ID NO:8;

(g) a protein consisting of an amino acid sequence wherein one or more amino acid residue(s) is/are deleted, substituted, inserted and/or added in the amino acid sequence represented by SEQ ID NO:7 and having an $\alpha$1,6-fucosyltransferase activity;

(h) a protein consisting of an amino acid sequence wherein one or more amino acid residue(s) is/are deleted, substituted, inserted and/or added in the amino acid sequence represented by SEQ ID NO:8 and having an $\alpha$1,6-fucosyltransferase activity;

(i) a protein consisting of an amino acid sequence which has 80% or more homology to the amino acid sequence represented by SEQ ID NO:7 and having an $\alpha$1,6-fucosyltransferase activity;

(j) a protein consisting of an amino acid sequence which has 80% or more homology to the amino acid sequence represented by SEQ ID NO:8 and having an $\alpha$1,6-fucosyltransferase activity.

The DNAs encoding the amino acid sequences of the enzymes relating to the synthesis of an intracellular sugar nucleotide, GDP-fucose include a DNA comprising the nucleotide sequence represented by SEQ ID NO: 1 or 3, and DNA which hybridizes with a DNA consisting of the nucleotide sequence represented by SEQ ID NO:1 or 3 under stringent conditions and which encodes a protein having the enzyme activity relating to the synthesis of an intracellular sugar nucleotide, GDP-fucose.

The DNAs encoding the amino acid sequences of $\alpha$1,6-fucosyltransferase include a DNA comprising the nucleotide sequence represented by SEQ ID NO:5 or 6, and a DNA which hybridizes with DNA consisting of the nucleotide sequence represented by SEQ ID NO:5 or 6 under stringent conditions and which encodes a protein having $\alpha$1,6-fucosyltransferase activity.

In the present invention, the DNA which hybridizes under stringent conditions refers to a DNA which is obtained by colony hybridization, plaque hybridization, Southern hybridization or the like using, for example, a DNA consisting of the nucleotide sequence represented by SEQ ID NO:1, 3, 5 or 6 or a fragment thereof as a probe. A specific example of such DNA is a DNA which can be identified by performing hybridization at 65°C in the presence of 0.7 to 1.0 M sodium chloride using a filter with colony- or plaque-derived DNA immobilized thereon, and then washing the filter at 65°C with a 0.1 to 2-fold concentration SSC solution (1-fold concentration SSC solution: 150 mM sodium chloride and 15 mM sodium citrate). Hybridization can be carried out according to the methods described in *Molecular Cloning, A Laboratory Manual,* Second Edition, Cold Spring Harbor Laboratory Press (1989) (hereinafter referred to as *Molecular Cloning,* Second Edition); *Current Protocols in Molecular Biology,* John Wiley & Sons (1987-1997) (hereinafter referred to as *Current Protocols in Molecular Biology*); *DNA Cloning 1: Core Techniques, A Practical Approach,* Second Edition, Oxford University (1995), *etc.* Specifically, the DNA capable of hybridization under stringent conditions includes DNA having at least 60% or more homology, preferably 70% or more homology, more preferably 80% or more homology, further preferably 90% or more homology, particularly preferably 95% or more homology, most preferably 98% or more homology to the nucleotide sequence represented by SEQ ID NO: 1, 3, 5 or 6.

In the present invention, the protein consisting of an amino acid sequence wherein one or more amino acid residue(s) is/are deleted, substituted, inserted and/or added in the amino acid sequence represented by SEQ ID NO:2 or 4 and having the activity of an enzyme relating to the synthesis of an intracellular sugar nucleotide, GDP-fucose, or the protein consisting of an amino acid sequence wherein one or more amino acid residue(s) is/are deleted, substituted, inserted and/or added in the amino acid sequence represented by SEQ ID NO:7 or 8 and having $\alpha$1,6-fucosyltransferase activity can be obtained, for example, by introducing a site-directed mutation into DNA having the nucleotide sequence represented by SEQ ID NO:1, 3, 5 or 6 by site-directed mutagenesis described in *Molecular Cloning,* Second Edition; *Current Protocols in Molecular Biology; Nucleic Acids Research,* 10, 6487 (1982); *Proc. Natl. Acad. Sci. USA,* 79, 6409 (1982); *Gene,* 34, 315 (1985); *Nucleic Acids Research,* 13, 4431 (1985); *Proc. Natl. Acad. Sci. USA,* 82, 488 (1985), *etc.* The number of amino acid residues which are deleted, substituted, inserted and/or added is one or more, and is not specifically limited, but it is within the range where deletion, substitution or addition is possible by known methods such as the above site-directed mutagenesis. The suitable number is 1 to dozens, preferably 1 to 20, more preferably 1 to 10, further preferably 1 to 5.

The protein consisting of an amino acid sequence which has 80% or more homology to the amino acid sequence represented by SEQ ID NO:2, 4, 7 or 8 and having GDP-mannose 4,6-dehydratase activity, GDP-4-keto-6-deoxy-D-mannose 3,5-epimerase activity or $\alpha$1,6-fucosyltransferase activity includes a protein having at least 80% or more homology, preferably 85% or more homology, more preferably 90% or more homology, further preferably 95% or more homology, particularly preferably 97% or more homology, most preferably 99% or more homology to the amino acid sequence represented by SEQ ID NO:2, 4, 7 or 8, respectively, as calculated by using analysis software such as BLAST [*J. Mol. Biol.,* 215, 403 (1990)] or FASTA [*Methods in Enzymology,* 183, 63 (1990)].

The host cell used in the present invention, that is, the host cell in which the activity of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is deleted may be obtained by any technique capable of deleting the above enzyme activity. For example, the following techniques can be employed for deleting the above enzyme activity:

(a) gene disruption targeting at a gene encoding the enzyme;
(b) introduction of a dominant-negative mutant of a gene encoding the enzyme;
(c) introduction of a mutation into the enzyme;
(d) inhibition of transcription or translation of a gene encoding the enzyme;
(e) selection of a cell line resistant to a lectin which recognizes a sugar chain structure in which 1-position of fucose is bound to 6-position ofN-acetylglucosamine in the reducing end through α-bond in a complex type N-glycoside-linked sugar chain.

As the lectin which recognizes a sugar chain structure in which 1-position of fucose is bound to 6-position of N-acetylglucosamine in the reducing end through α-bond in a complex type N-glycoside-linked sugar chain, any lectin capable of recognizing the sugar chain structure can be used. Specific examples include lentil lectin LCA (lentil agglutinin derived from *Lens culinaris*), pea lectin PSA (pea lectin derived from *Pisum sativum),* broad bean lectin VFA (agglutinin derived from *Vicia faba*), *Aleuria aurantia* lectin AAL (lectin derived from *Aleuria aurantia*) and the like.

The "cell resistant to a lectin" refers to a cell in which growth is not inhibited by the presence of a lectin at an effective concentration. The "effective concentration" is a concentration higher than the concentration which does not allow the normal growth of a cell before the genome modification (hereinafter also referred to as parent cell line), preferably equal to the concentration which does not allow the normal growth of a cell before the genome modification, more preferably 2 to 5 times, further preferably 10 times, and most preferably 20 or more times the concentration which does not allow the normal growth of a cell before the modification of the genomic gene.

The effective concentration of lectin that does not inhibit growth may be appropriately determined according to each cell line. It is usually 10 μg/ml to 10 mg/ml, preferably 0.5 mg/ml to 2.0 mg/ml.

The host cell for producing the fusion protein composition of the present invention may be any of the above host cells capable of expressing the fusion protein molecule of the present invention. For example, yeast cells, animal cells, insect cells, plant cells and the like can be used. Examples of the cells include those described in 1 below. Specifically, preferred among animal cells are CHO cell derived from Chinese hamster ovary tissue, rat myeloma cell line YB2/3HL.P2.G11.16Ag.20, mouse myeloma cell line NS0, mouse myeloma cell line SP2/0-Ag14, BHK cell derived from Syrian hamster kidney tissue, human leukemia cell line Namalwa, an embryonic stem cell, a fertilized egg cell, and the like

Examples of the transformant of the present invention include a transformant KC1200 of Chinese hamster ovary tissue-derived CHO cell line CHO/DG44 into which a gene encoding a fusion protein capable of binding to TAG (tumor-associated glycoprotein)-72 of the present invention, a transformant KC1194 derived from Chinese hamster ovary tissue-derived CHO cell line CHO/DG44 into which a gene encoding a fusion protein of a soluble TNF (tumore necrosis factor) receptor II, and a transformant KC1198 derived from Chinese hamster ovary tissue-derived CHO cell line CHO/DG44 into which a gene encoding a fusion protein of LFA-3 (leukocyte function antigen-3). Also, regarding the transformants derived from CHO cell line CHO/DG44, KC1194 and KC1198 were deposited with International Patent Organism Depositary, National Institute of Advanced Industrial Science and Technology, Central 6, 1, Higashi 1-chome, Tsukuba-shi, Ibaraki, Japan, on September 30, 2003 with accession No. FERM BP-8499 and FERM 8500, respectively, and KC1200 was deposited with the same on October 3, 2003 with accession No. FERM BP-8503.

Described below are the method for preparing a cell producing the fusion protein composition of the present invention, the method for producing the fusion protein composition of the present invention, and the method for analyzing and the method for utilizing the fusion protein composition of the present invention.

1. Preparation of a host cell producing the fusion protein composition of the present invention

The cell producing the fusion protein of the present invention (hereinafter referred to as the cell of the present invention) can be prepared by preparing a host cell used for the production of the fusion protein composition of the present invention by the following techniques and then introducing a gene encoding the fusion protein into the host cell by the method described in 2 below.

**(1) Gene disruption method targeting at a gene encoding an enzyme**

The host cell used for the production of the cell of the present invention can be prepared by a gene disruption method targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain. Examples of the enzymes relating to the synthesis of an intracellular sugar nucleotide, GDP-fucose include GDP-mannose 4,6-dehydratase (hereinafter referred to as "GMD"), GDP-4-keto-6-deoxy-D-mannose 3,5-epimerase (hereinafter referred to as "Fx"), and the like. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase, α-L-fucosidase and the like.

The gene as used herein includes DNA and RNA.

The method of gene disruption may be any method capable of disrupting the gene encoding the target enzyme. Useful methods include the antisense method, the ribozyme method, the homologous recombination method, the RNA-DNA oligonucleotide method (hereinafter referred to as "RDO method"), the RNA interference method (hereinafter referred to as "RNAi method"), the method using a retrovirus, the method using a transposon and the like. These methods are specifically described below.

**(a) Preparation of the host cell for the production of the cell of the present invention by the antisense method or the ribozyme method**

The host cell used for the production of the cell of the present invention can be prepared by the antisense method or the ribozyme method described in *Cell Technology,* 12, 239 (1993); *BIO/TECHNOLOGY,* 17, 1097 (1999); *Hum. Mol. Genet.,* 5, 1083 (1995); *Cell Technology,* 13, 255 (1994); *Proc. Natl. Acad. Sci. U.S.A.,* 96, 1886 (1999); *etc.* targeting at a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, for example, in the following manner.

A cDNA or a genomic DNA encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared.

The nucleotide sequence of the prepared cDNA or genomic DNA is determined.

Based on the determined DNA sequence, an antisense gene or a ribozyme of appropriate length is designed which comprises a DNA moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, non-translated regions and introns.

In order to express the antisense gene or ribozyme in a cell, a recombinant vector is prepared by inserting a fragment or full-length of the prepared DNA into a site downstream of a promoter in an appropriate expression vector.

The recombinant vector is introduced into a host cell suited for the expression vector to obtain a transformant.

The host cell used for the production of the fusion protein composition of the present invention can be obtained by selecting a transformant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. The host cell used for the production of the composition of the present invention can also be obtained by selecting a transformant using, as a marker, the sugar chain structure of a glycoprotein on the cell membrane or the sugar chain structure of the produced fusion protein molecule.

As the host cell used for the production of the fusion protein composition of the present invention, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the designed antisense gene or ribozyme. Examples of the expression vectors include those described in 2 below.

Introduction of a gene into various host cells can be carried out by the methods suitable for introducing a recombinant vector into various host cells described in 2 below.

Selection of a transformant using, as a marker, the activity of an enzyme relating to the synthesis of an intracellular sugar nucleotide GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain can be carried out, for example, by the following methods.

Methods for selecting a transformant

A cell in which the activity of an enzyme relating to the synthesis of the intracellular sugar nucleotide GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain is deleted can be selected by measuring the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain using biochemical methods or genetic engineering techniques described in *Shin Seikagaku Jikken Koza (New Lectures on Experiments in Biochemistry) 3 - Saccharides I, Glycoprotein* (Tokyo Kagaku Dojin), edited by The Japanese Biochemical Society (1988); *Cell Technology, Extra Edition, Experimental Protocol Series, Glycobiology Experimental Protocol, Glycoprotein, Glycolipid and Proteoglycan* (Shujunsha), edited by Naoyuki Taniguchi, Akemi Suzuki, Kiyoshi Furukawa and Kazuyuki Sugawara (1996); *Molecular Cloning,* Second Edition; *Current Protocols in Molecular Biology;* and the like. An example of the biochemical methods is a method in which the enzyme activity is evaluated using an enzyme-specific substrate. Examples of the genetic engineering techniques include Northern analysis, RT-PCR and the like in which the amount of mRNA for a gene encoding the enzyme is measured.

Selection of a transformant using, as a marker, the sugar chain structure of a glycoprotein on the cell membrane can be carried out, for example, by the method described in 1(5) below. Selection of a transformant using, as a marker, the sugar chain structure of a produced fusion protein molecule can be carried out, for example, by the methods described in 4 or 5 below.

Preparation of a cDNA encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain can be carried out, for example, by the following method.

Preparation of cDNA

Total RNA or mRNA is prepared from a various host cell tissue or cell.

A cDNA library is prepared from the total RNA or mRNA.

Degenerative primers are prepared based on the amino acid sequence of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain, and a gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond of a complex type N-glycoside-linked sugar chain is obtained by PCR using the prepared cDNA library as a template.

A DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be obtained by screening the cDNA library using the obtained gene fragment as a probe.

As the mRNA of a human or non-human animal tissue or cell, commercially available one (for example, manufactured by Clontech) may be used, or it may be prepared from a human or non-human animal tissue or cell in the following manner.

The methods for preparing total RNA from a human or non-human animal tissue or cell include the guanidine thiocyanate-cesium trifluoroacetate method [*Methods in Enzymology,* 154, 3 (1987)], the acidic guanidine thiocyanate-phenolchloroform (AGPC) method [*Analytical Biochemistry,* 162, 156 (1987); *Experimental Medicine,* 9, 1937 (1991)] and the like.

The methods for preparing mRNA as poly(A)$^+$RNA from the total RNA include the oligo (dT) immobilized cellulose column method (*Molecular Cloning,* Second Edition) and the like.

It is also possible to prepare mRNA by using a commercially available kit such as Fast Track mRNA Isolation Kit (manufactured by Invitrogen) or Quick Prep mRNA Purification Kit (manufactured by Pharmacia).

A cDNA library is prepared from the obtained mRNA of a human or non-human animal tissue or cell. The methods for preparing the cDNA library include the methods described in *Molecular Cloning,* Second Edition; *Current Protocols in Molecular Biology; A Laboratory Manual,* 2nd Ed. (1989); *etc.,* and methods using commercially available kits such as SuperScript Plasmid System for cDNA Synthesis and Plasmid Cloning (manufactured by Life Technologies) and ZAP-cDNA Synthesis Kit (manufactured by Stratagene).

As the cloning vector for preparing the cDNA library, any vectors, e.g. phage vectors and plasmid vectors, can be used, so long as they are autonomously replicable in *Escherichia coli* K12. Examples of suitable vectors include ZAP Express [manufactured by Stratagene; *Strategies, 5,* 58 (1992)], pBluescript II SK(+) [*Nucleic Acids Research, 17,* 9494 (1989)], λZAP II (manufactured by Stratagene), λgt10, λgt11 [*DNA Cloning, A Practical Approach, 1,* 49 (1985)], λTrip1Ex (manufactured by Clontech), λExcell (manufactured by Pharmacia), pT7T318U (manufactured by Pharmacia), pcD2 [*Mol. Cell. Biol., 3,* 280 (1983)], pUC18 [*Gene, 33,* 103 (1985)] and the like.

Any microorganism can be used as the host microorganism for preparing the cDNA library, but *Escherichia coli* is preferably used. Examples of suitable host microorganisms are *Escherichia coli* XL1-Blue MRF' [manufactured by Stratagene; *Strategies, 5,* 81 (1992)], *Escherichia coli* C600 [*Genetics, 39,* 440 (1954)], *Escherichia coli* Y1088 [*Science, 222,* 778 (1983)], *Escherichia coli* Y1090 [*Science, 222,* 778 (1983)], *Escherichia coli* NM522 [*J. Mol. Biol., 166,* 1 (1983)], *Escherichia coli* K802 [*J. Mol. Biol., 16,* 118 (1966)] and *Escherichia coli* JM105 [*Gene, 38,* 275 (1985)].

The cDNA library may be used as such in the following analysis. Alternatively, in order to efficiently obtain full-length cDNAs by decreasing the ratio of partial cDNAs, a cDNA library prepared using the oligo-cap method developed by Sugano, *et al.* [*Gene, 138,* 171 (1994); *Gene, 200,* 149 (1997); *Protein, Nucleic Acid and Enzyme, 41,* 603 (1996); *Experimental Medicine, 11,* 2491 (1993); *cDNA Cloning* (Yodosha) (1996); *Methods for Preparing Gene Libraries* (Yodosha) (1994)] may be used in the following analysis.

Degenerative primers specific for the 5'-terminal and 3'-terminal nucleotide sequences of a nucleotide sequence presumed to encode the amino acid sequence of an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain are prepared based on the amino acid sequence of the enzyme. A gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained by DNA amplification by PCR [*PCR Protocols,* Academic Press (1990)] using the prepared cDNA library as a template.

It can be confirmed that the obtained gene fragment is a DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain by analyzing the nucleotide sequence by generally employed methods such as the dideoxy method of Sanger, *et al.* [*Proc. Natl. Acad. Sci. U.S.A., 74,* 5463 (1977)] or by use of nucleotide sequencers such as ABI PRISM 377 DNA Sequencer (manufactured by Applied Biosystems).

A DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be obtained from the cDNA or cDNA library synthesized from the mRNA contained in a human or non-human animal tissue or cell by colony hybridization, plaque hybridization (*Molecular Cloning,* Second Edition; *Current Protocols in Molecular Biology*) or the like using the above gene fragment as a probe.

A cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can also be obtained by amplification by PCR using the cDNA or cDNA library synthesized from the mRNA contained in a human or non-human animal tissue or cell as a template and using the primers used for obtaining the gene fragment encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain.

The nucleotide sequence of the obtained cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be determined by generally employed nucleotide sequencing methods such as the dideoxy method of Sanger, *et al.* [*Proc. Natl. Acad. Sci. U.S.A., 74,* 5463 (1977)] or by use of nucleotide sequencers such as ABI PRISM 377 DNA Sequencer (manufactured by Applied Biosystems).

By carrying out a search of nucleotide sequence databases such as GenBank, EMBL or DDBJ using a homology search program such as BLAST based on the determined nucleotide sequence of the cDNA, it can be confirmed that the obtained DNA is a gene encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain among the genes in the nucleotide sequence database.

Examples of the nucleotide sequences of the genes encoding the enzyme relating to the synthesis of an intracellular sugar nucleotide, GDP-fucose obtained by the above methods include the nucleotide sequences represented by SEQ ID NO:1 or 3.

Examples of the nucleotide sequences of the genes encoding the enzyme relating to the modification of a sugar chain in which 1-position of fucose is bound to 6-position of N-acetylglucosamine in the reducing end through $\alpha$-bond in a complex type N-glycoside-linked sugar chain obtained by the above methods include the nucleotide sequences represented by SEQ ID NO:5 or 6.

The cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can also be obtained by chemical synthesis with a DNA synthesizer such as DNA Synthesizer Model 392 (manufactured by Perkin Elmer) utilizing the phosphoamidite method based on the determined nucleotide sequence of the DNA.

Preparation of a genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be carried out, for example, by the following method.

Method for preparing genomic DNA

The genomic DNA can be prepared by known methods described in *Molecular Cloning,* Second Edition; *Current Protocols in Molecular Biology; etc.* In addition, the genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be obtained by using a kit such as Genomic DNA Library Screening System (manufactured by Genome Systems) or Universal GenomeWalker™ Kits (manufactured by CLONTECH).

The nucleotide sequence of the obtained DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be determined by generally employed nucleotide sequencing methods such as the dideoxy method of Sanger, *et al.* [*Proc. Natl. Acad. Sci. U.S.A.,* 74, 5463 (1977)] or by use of nucleotide sequencers such as ABI PRISM 377 DNA Sequencer (manufactured by Applied Biosystems).

By carrying out a search of nucleotide sequence databases such as GenBank, EMBL or DDBJ using a homology search program such as BLAST based on the determined nucleotide sequence of the genomic DNA, it can be confirmed that the obtained DNA is a gene encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain among the genes in the nucleotide sequence database.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can also be obtained by chemical synthesis with a DNA synthesizer such as DNA Synthesizer Model 392 (manufactured by Perkin Elmer) utilizing the phosphoamidite method based on the determined nucleotide sequence of the DNA.

Examples of the nucleotide sequences of the genomic DNAs encoding the enzyme relating to the synthesis of an intracellular sugar nucleotide, GDP-fucose obtained by the above methods include the nucleotide sequences represented by SEQ ID NOs:110, 111, 112 and 113.

An example of the nucleotide sequence of the genomic DNA encoding the enzyme relating to the modification of a sugar chain in which 1-position of fucose is bound to 6-position of N-acetylglucosamine in the reducing end through $\alpha$-bond in a complex type N-glycoside-linked sugar chain obtained by the above methods is the nucleotide sequence represented by SEQ ID NO:55.

The host cell used for the production of the fusion protein composition of the present invention can also be obtained without using an expression vector by directly introducing into a host cell an antisense oligonucleotide or ribozyme designed based on the nucleotide sequence encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain.

The antisense oligonucleotide or ribozyme can be prepared by known methods or by using a DNA synthesizer. Specifically, based on the sequence information on an oligonucleotide having a sequence corresponding to 5 to 150, preferably 5 to 60, more preferably 10 to 40 contiguous nucleotides in the nucleotide sequence of the cDNA or genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, an oligonucleotide corresponding to the sequence complementary to the above oligonucleotide (antisense oligonucleotide) or a ribozyme comprising the oligonucleotide sequence can be synthesized.

The oligonucleotide includes oligo RNA and derivatives of the oligonucleotide (hereinafter referred to as oligonucleotide derivatives).

The oligonucleotide derivatives include an oligonucleotide derivative wherein the phosphodiester bond in the oligonucleotide is converted to a phosophorothioate bond, an oligonucleotide derivative wherein the phosphodiester bond in the oligonucleotide is converted to an N3'-P5' phosphoamidate bond, an oligonucleotide derivative wherein the ribose-phosphodiester bond in the oligonucleotide is converted to a peptide-nucleic acid bond, an oligonucleotide derivative wherein the uracil in the oligonucleotide is substituted by C-5 propynyluracil, an oligonucleotide derivative wherein the uracil in the oligonucleotide is substituted by C-5 thiazolyluracil, an oligonucleotide derivative wherein the cytosine in the oligonucleotide is substituted by C-5 propynylcytosine, an oligonucleotide derivative wherein the cytosine in the oligonucleotide is substituted by phenoxazine-modified cytosine, an oligonucleotide derivative wherein the ribose in the oligonucleotide is substituted by 2'-O-propylribose, an oligonucleotide derivative wherein the ribose in the oligonucleotide is substituted by 2'-methoxyethoxyribose *[Cell Technology,* 16, 1463 (1997)], and the like.

(b) Preparation of the host cell for the production of the fusion protein composition of the present invention by the homologous recombination method

The host cell used for the production of the fusion protein composition of the present invention can be prepared by modifying a target gene on the chromosome by the homologous recombination method targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain.

Modification of the target gene on the chromosome can be carried out by using the methods described in *Manipulating the Mouse Embryo, A Laboratory Manual,* Second Edition, Cold Spring Harbor Laboratory Press (1994) (hereinafter referred to as *Manipulating the Mouse Embryo, A Laboratory Manual); Gene Targeting, A Practical Approach,* IRL Press at Oxford University Press (1993); *Biomanual Series 8, Gene Targeting, Preparation of Mutant Mice Using ES Cells,* Yodosha (1995) (hereinafter referred to as *Preparation of Mutant Mice Using ES Cells); etc.,* for example, in the following manner.

A genomic DNA encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain is prepared.

Based on the nucleotide sequence of the genomic DNA, a target vector is prepared for homologous recombination of a target gene to be modified (e.g., the structural gene or promoter gene for the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain).

The host cell used for the preparation of the cell of the present invention can be obtained by introducing the prepared target vector into a host cell and selecting a cell in which homologous recombination occurred between the target gene on the chromosome and the target vector.

As the host cell, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain can be prepared by the methods for preparing a genomic DNA described in the above 1 (1) (a), *etc.*

Examples of the nucleotide sequences of the genomic DNAs encoding the enzyme relating to the synthesis of the intracellular sugar nucleotide GDP-fucose obtained by the above methods include the nucleotide sequences represented by SEQ ID NOs:110, 111, 112 and 113.

An example of the nucleotide sequence of the genomic DNA encoding the enzyme relating to the modification of a sugar chain in which 1-position of fucose is bound to 6-position of N-acetylglucosamine in the reducing end through

$\alpha$-bond in a complex type N-glycoside-linked sugar chain obtained by the above methods includes the nucleotide sequence represented by SEQ ID NO:55.

The target vector for use in the homologous recombination of the target gene on the chromosome can be prepared according to the methods described in *Gene Targeting, A Practical Approach,* IRL Press at Oxford University Press (1993); *Preparation of Mutant Mice Using ES Cells; etc.* The target vector may be either a replacement-type one or an insertion-type one.

Introduction of the target vector into various host cells can be carried out by the methods suitable for introducing a recombinant vector into various host cells described in 3 below.

The methods for efficiently selecting a homologous recombinant include positive selection, promoter selection, negative selection, polyA selection and the like described in *Gene Targeting, A Practical Approach,* IRL Press at Oxford University Press (1993); *Preparation of Mutant Mice Using ES Cells; etc.* The methods for selecting the desired homologous recombinant from the selected cell lines include Southern hybridization (*Molecular Cloning,* Second Edition) and PCR [*PCR Protocols,* Academic Press (1990)] with the genomic DNA.

(c) Preparation of the host cell for the production of the fusion protein composition of the present invention by the RDO method

The host cell used for the production of the fusion protein composition of the present invention can be prepared by the RDO method targeting a gene encoding an enzyme relating to the synthesis of the intracellular sugar nucleotide GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain, for example, in the following manner.

A cDNA or a genomic DNA encoding an enzyme relating to the synthesis of the intracellular sugar nucleotide GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain is prepared by the methods described in the above 1 (1) (a).

The nucleotide sequence of the prepared cDNA or genomic DNA is determined.

Based on the determined DNA sequence, an RDO construct of appropriate length which comprises a DNA moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain, non-translated regions and introns is designed and synthesized.

The host cell of the present invention can be obtained by introducing the synthesized RDO into a host cell and then selecting a transformant in which a mutation occurred in the target enzyme, that is, the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain.

As the host cell, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain. Examples of the host cells include those described in 2 below.

Introduction of the RDO into various host cells can be carried out by the methods suitable for introducing a recombinant vector into various host cells described in 2 below.

The cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be prepared by the methods for preparing a cDNA described in the above 1 (1) (a) or the like.

The genomic DNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be prepared by the methods for preparing a genomic DNA described in the above 1 (1) (b) or the like.

After DNA is cleaved with appropriate restriction enzymes, the nucleotide sequence of the DNA can be determined by cloning the DNA fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad. Sci. USA,* 74, 5463 (1977)] or the like, and then analyzing the clones by using an automatic nucleotide sequence analyzer such as ABI PRISM 377 DNA Sequencer (manufactured by Applied Biosystems) or the like.

The RDO can be prepared by conventional methods or by using a DNA synthesizer.

The methods for selecting a cell in which a mutation occurred by introducing the RDO into the host cell, in the gene encoding the target enzyme, that is, the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods for directly detecting mutations in chromosomal genes described in *Molecular Cloning,* Second Edition; *Current Protocols in Molecular Biology; etc.*

For the selection of the transformant, the following methods can also be employed: the method using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain described in the above 1 (1) (a); the method using, as a marker, the sugar chain structure of a glycoprotein on the cell membrane described in 1 (5) below; and the method using, as a marker, the sugar chain structure of a produced fusion protein molecule described in 4 and 5 below.

The RDO can be designed according to the descriptions in *Science,* 273, 1386 (1996); *Nature Medicine,* 4, 285 (1998); *Hepatology,* 25, 1462 (1997); *Gene Therapy,* 5, 1960 (1999); *Gene Therapy,* 5, 1960 (1999); *J. Mol. Med.,* 75, 829 (1997); *Proc. Natl. Acad. Sci. USA,* 96, 8774 (1999); *Proc. Natl. Acad. Sci. USA,* 96, 8768 (1999); *Nuc. Acids Res.,* 27, 1323 (1999); *Invest. Dematol.,* 111, 1172 (1998); *Nature Biotech.,* 16, 1343 (1998); *Nature Biotech.,* 18, 43 (2000); *Nature Biotech.,* 18, 555 (2000); *etc.*

(d) Preparation of the host cell for the production of the fusion protein composition of the present invention by the RNAi method

The host cell used for the production of the fusion protein composition of the present invention can be prepared by the RNAi method targeting a gene encoding an enzyme relating to the synthesis of an intracellular sugar nucleotide, GDP-fucose or an enzyme relating to the modification of a sugar chain in which 1-position of fucose is bound to 6-position of N-acetylglucosamine in the reducing end through α-bond in a complex type N-glycoside-linked sugar chain, for example, in the following manner.

A cDNA encoding an enzyme relating to the synthesis of the intracellular sugar nucleotide GDP-fucose or an enzyme relating to the modification of a sugar chain in which 1-position of fucose is bound to 6-position of N-acetylglucosamine in the reducing end through α-bond in a complex type N-glycoside-linked sugar chain is prepared by the methods described in the above 1 (1) (a).

The nucleotide sequence of the prepared cDNA is determined.

Based on the determined cDNA sequence, an RNAi gene of appropriate length is designed which comprises a moiety encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or non-translated regions.

In order to express the RNAi gene in a cell, a recombinant vector is prepared by inserting a fragment or full-length of the prepared cDNA into a site downstream of a promoter in an appropriate expression vector.

The recombinant vector is introduced into a host cell suited for the expression vector to obtain a transformant.

The host cell used for the preparation of the cell of the present invention can be prepared by selecting a transformant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or the sugar chain structure of a produced fusion protein molecule or a glycoprotein on the cell membrane.

As the host cell, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the designed RNAi gene. Examples of the expression vectors include those described in 2 below.

Introduction of a gene into various host cells can be carried out by the methods suitable for introducing a recombinant vector into various host cells described in 2 below.

The methods for selecting the transformant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a).

The methods for selecting the transformant using, as a marker, the sugar chain structure of a glycoprotein on the cell membrane include the method described in 1 (5). The methods for selecting the transformant using, as a marker, the sugar chain structure of a produced fusion protein molecule include the methods described in 4 or 5 below.

The cDNA encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain can be prepared by the methods for preparing a cDNA described in the above 1 (1) (a), *etc.*

The host cell used for the preparation of the cell of the present invention can also be obtained without using an expression vector by directly introducing into a host cell the RNAi gene designed based on the nucleotide sequence encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain.

The RNAi gene can be prepared by known methods or by using a DNA synthesizer.

The RNAi gene construct can be designed according to the descriptions in *Nature,* *391*, 806 (1998); *Proc. Natl. Acad. Sci. USA,* *95*, 15502 (1998); *Nature, 395*, 854 (1998); *Proc. Natl. Acad. Sci. USA, 96*, 5049 (1999); *Cell, 95*, 1017 (1998); *Proc. Natl. Acad. Sci. USA, 96*, 1451 (1999); *Proc. Natl. Acad. Sci. USA, 95*, 13959 (1998); *Nature Cell Biol., 2*, 70 (2000); *etc.*

(e) Preparation of the host cell for the production of the fusion protein composition of the present invention by the method using a transposon

The host cell used for the production of the fusion protein composition of the present invention can be prepared by using the transposon system described in *Nature Genet., 25*, 35 (2000), *etc.,* and then selecting a mutant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain, or the sugar chain structure of a produced fusion protein molecule or a glycoprotein on the cell membrane.

The transposon system is a system for inducing a mutation by random insertion of an exogenous gene into the chromosome, wherein usually an exogenous gene inserted into a transposon is used as a vector for inducing a mutation and a transposase expression vector for randomly inserting the gene into the chromosome is introduced into the cell at the same time.

Any transposase can be used, so long as it is suitable for the sequence of the transposon to be used.

As the exogenous gene, any gene can be used, so long as it can induce a mutation in the DNA of a host cell.

As the host cell, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain. Examples of the host cells include those described in 2 below. Introduction of the gene into various host cells can be carried out by the methods suitable for introducing a recombinant vector into various host cells described in 2 below.

The methods for selecting the mutant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through $\alpha$-bond in a complex type N-glycoside-linked sugar chain include the methods described in the above 1 (1) (a).

The methods for selecting the mutant using, as a marker, the sugar chain structure of a glycoprotein on the cell membrane include the method described in 1 (5). The methods for selecting the mutant using, as a marker, the sugar chain structure of a produced fusion protein molecule include the methods described in 4 or 5 below.

(2) Technique of introducing a dominant-negative mutant of a gene encoding an enzyme

The host cell used for the production of the fusion protein composition of the present invention can be prepared by using the method of introducing a dominant-negative mutant of a target gene, i.e., a gene encoding an enzyme relating to the synthesis of the intracellular sugar nucleotide, GDP-fucose or an enzyme relating to the modification of a sugar chain in which 1-position of fucose is bound to 6-position of N-acetylglucosamine in the reducing end through $\alpha$-bond in a complex type N-glycoside-linked sugar chain. Examples of the enzymes relating to the synthesis of the intracellular sugar nucleotide GDP-fucose include GMD and Fx. Examples of the enzymes relating to the modification of a sugar chain in which 1-position of fucose is bound to 6-position of N-acetylglucosamine in the reducing end through $\alpha$-bond in a complex type N-glycoside-linked sugar chain include $\alpha$1,6-fucosyltransferase, $\alpha$-L-fucosidase and the like.

These enzymes have substrate specificity and catalyze specific reactions. By disrupting the active center of such enzymes having substrate specificity and catalytic action, their dominant-negative mutants can be prepared. Preparation of a dominant-negative mutant is described in detail below, using for an example GMD among the target enzymes.

As a result of the analysis of the tertiary structure of GMD derived from *Escherichia coli,* it has been revealed that four amino acids (threonine at position 133, glutamic acid at position 135, tyrosine at position 157 and lysine at position 161) have an important function for the enzyme activity (*Structure,* 8, 2, 2000). That is, all mutants prepared by substituting the above four amino acids by other amino acids based on the tertiary structure information showed significantly decreased enzyme activity. On the other hand, little change was observed in the ability of the mutants to bind to the GMD coenzyme NADP or the substrate GDP-mannose. Accordingly, a dominant-negative mutant can be prepared by substituting the four amino acids which are responsible for the enzyme activity of GMD. On the basis of the result of preparation of a dominant-negative mutant of GMD derived from *Escherichia coli,* dominant-negative mutants of GMDs can be prepared by performing homology comparison and tertiary structure prediction using the amino acid sequence information. For example, in the case of GMD derived from CHO cell (SEQ ID NO:2), a dominant-negative mutant can be prepared by substituting threonine at position 155, glutamic acid at position 157, tyrosine at position 179 and lysine at position 183 by other amino acids. Preparation of such a gene carrying introduced amino acid substitutions can be carried out by site-directed mutagenesis described in *Molecular Cloning,* Second Edition; *Current Protocols in Molecular Biology; etc.*

The host cell used for the production of the fusion protein composition of the present invention can be prepared according to the method of gene introduction described in *Molecular Cloning,* Second Edition; *Current Protocols in Molecular Biology; Manipulating the Mouse Embryo,* Second Edition; *etc.* using a gene encoding a dominant-negative mutant of a target enzyme (hereinafter abbreviated as "dominant-negative mutant gene") prepared as above, for example, in the following manner.

A dominant-negative mutant gene encoding the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain is prepared.

Based on the full-length DNA of the prepared dominant-negative mutant gene, a DNA fragment of appropriate length containing a region encoding the protein is prepared according to need.

A recombinant vector is prepared by inserting the DNA fragment or full-length DNA into a site downstream of a promoter in an appropriate expression vector.

The recombinant vector is introduced into a host cell suited for the expression vector to obtain a transformant.

The host cell used for the preparation of the cell of the present invention can be obtained by selecting a transformant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain, or the sugar chain structure of a produced fusion protein molecule or a glycoprotein on the cell membrane.

As the host cell, any yeast cell, animal cell, insect cell, plant cell or the like can be used, so long as it has a gene encoding the target enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain. Examples of the host cells include those described in 2 below.

The expression vectors that can be employed are those capable of autonomous replication or integration into the chromosome in the above host cells and comprising a promoter at a position appropriate for the transcription of the DNA encoding the desired dominant-negative mutant. Examples of the expression vectors include those described in 2 below.

Introduction of a gene into various host cells can be carried out by the methods suitable for introducing a recombinant vector into various host cells described in 2 below.

The methods for selecting the transformant using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1(1)(a).

The methods for selecting the transformant using, as a marker, the sugar chain structure of a glycoprotein on the cell membrane include the method described in 1 (5) below. The methods for selecting the transformant using, as a marker, the sugar chain structure of a produced fusion protein molecule include the methods described in 4 or 5 below.

(3) Technique of introducing a mutation into an enzyme

The host cell used for the production of the fusion protein composition of the present invention can be prepared by introducing a mutation into a gene encoding an enzyme relating to the synthesis of the intracellular sugar nucleotide GDP-fucose or an enzyme relating to the modification of a sugar chain in which 1-position of fucose is bound to 6-position of N-acetylglucosamine in the reducing end through α-bond in a complex type N-glycoside-linked sugar chain, and then selecting a desired cell line in which the mutation occurred in the enzyme.

Examples of the enzymes relating to the synthesis of the intracellular sugar nucleotide GDP-fucose include GMD, Fx and the like. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase, α-L-fucosidase and the like.

The methods for introducing a mutation into the enzyme include: 1) a method in which a desired cell line is selected from mutants obtained by subjecting a parent cell line to mutagenesis or by spontaneous mutation using, as a marker, the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain; 2) a method in which a desired cell line is selected from mutants obtained by subjecting a parent cell line to mutagenesis or by spontaneous mutation using, as a marker, the sugar chain structure of a produced fusion protein molecule; and 3) a method in which a desired cell line is selected from mutants obtained by subjecting a parent cell line to mutagenesis or by spontaneous mutation using, as a marker, the sugar chain structure of a glycoprotein on the cell membrane.

Mutagenesis may be carried out by any method capable of inducing a point mutation, a deletion mutation or a frameshift mutation in DNA of a cell of a parent cell line.

Suitable methods include treatment with ethyl nitrosourea, nitrosoguanidine, benzopyrene or an acridine dye, radiation treatment and the like. Various alkylating agents and carcinogens are also useful as mutagens. A mutagen is allowed to act on a cell by the methods described in *Soshiki Baiyo no Gijutsu (Tissue Culture Techniques),* Third Edition (Asakura Shoten), edited by The Japanese Tissue Culture Association (1996); *Nature Genet.,* 24, 314 (2000); *etc.*

Examples of the mutants generated by spontaneous mutation include spontaneous mutants obtained by continuing subculture under usual cell culture conditions without any particular treatment for mutagenesis.

The methods for measuring the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a). The methods for determining the sugar chain structure of a produced fusion protein molecule include the methods described in 4 or 5 below. The methods for determining the sugar chain structure of a glycoprotein on the cell membrane include the method described in 1 (5).

(4) Technique of suppressing transcription or translation of a gene encoding an enzyme

The host cell used for the production of the fusion protein composition of the present invention can be prepared by inhibiting transcription or translation of a target gene, i.e., a gene encoding an enzyme relating to the synthesis of the intracellular sugar nucleotide GDP-fucose or an enzyme relating to the modification of a sugar chain in which 1-position of fucose is bound to 6-position of N-acetylglucosamine in the reducing end through α-bond in a complex type N-glycoside-linked sugar chain using the antisense RNA/DNA technique *[Bioscience and Industry,* 50, 322 (1992); *Chemistry,* 46, 681 (1991); *Biotechnology,* 9, 358 (1992); *Trends in Biotechnology,* 10, 87 (1992); *Trends in Biotechnology,* 10, 152 (1992); *Cell Technology,* 16, 1463 (1997)], the triple helix technique [*Trends in Biotechnology,* 10, 132 (1992)], *etc.*

Examples of the enzymes relating to the synthesis of the intracellular sugar nucleotide GDP-fucose include GMD, Fx and the like. Examples of the enzymes relating to the modification of a sugar chain in which 1-position of fucose is bound to 6-position of N-acetylglucosamine in the reducing end through α-bond in a complex type N-glycoside-linked sugar chain include α1,6-fucosyltransferase, α-L-fucosidase and the like.

The methods for measuring the activity of the enzyme relating to the synthesis of an intracellular sugar nucleotide, GDP-fucose or the enzyme relating to the modification of a sugar chain in which 1-position of fucose is bound to 6-position of N-acetylglucosamine in the reducing end through α-bond in a complex type N-glycoside-linked sugar chain include the methods described in the above 1 (1) (a).

The methods for determining the sugar chain structure of a glycoprotein on the cell membrane include the method described in 1 (5). The methods for determining the sugar chain structure of a produced fusion protein molecule include the methods described in 4 or 5 below.

(5) Technique of selecting a cell line resistant to a lectin which recognizes a sugar chain structure in which 1-position of fucose is bound to 6-position of N-acetylglucosamine in the reducing end through α-bond in a complex type N-glycoside-linked sugar chain

The host cell used for the production of the fusion protein composition of the present invention can be prepared by selecting a cell line resistant to a lectin which recognizes a sugar chain structure in which 1-position of fucose is bound to 6-position of N-acetylglucosamine in the reducing end through α-bond in a complex type N-glycoside-linked sugar chain.

Selection of a cell line resistant to a lectin which recognizes a sugar chain structure in which 1-position of fucose is bound to 6-position of N-acetylglucosamine in the reducing end through α-bond in a complex type N-glycoside-linked sugar chain can be carried out, for example, by the method using a lectin described in *Somatic Cell Mol. Genet.,* 12, 51 (1986), *etc.*

As the lectin, any lectin can be used, so long as it recognizes a sugar chain structure in which 1-position of fucose is bound to 6-position of N-acetylglucosamine in the reducing end through α-bond in a complex type N-glycoside-linked sugar chain. Specific examples include lentil lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

Specifically, the cell line of the present invention resistant to a lectin which recognizes a sugar chain structure in which 1-position of fucose is bound to 6-position of N-acetylglucosamine in the reducing end through α-bond in a complex type N-glycoside-linked sugar chain can be selected by culturing cells in a medium containing the above lectin in a concentration of 1 μg/ml to 1 mg/ml for 1 day to 2 weeks, preferably 1 day to 1 week, subculturing surviving cells or picking up a colony and transferring it into a culture vessel, and subsequently continuing the culturing using the medium containing the lectin.

2. Process for producing the fusion protein composition

The fusion protein composition can be obtained by expressing it in a host cell using the methods described in *Molecular Cloning, A Laboratory Manual,* Second Edition, Cold Spring Harbor Laboratory Press (1989), *Current Protocols in Molecular Biology,* John Wiley & Sons (1987-1997), *Antibodies, A Laboratory Manual,* Cold Spring Harbor Laboratory (1988), *Monoclonal Antibodies: Principles and Practice,* Third Edition, Acad. Press (1993), *Antibody Engineering, A Practical Approach,* IRL Press at Oxford University Press (1996); *etc.,* for example, in the following manner.

A full-length cDNA encoding the fusion protein molecule is prepared, and a DNA fragment of appropriate length comprising a region encoding the fusion protein molecule is prepared.

An expression vector is prepared by inserting the DNA fragment or full-length DNA into a site downstream of a promoter in an appropriate expression vector.

The expression vector is introduced into a host cell suited for the expression vector to obtain a transformant producing the fusion protein molecule.

As the host cell, any yeast cells, animal cells, insect cells, plant cells, *etc.* that are capable of expressing the fusion protein can be used.

Also useful are cells obtained by selecting cells in which the activity of an enzyme relating to the modification of an N-glycoside-linked sugar chain bound to the Fc region of an fusion protein molecule, i.e., an enzyme relating to the synthesis of an intracellular sugar nucleotide GDP-fucose or an enzyme relating to the modification of a sugar chain in which 1-position of fucose is bound to 6-position of N-acetylglucosamine in the reducing end through α-bond in a complex type N-glycoside-linked sugar chain is deleted, or cells obtained by various artificial techniques described in the above 1.

The expression vectors that can be employed are those capable of autonomous replication or integration into the chromosome in the above host cells and comprising a promoter at a position appropriate for the transcription of the DNA encoding the desired fusion protein molecule.

The cDNA can be prepared from a human or non-human animal tissue or cell according to the methods for preparing a cDNA described in the above 1 (1) (a) using, e.g., a probe or primers specific for the cDNA encoding the desired fusion protein molecule.

When a yeast cell is used as the host cell, YEP13 (ATCC 37115), YEp24 (ATCC 37051), YCp50 (ATCC 37419), *etc.* can be used as the expression vector.

As the promoter, any promoters capable of expressing in yeast strains can be used. Suitable promoters include promoters of genes of the glycolytic pathway such as hexokinase, PHO5 promoter, PGK promoter, GAP promoter, ADH promoter, gal 1 promoter, gal 10 promoter, heat shock protein promoter, MFα1 promoter and CUP 1 promoter.

Examples of suitable host cells are microorganisms belonging to the genera *Saccharomyces, Schizosaccharomyces, Kluyveromyces, Trichosporon* and *Schwanniomyces,* and specifically, *Saccharomyces cerevisiae, Schizosac-*

*charomyces pombe, Kluyveromyces lactis, Trichosporon pullulans* and *Schwanniomyces alluvius.*

Introduction of the expression vector can be carried out by any of the methods for introducing DNA into yeast, for example, electroporation [*Methods Enzymol.,* 194, 182 (1990)], the spheroplast method [*Proc. Natl. Acad. Sci. USA,* 84, 1929 (1978)], the lithium acetate method [*J. Bacteriology,* 153, 163 (1983)] and the method described in *Proc. Natl. Acad. Sci. USA,* 75, 1929 (1978).

When an animal cell is used as the host cell, pcDNAI, pcDM8 (commercially available from Funakoshi Co., Ltd.), pAGE107 [Japanese Published Unexamined Patent Application No. 22979/91; *Cytotechnology,* 3, 133 (1990)], pAS3-3 (Japanese Published Unexamined Patent Application No. 227075/90), pCDM8 [*Nature,* 329, 840 (1987)], pcDNAI/Amp (manufactured by Invitrogen Corp.), pREP4 (manufactured by Invitrogen Corp.), pAGE103 [*J. Biochemistry,* 101, 1307 (1987)], pAGE210, *etc.* can be used as the expression vector.

As the promoter, any promoters capable of expressing in animal cells can be used. Suitable promoters include the promoter of IE (immediate early) gene of cytomegalovirus (CMV), SV40 early promoter, the promoter of a retrovirus, metallothionein promoter, heat shock promoter, SRα promoter, *etc.* The enhancer of IE gene of human CMV may be used in combination with the promoter.

Examples of suitable host cells are human-derived Namalwa cells, monkey-derived COS cells, Chinese hamster-derived CHO cells, HBT5637 (Japanese Published Unexamined Patent Application No. 299/88), rat myeloma cells, mouse myeloma cells, cells derived from Syrian hamster kidney, embryonic stem cells, fertilized egg cells and the like.

Introduction of the expression vector can be carried out by any of the methods for introducing DNA into animal cells, for example, electroporation [*Cytotechnology,* 3, 133 (1990)], the calcium phosphate method (Japanese Published Unexamined Patent Application No. 227075/90), lipofection [*Proc. Natl. Acad. Sci. USA,* 84, 7413 (1987)], the injection method (*Manipulating the Mouse Embryo, A Laboratory Manual*), the method using particle gun (gene gun) (Japanese Patent Nos. 2606856 and 2517813), the DEAE-dextran method [*Biomanual Series 4 - Methods of Gene Introduction, Expression and Analysis* (Yodosha), edited by Takashi Yokota and Kenichi Arai (1994)] and the virus vector method (*Manipulating the Mouse Embryo, A Laboratory Manual*).

When an insect cell is used as the host cell, the protein can be expressed by the methods described in *Current Protocols in Molecular Biology; Baculovirus Expression Vectors, A Laboratory Manual,* W. H. Freeman and Company, New York (1992); Bio/Technology, 6, 47 (1988), *etc.*

That is, the expression vector and a baculovirus are cotransfected into insect cells to obtain a recombinant virus in the culture supernatant of the insect cells, and then insect cells are infected with the recombinant virus, whereby the protein can be expressed.

The gene introduction vectors useful in this method include pVL1392, pVL1393, pBlueBacIII (products of Invitrogen Corp.) and the like.

Examples of the baculovirus includes Autographa californica nuclear polyhedrosis virus and the like, which are virus infecting insects belonging to the family *Barathra.*

Examples of the insect cells are *Spodoptera frugiperda* ovarian cells Sf9 and Sf21 [*Current Protocols in Molecular Biology; Baculovirus Expression Vectors, A Laboratory Manual,* W. H. Freeman and Company, New York (1992)], *Trichoplusiani* ovarian cell High 5 (manufactured by Invitrogen Corp.) and the like.

Cotransfection of the above expression vector and the above baculovirus into insect cells for the preparation of the recombinant virus can be carried out by the calcium phosphate method (Japanese Published Unexamined Patent Application No. 227075/90), lipofection [*Proc. Natl. Acad Sci. USA,* 84, 7413 (1987)], *etc.*

When a plant cell is used as the host cell, Ti plasmid, tobacco mosaic virus vector, *etc.* can be used as the expression vector.

As the promoter, any promoters capable of expressing in plant cells can be used. Suitable promoters include 35S promoter of cauliflower mosaic virus (CaMV), rice actin 1 promoter, *etc.*

Examples of suitable host cells are cells of plants such as tobacco, potato, tomato, carrot, soybean, rape, alfalfa, rice, wheat and barley.

Introduction of the expression vector can be carried out by any of the methods for introducing DNA into plant cells, for example, the method using *Agrobacterium* (Japanese Published Unexamined Patent Application Nos. 140885/84 and 70080/85, WO94/00977), electroporation (Japanese Published Unexamined Patent Application No. 251887/85) and the method using particle gun (gene gun) (Japanese Patent Nos. 2606856 and 2517813).

Expression of the fusion protein gene can be carried out not only by direct expression but also by secretory production, expression of a fusion protein *etc.* according to the methods described in *Molecular Cloning,* Second Edition, *etc.*

When the gene is expressed in yeast, an animal cell, an insect cell or a plant cell carrying an introduced gene relating to the synthesis of a sugar chain, a fusion protein composition to which a sugar or a sugar chain is added by the introduced gene can be obtained.

The fusion protein composition can be produced by culturing the transformant obtained as above in a medium, allowing the fusion protein compositions to form and accumulate in the culture, and recovering them from the culture.

Culturing of the transformant in a medium can be carried out by conventional methods for culturing the host cell.

As the medium for culturing the transformant obtained by using a eucaryote such as yeast as the host, any of natural media and synthetic media can be used insofar as it is a medium suitable for efficient culturing of the transformant which contains carbon sources, nitrogen sources, inorganic salts, *etc.* which can be assimilated by the host used.

As the carbon sources, any carbon sources that can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells, digested products thereof and the like.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate, calcium carbonate and the like.

Culturing is usually carried out under aerobic conditions, for example, by shaking culture or submerged spinner culture under aeration. The culturing temperature is preferably 15 to 40°C, and the culturing period is usually 16 hours to 7 days. The pH is maintained at 3.0 to 9.0 during the culturing. The pH adjustment is carried out by using an organic or inorganic acid, an alkali solution, urea, calcium carbonate, ammonia, *etc.*

If necessary, antibiotics such as ampicillin and tetracycline may be added to the medium during the culturing.

When a microorganism transformed with a recombinant vector comprising an inducible promoter is cultured, an inducer may be added to the medium, if necessary. For example, in the case of a microorganism transformed with a recombinant vector comprising *lac* promoter, isopropyl-β-D-thiogalactopyranoside or the like may be added to the medium; and in the case of a microorganism transformed with a recombinant vector comprising *trp* promoter, indoleacrylic acid or the like may be added.

As the medium for culturing the transformant obtained by using an animal cell as the host cell, generally employed media such as RPMI1640 medium [*The Journal of the American Medical Association,* 199, 519 (1967)], Eagle's MEM medium [*Science,* 122, 501 (1952)], Dulbecco's modified MEM medium [*Virology,* 8, 396 (1959)], 199 medium [*Proceeding of the Society for the Biological Medicine,* 73, 1 (1950)] and Whitten's medium [*Developmental Engineering Experimentation Manual - Preparation of Transgenic Mice* (Kodansha), edited by Motoya Katsuki (1987)], media prepared by adding fetal calf serum or the like to these media, *etc.* can be used as the medium.

Culturing is usually carried out under conditions of pH 6.0 to 8.0 at 30 to 40°C for 1 to 7 days in the presence of 5% $CO_2$.

If necessary, antibiotics such as kanamycin and penicillin may be added to the medium during the culturing.

As the medium for culturing the transformant obtained by using an insect cell as the host cell, generally employed media such as TNM-FH medium (manufactured by Pharmingen, Inc.), Sf-900 II SFM medium (manufactured by Life Technologies, Inc.), ExCell 400 and ExCell 405 (manufactured by JRH Biosciences, Inc.) and Grace's Insect Medium [*Nature,* 195, 788 (1962)] can be used as the medium.

Culturing is usually carried out under conditions of pH 6.0 to 7.0 at 25 to 30°C for 1 to 5 days.

If necessary, antibiotics such as gentamicin may be added to the medium during the culturing.

The transformant obtained by using a plant cell as the host cell may be cultured in the form of cells as such or after differentiation into plant cells or plant organs. As the medium for culturing such transformant, generally employed media such as Murashige-Skoog (MS) medium and White medium, media prepared by adding phytohormones such as auxin and cytokinin to these media, *etc.* can be used as the medium.

Culturing is usually carried out under conditions of pH 5.0 to 9.0 at 20 to 40°C for 3 to 60 days.

If necessary, antibiotics such as kanamycin and hygromycin may be added to the medium during the culturing.

As described above, the fusion protein composition can be produced by culturing, according to a conventional culturing method, the transformant derived from a yeast cell, an animal cell, an insect cell or a plant cell and carrying an expression vector into which DNA encoding the fusion protein molecule has been inserted, allowing the fusion protein composition to form and accumulate, and recovering the fusion protein composition from the culture.

Expression of the fusion protein gene can be carried out not only by direct expression but also by secretory production, fusion protein expression, *etc.* according to the methods described in *Molecular Cloning,* Second Edition.

The fusion protein composition may be produced by intracellular production in host cells, extracellular secretion from host cells or production on outer membranes of host cells. The production method can be adopted by changing the kind of the host cells used or the structure of the fusion protein molecule to be produced.

When the fusion protein composition is produced in host cells or on outer membranes of host cells, it is possible to force the fusion protein composition to be secreted outside the host cells by applying the method of Paulson, *et al.* [*J. Biol. Chem.,* 264, 17619 (1989)], the method of Lowe, *et al.* [*Proc. Natl. Acad. Sci. USA,* 86, 8227 (1989); *Genes Develop.,* 4, 1288 (1990)], or the methods described in Japanese Published Unexamined Patent Application No.

336963/93, 823021/94, *etc.*

That is, it is possible to force the desired fusion protein composition to be secreted outside the host cells by inserting DNA encoding the fusion protein molecule and DNA encoding a signal peptide suitable for the expression of the fusion protein molecule into an expression vector, introducing the expression vector into the host cells, and then expressing the fusion protein molecule by use of recombinant DNA techniques.

It is also possible to increase the production of the fusion protein composition by utilizing a gene amplification system using a dihydrofolate reductase gene or the like according to the method described in Japanese Published Unexamined Patent Application No. 227075/90.

When the fusion protein composition produced by the transformant carrying the introduced gene encoding the fusion protein molecule is expressed in a soluble form in cells, the cells are recovered by centrifugation after the completion of culturing and suspended in an aqueous buffer, followed by disruption using a sonicator, French press, Manton Gaulin homogenizer, Dynomill or the like to obtain a cell-free extract. A purified preparation of the fusion protein composition can be obtained by centrifuging the cell-free extract to obtain the supernatant and then subjecting the supernatant to ordinary means for isolating and purifying enzymes, e.g., extraction with a solvent, salting-out with ammonium sulfate, *etc.*, desalting, precipitation with an organic solvent, anion exchange chromatography using resins such as diethylaminoethyl (DEAE)-Sepharose and DIAION HPA-75 (manufactured by Mitsubishi Chemical Corporation), cation exchange chromatography using resins such as S-Sepharose FF (manufactured by Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the fusion protein composition is expressed as an inclusion body in cells, the cells are similarly recovered and disrupted, followed by centrifugation to recover the inclusion body of the fusion protein composition as a precipitate fraction. The recovered inclusion body of the fusion protein composition is solubilized with a protein-denaturing agent. The solubilized solution is diluted or dialyzed, whereby the fusion protein composition is renatured to have normal conformation. Then, a purified preparation of the fusion protein composition can be obtained by the same isolation and purification steps as described above.

When the fusion protein composition is extracellularly secreted, the fusion protein composition or its derivative can be recovered in the culture supernatant. That is, the culture is treated by the same means as above, e.g., centrifugation, to obtain the culture supernatant. A purified preparation of the fusion protein composition can be obtained from the culture supernatant by using the same isolation and purification methods as described above.

The methods for producing the fusion protein composition of the present invention are specifically described below.

(1) Construction of a vector for expression of fusion protein

A vector for expression of fusion protein is an expression vector for animal cells carrying inserted genes encoding CH and the like of a human antibody, which can be constructed by cloning each of the genes encoding CH and the like of a human antibody into an expression vector for animal cells.

The C regions of a human antibody may be CH and CL of any human antibody. Examples of the C regions include the C region of IgG1 subclass human antibody H chain (hereinafter referred to as hCγ1), the C region of κ class human antibody L chain (hereinafter referred to as hCκ) and the like.

As the genes encoding CH and CL of a human antibody, a genomic DNA consisting of exons and introns can be used. Also useful is a cDNA.

As the expression vector for animal cells, expression vector capable of inserting and expressing the gene encoding the constant region of a human antibody can be used. Suitable vectors include pAGE107 [*Cytotechnology,* 3, 133 (1990)], pAGE103 [*J. Biochem.,* 101, 1307 (1987)], pHSG274 [*Gene,* 27, 223 (1984)], pKCR [*Proc. Natl. Acad. Sci. USA,* 78, 1527 (1981)], pSG1βd2-4 [*Cytotechnology,* 4, 173 (1990)] and the like. Examples of the promoter and enhancer for use in the expression vector for animal cells include SV40 early promoter and enhancer [*J. Biochem.,* 101, 1307 (1987)], LTR of Moloney mouse leukemia virus [*Biochem. Biophys. Res. Commun.,* 149, 960 (1987)], immunoglobulin H chain promoter [*Cell,* 41, 479 (1985)] and enhancer [*Cell,* 33, 717 (1983)] and the like.

As the vector for expression of fusion protein, the vector which fits the form of the fusion protein to be expressed may be used. For example, when both antibody H chain and L chain in the Fc region of an antibody is used, either of the type in which antibody H chain and L chain exist on separate vectors or of the type in which both exist on the same vector (hereinafter referred to as tandem-type) may be used. The tandem-type ones are preferred in view of the easiness of construction of the fusion protein expression vector, the easiness of introduction into animal cells, the equilibrium of balance between the expression of fusion protein H chain and that of L chain in animal cells, *etc.* [*J. Immunol. Methods,* 167, 271 (1994)]. Examples of the tandem-type fusion protein expression vectors include pKANTEX93 [*Mol. Immunol.,* 37, 1035 (2000)], pEE18 [*Hybridoma,* 17, 559 (1998)] and the like.

The constructed vector for expression of fusion protein can be used for the expression of a fusion protein of

the present invention.

(2) Obtaining of cDNA encoding binding protein

cDNAs encoding an binding protein can be obtained in the following manner.

For example, when the binding protein is a single-chain antibody, a cDNA is synthesized using, as a template, an mRNA extracted from a hybridoma cell producing an antibody. The synthesized cDNA is inserted into a vector such as a phage or a plasmid to prepare a cDNA library. A recombinant phage or a recombinant plasmid carrying a cDNA encoding VH and a recombinant phage or a recombinant plasmid carrying a cDNA encoding VL are isolated from the cDNA library using DNA encoding the C region or V region of a known mouse antibody as a probe. The entire nucleotide sequences of VH and VL of the desired mouse antibody on the recombinant phages or recombinant plasmids are determined, and the whole amino acid sequences of VH and VL are deduced from the nucleotide sequences.

Also, when the binding protein is a proteinous ligand or a soluble receptor, a cDNA can be obtained from a cell line or a tissue which is known to express the binding protein in the same manner as described above.

The methods for preparing total RNA from a cell or tissue include the guanidine thiocyanate-cesium trifluoroacetate method [*Methods in Enzymol.,* 154, 3 (1987)], and the methods for preparing mRNA from the total RNA include the oligo (dT) immobilized cellulose column method (*Molecular Cloning: A Laboratory Manual,* Cold Spring Harbor Lab. Press New York, 1989) and the like. Examples of the kits for preparing mRNA from a hybridoma cell include Fast Track mRNA Isolation Kit (Invitrogen), Quick Prep mRNA Purification Kit (manufactured by Pharmacia) and the like.

The methods for synthesizing the cDNA and preparing the cDNA library include conventional methods (*Molecular Cloning: A Laboratory Manual,* Cold Spring Harbor Lab. Press New York, 1989; *Current Protocols in Molecular Biology,* Supplement 1-34), or methods using commercially available kits such as SuperScript™ Plasmid System for cDNA Synthesis and Plasmid Cloning (manufactured by GIBCO BRL) and ZAP-cDNA Synthesis Kit (manufactured by Stratagene).

In preparing the cDNA library, the vector for inserting the cDNA synthesized using the mRNA extracted from a hybridoma cell as a template may be any vector so long as the cDNA can be inserted. Examples of suitable vectors include ZAP Express [*Strategies,* 5, 58 (1992)], pBluescript II SK(+) [*Nucleic Acids Research,* 17, 9494 (1989)], λZAP II (manufactured by Stratagene), λgt10, λgt11 [*DNA Cloning: A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* for introducing the cDNA library constructed with a phage or a plasmid vector, any *Escherichia coli* can be used, so long as the cDNA library can be introduced, expressed and maintained. Examples of suitable *Escherichia coli* include XL1-Blue MRF' [*Strategies,* 5, 81 (1992)], C600 [Genetics, 39, 440 (1954)], Y1088, Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

The methods for selecting the cDNA clone encoding a desired binding protein from the cDNA library include colony hybridization or plaque hybridization (*Molecular Cloning: A Laboratory Manual,* Cold Spring Harbor Lab. Press New York, 1989) using an isotope- or fluorescence-labeled probe. It is also possible to prepare the cDNAs encoding the desired binding protein by preparing primers and performing PCR (*Molecular Cloning: A Laboratory Manual,* Cold Spring Harbor Lab. Press New York, 1989; *Current Protocols in Molecular Biology,* Supplement 1-34) using the cDNA or cDNA library as a template.

The nucleotide sequences of the cDNAs selected by the above methods can be determined by cleaving the cDNAs with appropriate restriction enzymes, cloning the fragments into a plasmid such as pBluescript SK(-) (manufactured by Stratagene), and then analyzing the sequences by generally employed sequencing methods such as the dideoxy method of Sanger, *et al.* [*Proc. Natl. Acad. Sci. USA,* 74, 5463 (1977)] or by using nucleotide sequencers such as ABI PRISM 377 DNA Sequencer (manufactured by Applied Biosystems).

Furthermore, when the amino acid sequence of a binding protein or the nucleotide sequence of a DNA encoding the binding protein is known, it can be produced by the following method.

When the amino acid sequence is known, the desired DNA can be obtained by designing a DNA sequence encoding the variable region taking into consideration the frequency of occurrence of codons (*Sequences of Proteins ofimmunological Interest,* US Dept. Health and Human Services, 1991), synthesizing several synthetic DNAs constituting of approximately 100-nucleotides based on the designed DNA sequence, and carrying out PCR using the synthetic DNAs. When the nucleotide sequence is known, the desired DNA can be obtained by synthesizing several synthetic DNAs constituting of approximately 100-nucleotides based on the nucleotide sequence information and carrying out PCR using the synthetic DNAs.

(3) Analysis of the amino acid sequence of binding fusion protein

Whether the obtained cDNA includes the full length of the desired binding protein can be confirmed by deducing the whole amino acid sequence of the binding protein based on the determined nucleotide sequence and comparing it with the whole amino acid sequence of the binding protein (*Sequences of Proteins of Immunological Interest,* US Dept. Health and Human Services, 1991) using a known data base (GenBank, Swiss Prott).

(4) Construction of a cDNA encoding a fusion protein

A cDNA encoding a fusion protein can be constructed in the following manner. First, a primary amino acid sequence is designed according to a fusion protein to be constructed. The constructed amino acid sequence is converted to a DNA sequence by taking codon usage into consideration. Based on the converted DNA sequence, the desired DNA sequence can be constructed by designing and synthesizing several synthetic DNAs having a length of about 100 nucleotides and ligating them by PCR.

Based on the form of the fusion protein, a desired fusion protein expression vector can be constructed by producing only a cDNA encoding the binding protein by the above method and introducing it into an expression vector having a cDNA encoding an antibody constant region. Also, a desired fusion protein expression vector can be constructed by construction of a cDNA in the linking form of the binding protein and the antibody Fc region by the above method and introducing it into a site downstream of a promoter of an appropriate expression vector.

(5) Construction of a fusion protein expression vector

A fusion protein expression vector can be constructed by inserting the cDNA encoding the fusion protein constructed in the above 2 (4) into a site upstream of the genes encoding CH or the like of a human antibody in the vector for fusion protein expression described in the above 2 (1). For example, a fusion protein expression vector can be constructed by introducing recognition sequences for appropriate restriction enzymes to the 5'-terminals of synthetic DNAs present on both ends among the synthetic DNAs used for constructing the fusion protein in the above 2 (4), and inserting them into sites upstream of the genes encoding CH and CL of a human antibody in the vector for expression of humanized antibody described in the above 2 (1). In this case, if necessary, an expression vector can also be obtained by remaining only a region encoding a desired amino acid sequence in the gene encoding CH or CL of a human antibody.

(6) Stable production of a fusion protein

Transformants capable of stably producing a fusion protein can be obtained by introducing the fusion protein expression vectors described in the above 2 (4) and (5) into appropriate animal cells.

Introduction of the fusion protein expression vector into an animal cell can be carried out by electroporation [Japanese Published Unexamined Patent Application No. 257891/90; *Cytotechnology,* 3, 133 (1990)], *etc.*

As the animal cell for introducing the fusion protein expression vector, any animal cell capable of producing a fusion protein can be used.

Examples of the animal cells include mouse myeloma cell lines NS0 and SP2/0, Chinese hamster ovary cell lines CHO/dhfr- and CHO/DG44, rat myeloma cell lines YB2/0 and IR983F, Syrian hamster kidney-derived BHK cell line, human myeloma cell line Namalwa and the like. Preferred are Chinese hamster ovary cell line CHO/DG44 and rat myeloma cell line YB2/0.

After the introduction of the fusion protein expression vector, the transformant capable of stably producing the fusion protein can be selected using a medium for animal cell culture containing a compound such as G418 sulfate (hereinafter referred to as G418; manufactured by SIGMA) according to the method described in Japanese Published Unexamined Patent Application No. 257891/90. Examples of the media for animal cell culture include RPMI1640 medium (manufactured by Nissui Pharmaceutical Co., Ltd.), GIT medium (manufactured by Nihon Pharmaceutical Co., Ltd.), EX-CELL 302 medium (manufactured by JRH), IMDM medium (manufactured by GIBCO BRL), Hybridoma-SFM medium (manufactured by GIBCO BRL), media prepared by adding various additives such as fetal calf serum (hereinafter referred to as FCS) to these media and the like. By culturing the obtained transformant in the medium, the fusion protein can be formed and accumulated in the culture supernatant. The amount and the antigen-binding activity of the fusion protein produced in the culture supernatant can be measured by enzyme-linked immunosorbent assay (hereinafter referred to as ELISA; *Antibodies: A Laboratory Manual,* Cold Spring Harbor Laboratory, Chapter 14, 1998; *Monoclonal Antibodies: Principles and Practice,* Academic Press Limited, 1996) or the like. The production of the fusion protein by the transformant can be increased by utilizing a DHFR gene amplification system or the like according to the method described in Japanese Published Unexamined Patent Application No. 257891/90.

The fusion protein can be purified from the culture supernatant of the transformant using a protein A column (*Antibodies: A Laboratory Manual,* Cold Spring Harbor Laboratory, Chapter 8, 1988; *Monoclonal Antibodies: Principles and Practice,* Academic Press Limited, 1996). In addition, purification methods generally employed for the purification of proteins can also be used. For example, the purification can be carried out by combinations of gel filtration, ion exchange chromatography, ultrafiltration and the like. The molecular weight of the purified humanized fusion protein can be measured by SDS-denatured polyacrylamide gel electrophoresis [hereinafter referred to as SDS-PAGE; *Nature,* 227, 680 (1970)], Western blotting (*Antibodies: A Laboratory Manual,* Cold Spring Harbor Laboratory, Chapter 12, 1988; *Monoclonal Antibodies: Principles and Practice,* Academic Press Limited, 1996), *etc.*

Shown above is the method for producing the fusion protein composition using an animal cell as the host. As described above, the fusion protein composition can also be produced by using a yeast cell, an insect cell, a plant cell, an animal or a plant by similar methods.

When a host cell inherently has the ability to express the fusion protein composition, the fusion protein composition of the present invention can be produced by preparing a cell expressing the fusion protein composition using the method described in the above 1, culturing the cell, and then purifying the desired fusion protein composition from the culture.

3. Evaluation of activity of the fusion protein composition

As methods for measuring the protein amount of the purified fusion protein composition, the binding activity to an antigen and ADCC activity, known methods described in *Antibodies: A Laboratory Manual,* Cold Spring Harbor Laboratory, Chapter 12 (1988), *Monoclonal Antibodies: Principles and Practice,* Academic Press Limited (1996) and the like can be used.

As an example, when the fusion protein composition is a fusion protein, binding activity for an antigen and binding activity for an antigen-positive cultured cell line can be measured by the ELISA, fluorescent antibody technique [*Cancer Immunol. Immunother.,* 36, 373 (1993)] and the like. The cytotoxic activity against an antigen-positive cultured cell line can be evaluated by measuring CDC activity, ADCC activity and the like [*Cancer Immunol. Immunother.,* 36, 373 (1993)].

It is considered that the ADCC activity is generated as a result of the activation of effector cells such as NK cell, neutrophil, monocyte and macrophage, and among them, the NK cell is particularly taking the main role [*Blood,* 76, 2421 (1990), *Trends in Immunol.,* 22, 633 (2001), *Int. Rev. Immunol.,* 20, 503 (2001)].

Since the FcγR expressing on the NK cell is FcγRIIIa, and the ADCC activity of antibody correlates therefore with the strength of binding ability to FcγIIIa, the ADCC activity possessed by a fusion protein composition can be estimated from the binding ability of the fusion protein composition for FcγIIIa. As the method for measuring binding ability of a fusion protein composition for FcγIIIa, it can be measured by a method analogous to the ELISA [*Antibodies: A Laboratory Manual,* Cold Spring Harbor Laboratory, Chapter 14 (1998), *Monoclonal Antibodies: Principles and Practice,* Academic Press Limited (1996)].

Specifically, binding ability of a fusion protein composition for FcγIIIa can be evaluated by a method in which a fusion protein composition is incubated with FcγIIIa immobilized on an ELISA plate, and subsequently detecting the fusion protein composition bound to FcγIIIa, or by a method in which a fusion protein composition antibody is allowed to bind to a substrate such as an antigen immobilized on an ELISA plate, and subsequently allowing labeled FcγIIIa to react with the fusion protein composition bound to the substrate such as an antigen and detecting it.

The FcγIIIa can be obtained by preparing cDNA from human peripheral blood or the like by the method described in the above-described item 1, and integrating it into an appropriate expression vector. When FcγIIIa is expressed, it can be labeled by fusing with an appropriate tag molecule.

The safety and therapeutic effect of the fusion protein composition in human can be evaluated using an appropriate animal model of a kinds relatively close to human, e.g., cynomolgus monkey.

4. Analysis of sugar chains in the fusion protein composition

The sugar chain structure of fusion protein composition expressed in various cells can be analyzed according to general methods of analysis of the sugar chain structure of glycoproteins. For example, a sugar chain bound to the fusion protein molecule consists of neutral sugars such as galactose, mannose and fucose, amino sugars such as N-acetylglucosamine, and acidic sugars such as sialic acid, and can be analyzed by methods such as sugar composition analysis and sugar chain structure analysis using two-dimensional sugar chain mapping and the like.

(1) Analysis of neutral sugar and amino sugar compositions

The sugar chain composition of a fusion protein composition can be analyzed by carrying out acid hydrolysis of sugar chains with trifluoroacetic acid or the like to release neutral sugars or amino sugars and analyzing the composition ratio.

Specifically, the analysis can be carried out by a method using a carbohydrate analysis system (BioLC; product of Dionex). BioLC is a system for analyzing the sugar composition by HPAEC-PAD (high performance anion-exchange chromatography-pulsed amperometric detection) [*J. Liq. Chromatogr.,* 6, 1577 (1983)].

The composition ratio can also be analyzed by the fluorescence labeling method using 2-aminopyridine. Specifically, the composition ratio can be calculated by fluorescence labeling an acid-hydrolyzed sample by 2-aminopyridylation according to a known method [*Agric. Biol. Chem.,* 55(1), 283 (1991)] and then analyzing the composition by HPLC.

(2) Analysis of sugar chain structure

The sugar chain structure of a fusion protein molecule can be analyzed by two-dimensional sugar chain mapping [*Anal. Biochem.,* 171, 73 (1988); *Seibutsukagaku Jikkenho (Biochemical Experimentation Methods) 23 - Totanpakushitsu Tosa Kenkyuho (Methods of Studies on Glycoprotein Sugar Chains),* Gakkai Shuppan Center, edited by Reiko Takahashi (1989)]. The two-dimensional sugar chain mapping is a method of deducing a sugar chain structure, for example, by plotting the retention time or elution position of a sugar chain by reversed phase chromatography as the X axis and the retention time or elution position of the sugar chain by normal phase chromatography as the Y axis, and comparing them with the results on known sugar chains.

Specifically, a sugar chain is released from a fusion protein composition by hydrazinolysis of the fusion protein composition and subjected to fluorescence labeling with 2-aminopyridine (hereinafter referred to as "PA") [*J. Biochem.,* 95, 197 (1984)]. After being separated from an excess PA-treating reagent by gel filtration, the sugar chain is subjected to reversed phase chromatography. Then, each peak of the sugar chain is subjected to normal phase chromatography. The sugar chain structure can be deduced by plotting the obtained results on a two-dimensional sugar chain map and comparing them with the spots of a sugar chain standard (manufactured by Takara Shuzo Co., Ltd.) or those in the literature [*Anal. Biochem.,* 171, 73 (1988)].

The structure deduced by the two-dimensional sugar chain mapping can be confirmed by carrying out mass spectrometry, e.g., MALDI-TOF-MS, of each sugar chain.

5. Immunoassay for determining the sugar chain structure of fusion protein molecule

A fusion protein composition comprises a fusion protein molecule having different sugar chain structures binding to the Fc region of a fusion protein. The fusion protein composition of the present invention, in which the ratio of a sugar chains in which fucose is not bound to the N-acetylglucosamine in the reducing end to the total complex type N-glycoside-linked sugar chains bound to the Fc region is 100%, has high ADCC activity. Such a fusion protein composition can be identified using the method for analyzing the sugar chain structure of a fusion protein composition described in the above 4. Furthermore, it can also be identified by immunoassays using lectins.

Discrimination of the sugar chain structure of a fusion protein molecule by immunoassays using lectins can be made according to the immunoassays such as Western staining, RIA (radioimmunoassay), VIA (viroimmunoassay), EIA (enzymoimmunoassay), FIA (fluoroimmunoassay) and MIA (metalloimmunoassay) described in the literature [*Monoclonal Antibodies: Principles and Applications,* Wiley-Liss, Inc. (1995); *Enzyme Immunoassay,* 3rd Ed., Igaku Shoin (1987); *Enzyme Antibody Technique,* Revised Edition, Gakusai Kikaku (1985); *etc.*], for example, in the following manner.

A lectin recognizing the sugar chain structure of a fusion protein molecule constituting a fusion protein composition is labeled, and the labeled lectin is subjected to reaction with a sample fusion protein composition, followed by measurement of the amount of a complex of the labeled lectin with the fusion protein molecule.

Examples of lectins useful for determining the sugar chain structure of an fusion protein molecule include WGA (wheat-germ agglutinin derived from *T. vulgaris*), ConA (concanavalin A derived from *C. ensiformis*), RIC (a toxin derived from *R. communis*), L-PHA (leukoagglutinin derived from *P. vulgaris*), LCA (lentil agglutinin derived from *L. culinaris*), PSA (pea lectin derived from *P. sativum*), AAL (*Aleuria aurantia* lectin), ACL (*Amaranthus caudatus* lectin), BPL (*Bauhinia purpurea* lectin), DSL (*Datura stramonium* lectin), DBA (*Dolichos biflorus* agglutinin), EBL (elderberry balk lectin), ECL (*Erythrina cristagalli* lectin), EEL (*Euonymus europaeus* lectin), GNL (*Galanthus nivalis* lectin), GSL (*Griffonia simplicifolia* lectin), HPA (*Helix pomatia* agglutinin), HHL (*Hippeastrum* hybrid lectin), Jacalin, LTL (*Lotus tetragonolobus* lectin), LEL (*Lycopersicon esculentum* lectin), MAL (*Maackia amurensis* lectin), MPL (*Maclura pomifera* lectin), NPL (*Narcissus pseudonarcissus* lectin), PNA (peanut agglutinin), E-PHA (*Phaseolus vulgaris* erythroagglutinin), PTL (*Psophocarpus tetragonolobus* lectin), RCA (*Ricinus communis* agglutinin), STL (*Solanum tuberosum* lectin), SJA (*Sophora japonica* agglutinin), SBA (soybean agglutinin), UEA (*Ulex europaeus* agglutinin), WL (*Vicia villosa* lectin), WFA (*Wisteria floribunda* agglutinin) and the like.

It is preferred to use lectins specifically recognizing a sugar chain structure wherein fucose is bound to the N-acetylglucosamine in the reducing end in complex type N-glycoside-linked sugar chains. Examples of such lectins include lentil lectin LCA (lentil agglutinin derived from *Lens culinaris*), pea lectin PSA (pea lectin derived from *Pisum sativum*),

broad bean lectin VFA (agglutinin derived from *Vicia faba*), *Aleuria aurantia* lectin AAL (lectin derived from *Aleuria aurantia*) and the like.

6. Utilization of the fusion protein composition of the present invention

The fusion protein composition of the present invention has high ADCC activity. A fusion protein having high ADCC activity is useful in preventing and treating various diseases such as a tumor, an inflammatory disease, immune diseases such as autoimmune disease and allergy, a circulatory organ disease, a disease which accompanies microbial infection and the like.

The tumor includes malignant tumors of, for example, acute leukemia such as acute lymphocytic leukemia and acute myelocytic leukemia; T cellular tumors such as lymphomatosis, adult T cell leukemia and lymphomatosis, and NK/T cellular lymphomatosis; leukemia such as chronic leukemia; blood tumors and cancer such as myeloma, Hodgkin disease, non-Hodgkin lymphoma and multiple myeloma; and the like.

The inflammatory disease includes inflammatory diseases of, for example, acute or chronic airway oversensitivity and bronchial asthma, atopic skin diseases including atopic dermatitis, inflammatory diseases such as allergic rhinitis and pollinosis, chronic sinusitis, Churg-Strauss syndrome, inflammatory bowel diseases such as Crohn disease and ulcerative colitis, and the like.

The autoimmune disease includes rheumatoid arthritis, juvenile rheumatoid arthritis, psoriasis, psoriatic arthritis, ankylosing spondylitis, systemic lupus erythematosus, Sjogren syndrome, systemic sclerosis, polymyositis, Guillain-Barre syndrome, idiopathic thrombocytopenic purpura, autoimmune hemolytic anemia, a disease caused by an immune system abnormality in which antigen presentation by memory T cell is concerned. The memory T cell means an activated T cell mainly showing CD45 RO-positive and represents a group of cells which activate an immune system by receiving information on an antigen from an antigen presenting cell (APC).

The circulatory organ disease includes arteriosclerosis, ischemic heart disease, valvular disease of heart, hypertension, stroke, renal insufficiency, aortic aneurysm, arteriosclerosis obliterans, primary pulmonary hypertension and the like.

The disease which accompanies microbial infection includes viral infections caused by infection with human T cell virus type I (HTLV-I) of retrovirus, hepatitis virus, Epstein-Barr (EB) virus, Kapossi sarcoma related virus, hepatitis virus and the like, bacterial infections caused by infection with staphylococcus, streptococcus, pneumococcus and the like, fungal infections caused by infection with *Trichophyton,* and the like.

Since the fusion protein composition of the present invention possesses high cytotoxic activity, it can be used in treating patients of the above-described various diseases such as a tumor, an inflammatory disease, immune diseases such as autoimmune disease and allergy, a circulatory organ disease, a disease which accompanies microbial infection and the like, which cannot be cured by the conventional fusion protein compositions.

An antibody which recognizes a tumor-related antigen, an antibody which recognizes an allergy- or inflammation-related antigen, an antibody which recognizes cardiovascular disease-related antigen, an antibody which recognizes autoimmune disease-related antigen and an antibody which recognizes a viral or bacterial infection-related antigen which are used as a binding fragment of an antibody as the binding protein are described below.

The antibody which recognizes a tumor-related antigen includes anti-GD2 antibody [*Anticancer Res.,* 13, 331 (1993)], anti-GD3 antibody [*Cancer Immunol. Immunother.,* 36, 260 (1993)], anti-GM2 antibody [*Cancer Res.,* 54, 1511 (1994)], anti-HER2 antibody [*Proc. Natl. Acad. Sci. USA,* 89, 4285 (1992)], anti-CD52 antibody [*Nature,* 332, 323 (1998)], anti-MAGE antibody [*British J. Cancer,* 83, 493 (2000)], anti-HM1.24 antibody *[Molecular Immunol.,* 36, 387 (1999)], anti-parathyroid hormone-related protein (PTHrP) antibody [*Cancer,* 88, 2909 (2000)], anti-FGF8 antibody [*Proc. Natl. Acad. Sci. USA,* 86, 9911 (1989)], anti-basic fibroblast growth factor antibody, anti-FGF8 receptor antibody [*J. Biol. Chem.,* 265, 16455-16463 (1990)], anti-basic fibroblast growth factor receptor antibody, anti-insulin-like growth factor antibody [*J. Neurosci. Res.,* 40, 647 (1995)], anti-insulin-like growth factor receptor antibody [*J. Neurosci. Res.,* 40, 647 (1995)], anti-PMSA antibody [*J. Urology,* 160, 2396 (1998)], anti-vascular endothelial cell growth factor antibody [*Cancer Res.,* 57, 4593 (1997)], anti-vascular endothelial cell growth factor receptor antibody [*Oncogene,* 19, 2138 (2000)] and the like.

The antibody which recognizes an allergy- or inflammation-related antigen includes anti-interleukin 6 antibody [*Immunol. Rev.,* 127, 5 (1992)], anti-interleukin 6 receptor antibody [*Molecular Immunol.,* 31, 371 (1994)], anti-interleukin 5 antibody [*Immunol. Rev.,* 127, 5 (1992)], anti-interleukin 5 receptor antibody, anti-interleukin 4 antibody [*Cytokine,* 3, 562 (1991)], anti-interleukin 4 receptor antibody [*J. Immunol. Meth.,* 217, 41 (1998)], anti-tumor necrosis factor antibody [*Hybridoma,* 13, 183 (1994)], anti-tumor necrosis factor receptor antibody [*Molecular Pharmacol.,* 58, 237 (2000)], anti-CCR4 antibody [*Nature,* 400, 776 (1999)], anti-chemokine antibody [*J. Immuno. Meth.,* 174, 249 (1994)], anti-chemokine receptor antibody [*J. Exp. Med.,* 186, 1373 (1997)] and the like.

The antibody which recognizes a cardiovascular disease-related antigen includes anti-GpIIb/IIIa antibody [*J. Immunol.,* 152, 2968 (1994)], anti-platelet-derived growth factor antibody [*Science,* 253, 1129 (1991)], anti-platelet-derived growth factor receptor antibody [*J. Biol. Chem.,* 272, 17400 (1997)], anti-blood coagulation factor antibody [*Circulation,* 101, 1158 (2000)] and the like.

The antibody which recognizes an antigen relating to autoimmune diseases includes an anti-auto-DNA antibody [*Immunol. Letters,* 72, 61 (2000)] and the like.

The antibody which recognizes a viral or bacterial infection-related antigen includes anti-gp120 antibody [*Structure,* 8, 385 (2000)], anti-CD4 antibody [*J. Rheumatology,* 25, 2065 (1998)], anti-CCR5 antibody, anti-Vero toxin antibody [*J. Clin. Microbiol.,* 37, 396 (1999)] and the like.

These antibodies can be obtained from public organizations such as ATCC (The American Type Culture Collection), RIKEN Gene Bank at The Institute of Physical and Chemical Research, and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

Specific examples of the fusion antibody of the binding protein other than the above antibodies and the antibody Fc region of the present invention are described below.

Examples of the Fc fusion protein of the binding protein relating to an inflammatory disease, immune diseases such as autoimmune disease and allergy include etanercept which is an Fc fusion protein of sTNFRII (USP 5605690), alefacept which is an Fc fusion protein of LFA-3 expressed on antigen presenting cells (USP 5914111), an Fc fusion protein of cytotoxic T lymphocyte-associated antigen-4 (CTLA-4) [*J. Exp. Med.,* 181, 1869 (1995)], an Fc fusion protein of interleukin-15 [*J. Immunol.,* 160, 5742 (1998)], an Fc fusion protein of Factor VII [*Proc. Natl. Acad. Sci. USA,* 98, 12180 (2001)], an Fc fusion protein of interleukin-10 [*J. Immunol.,* 154, 5590 (1995)], an Fc fusion protein of interleukin-2 [*J. Immunol.,* 146, 915 (1991)], an Fc fusion protein of CD40 [*Surgery.* 132, 149 (2002)], an Fc fusion protein of Flt-3 (fms-like tyrosine kinase) [*Acta. Haemato.,* 95, 218 (1996)], an Fc fusion protein of OX40 [J. Leu. Biol., 72, 522 (2002)] and the like. In addition to them, a large number of fusion proteins of various human CD molecules [CD2, CD30 (TNFRSF8), CD95 (Fas), CD106 (VCAM-1), CD137], adhesion molecules [ALCAM (activated leukocyte cell adhesion molecule), Cadherins, ICAM (Intercellular adhesion molecule)-1, ICAM-2, ICAM-3], cytokine receptors (hereinafter the receptor is referred to as "R") (IL-4R, IL-5R, IL-6R, IL-9R, IL-10R, IL-12R, IL-13R$\alpha$1, IL-13R$\alpha$2, IL-15R, IL-21R), chemokine, cell death-inducing signal molecules [B7-H1, DR6 (death receptor 6), PD-1 (programmed death-1), TRAIL R1], costimulation molecules [B7-1, B7-2, B7-H2, ICOS (inducible costimulator)], growth factors (ErbB2, ErbB3, ErbB4, HGFR), differentiation-inducing factors (B7-H3), activation factors (NKG2D), signaling factors (gp130) and receptors or ligands of the binding proteins with an antibody Fc region have been reported.

A pharmaceutical composition comprising the fusion protein composition obtained in the present invention may be administered alone as a therapeutic agent. However, it is preferably mixed with one or more pharmaceutically acceptable carriers and provided as a pharmaceutical preparation produced by an arbitrary method well known in the technical field of pharmaceutics.

It is desirable to administer the pharmaceutical composition by the route that is most effective for the treatment. Suitable administration routes include oral administration and parenteral administration such as intraoral administration, intratracheal administration, intrarectal administration, subcutaneous administration, intramuscular administration and intravenous administration. In the case of a protein preparation, intravenous administration is preferable.

The pharmaceutical preparation may be in the form of spray, capsules, tablets, granules, syrup, emulsion, suppository, injection, ointment, tape, and the like.

The pharmaceutical preparations suitable for oral administration include emulsions, syrups, capsules, tablets, powders, granules and the like.

Liquid preparations such as emulsions and syrups can be prepared using, as additives, water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), flavors (e.g., strawberry flavor and peppermint), and the like.

Capsules, tablets, powders, granules, *etc.* can be prepared using, as additives, excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

The pharmaceutical preparations suitable for parenteral administration include injections, suppositories, sprays and the like.

Injections can be prepared using carriers comprising a salt solution, a glucose solution, or a mixture thereof, *etc.* It is also possible to prepare powder injections by freeze-drying the Fc fusion protein composition according to a conventional method and adding sodium chloride thereto.

Suppositories can be prepared using carriers such as cacao butter, hydrogenated fat and carboxylic acid.

The Fc fusion protein composition may be administered as such in the form of spray, but sprays may be prepared using carriers which do not stimulate the oral or airway mucous membrane of a recipient and which can disperse the Fc fusion protein composition as fine particles to facilitate absorption thereof.

Suitable carriers include lactose, glycerin and the like. It is also possible to prepare aerosols, dry powders, *etc.*

according to the properties of the Fc fusion protein composition and the carriers used. In preparing these parenteral preparations, the above-mentioned additives for the oral preparations may also be added.

The dose and administration frequency will vary depending on the desired therapeutic effect, the administration route, the period of treatment, the patient's age and body weight, *etc.* However, an appropriate dose of the active ingredient for an adult person is generally 10 μg/kg to 20 mg/kg per day.

The anti-tumor effect of the Fc fusion protein composition against various tumor cells can be examined by *in vitro* tests such as CDC activity measurement and ADCC activity measurement and in vivo tests such as anti-tumor experiments using tumor systems in experimental animals (e.g., mice).

The CDC activity and ADCC activity measurements and anti-tumor experiments can be carried out according to the methods described in the literature [*Cancer Immunology Immunotherapy,* 36, 373 (1993); *Cancer Research,* 54, 1511 (1994); *etc.*].

Brief Description of the Drawings

Fig. 1 shows the steps for constructing plasmid pKOFUT8Neo.

Fig. 2 shows the result of genomic Southern hybridization analysis of a hemi-knockout clone wherein one copy of the FUT8 allele was disrupted in CHO/DG44 cell. The lanes respectively show the following, from left to right: molecular weight marker, hemi-knockout clone 50-10-104, and parent cell CHO/DG44.

Fig. 3 shows the result of genomic Southern hybridization analysis of double-knockout clone WK704 wherein both FUT8 alleles were disrupted in CHO/DG44 cell. The arrow indicates the detection spot of a positive fragment resulting from homologous recombination.

Fig. 4 shows the result of genomic Southern analysis of a clone obtained by removing a drug-resistance gene from a double-knockout clone wherein both FUT8 alleles were disrupted in CHO/DG44 cell. The lanes respectively show the following, from left to right: molecular weight marker, drug resistance gene-removed double-knockout clone 4-5-C3, double-knockout clone WK704, hemi-knockout clone 50-10-104, and parent cell CHO/DG44.

Fig. 5 shows a plasmid pBSIISK(-)/CC49VH.

Fig. 6 shows a plasmid pBSIISK(-)/CC49VL.

Fig. 7 shows a plasmid pKANTEX93/CC49scFv-Fc.

Fig. 8 shows SDS-PAGE electrophoresis patterns of purified anti-TAG-72 scFv-Fc(-) and anti-TAG-72 scFv-Fc(+) under reducing conditions and non-reducing conditions. Staining of protein was carried out using Coomassie Brilliant Blue (CBB).

Fig. 9 shows binding activities of anti-TAG-72 scFv-Fc for Jurkat cell by fluorescent antibody technique. The abscissa shows fluorescence intensity, and the ordinate shows the number of cells. A shows a result with anti-TAG-72 scFv-Fc(+), B shows that with anti-TAG-72 scFv-Fc(-), and C shows that with KM8404 as the negative control. 1 shows a result of no antibody addition, 2 shows that of antibody concentration 50 μg/ml, and 3 shows that of antibody concentration 2 μg/ml.

Fig. 10 shows binding activities of anti-TAG-72 scFv-Fc for TAG-72 antigen, measured by ELISA. The abscissa shows sample concentration, and the ordinate shows absorbance at each sample concentration. Closed circles show anti-TAG-72 scFv-Fc(-), open circles show anti-TAG-72 scFv-Fc(+), and open triangles show KM8404 as the negative control.

Fig. 11 shows binding activities of anti-TAG-72 scFv-Fc for shFcγRIIIa in the absence of antigen TAG-72. The abscissa shows sample concentration, and the ordinate shows absorbance at each sample concentration. Closed circles show anti-TAG-72 scFv-Fc(-) and open circles show anti-TAG-72 scFv-Fc(+). A shows a result with shFcγRIIIa (F), and B shows that with shFcγRIIIa(V).

Fig. 12 shows binding activities of anti-TAG-72 scFv-Fc for shFcγRIIIa in the presence of the antigen TAG-72. The abscissa shows sample concentration, and the ordinate shows absorbance at each sample concentration. Closed circles show anti-TAG-72 scFv-Fc(-) and open circles show anti-TAG-72 scFv-Fc(+). A shows a result with shFcγRIIIa (F), and B shows that with shFcγRIIIa(V).

Fig. 13 shows ADCC activities of anti-TAG-72 scFv-Fc for Jurkat cell and Raji cell. The abscissa shows sample concentration, and the ordinate shows ADCC activity (%) at each sample concentration. Closed circles show anti-TAG-72 scFv-Fc(-) and open circles show anti-TAG-72 scFv-Fc(+). A shows a result with Jurkat cell, and B that with Raji cell.

Fig. 14 shows a construction process of a plasmid pNUTS.

Fig. 15 shows a construction process of a plasmid pNUTS/scFvM-Fc.

Fig. 16 shows SDS-PAGE electrophoresis patterns of purified Fc fusion proteins of scFv under reducing conditions and non-reducing conditions. Staining of protein was carried out using Coomassie Brilliant Blue (CBB). Lane 1 shows anti-TAG-72 scFv-Fc(-), lane 2 shows anti-TAG-72 scFv-Fc(+), lane 3 shows anti-MUC1 scFv-Fc(-), lane 4 shows anti-MUC1 scFv-Fc(+), lane 5 shows anti-MUC1 anti-TAG-72 scFvM-scFvT-Fc(-), lane 6 shows anti-TAG-72 anti-MUC1 scFvM-scFvT-Fc(+), lane 7 shows anti-TAG-72 anti-MUC1 scFvT-scFvM-Fc(-) and lane 8 shows anti-MUC1 anti-TAG-72 scFvT-scFvM-Fc(+).

Fig. 17 shows binding activities of anti-MUC1 scFv-Fc for T47D cell or Raji cell, measured by fluorescent antibody technique. The abscissa shows fluorescence intensity, and the ordinate shows the number of cells. A shows a result for T47D cell, and B shows that for Raji cell. 1 shows a result with 50 $\mu$g/ml of anti-MUC1 scFv-Fc(-), 2 shows that with 50 $\mu$g/ml of anti-MUC1 scFv-Fc(+), and 3 shows that with no addition of scFv-Fc.

Fig. 18 shows binding activity for MUC1 as the antigen of anti-MUC1 scFv-Fc, measured by ELISA. The abscissa shows concentration of anti-MUC1 scFv-Fc fusion protein, and the ordinate shows absorbance. Closed circles show anti-MUC1 scFv-Fc(-), and open circles show anti-MUC1 scFv-Fc(+). A shows a result of using MUC1 as the antigen, and B shows that of using TAG-72 as the negative control antigen.

Fig. 19 shows binding activities of anti-MUC1 scFv-Fc for shFc$\gamma$RIIIa in the absence of the antigen MUC1. The abscissa shows concentration of anti-MUC1 scFv-Fc fusion protein, and the ordinate shows absorbance. Closed circles show anti-MUC1 scFv-Fc(-), and open circles show anti-MUC1 scFv-Fc(+). A shows a result with shFc$\gamma$RIIIa (V), and B shows that with shFc$\gamma$RIIIa(F).

Fig. 20 shows binding activities of anti-MUC1 scFv-Fc for shFc$\gamma$RIIIa in the presence of the antigen MUC1. The abscissa shows concentration of anti-MUC1 scFv-Fc fusion protein, and the ordinate shows absorbance. Closed circles show anti-MUC1 scFv-Fc(-), and open circles show anti-MUC1 scFv-Fc(+). A shows a result with shFc$\gamma$RIIIa (V), and B shows that with shFc$\gamma$RIIIa(F).

Fig. 21 shows ADCC activity of anti-MUC1 scFv-Fc. The abscissa shows sample concentration, and the ordinate shows ADCC activity at each sample concentration. Closed circles show anti-MUC1 scFv-Fc(-), and open circles show anti-MUC1 scFv-Fc(+). A shows a result with T47D cell, and B shows that with Raji cell.

Fig. 22 shows a construction process of a plasmid pNUTS/scFvM-scFvT-Fc.

Fig. 23 shows a construction process of a plasmid pNUTS/scFvT-scFvM-Fc.

Fig. 24 shows binding activities of scFv$_2$-Fc for Raji cell, Jurkat cell or T47D cell, measured by fluorescent antibody technique. The abscissa shows fluorescence intensity, and the ordinate shows the number of cells. A shows a result for Raji cell, B shows that for Jurkat cell, and C shows that for T47D. 1 shows a result with 75 $\mu$g/ml of scFvM-scFvT-Fc(-), and 2 shows that with 75 $\mu$g/ml of scFvM-scFvT-Fc(+), 3 shows that with 75 $\mu$g/ml of scFvT-scFvM-Fc(-), 4 shows that with 75 $\mu$g/ml of scFvT-scFvM-Fc(+), and 5 shows that with no addition of scFv$_2$-Fc.

Fig. 25 shows binding activities of scFv$_2$-Fc for TAG-72, measured by ELISA. The abscissa shows scFv$_2$-Fc concentration, and the ordinate shows absorbance at each scFv$_2$-Fc concentration. Closed triangles show anti-MUC1 anti-TAG-72 scFvM-scFvT-Fc(-), open triangles show anti-MUC1 anti-TAG-72 scFvM-scFvT-Fc(+), closed circles show anti-TAG-72 anti-MUC1 scFvT-scFvM-Fc(-), and open circles show anti-TAG-72 anti-MUC1 scFvT-scFvM-Fc(+). A shows a result with anti-TAG-72 anti-MUC1 scFvM-scFvT-Fc, and B shows that with anti-TAG-72 anti-MUC1 scFvT-scFvM-Fc.

Fig. 26 shows binding activities of scFv$_2$-Fc for MUC1, measured by ELISA. The abscissa shows scFv$_2$-Fc concentration, and the ordinate shows absorbance at each scFv$_2$-Fc concentration. Closed triangles show anti-MUC1 anti-TAG-72 scFvM-scFvT-Fc(-), open triangles show anti-MUC1 anti-TAG-72 scFvM-scFvT-Fc(+), closed circles show anti-TAG-72 anti-MUC1 scFvT-scFvM-Fc(-), and open circles show anti-TAG-72 anti-MUC1 scFvT-scFvM-Fc(+). A shows a result with anti-TAG-72 anti-MUC1 scFvM-scFvT-Fc, and B shows that with anti-TAG-72 anti-MUC1 scFvT-scFvM-Fc.

Fig. 27 shows binding activity of anti-MUC1 anti-TAG-72 scFvM-scFvT-Fc for shFc$\gamma$RIIIa. The abscissa shows sample concentration, and the ordinate shows absorbance at each sample concentration. Closed triangles show anti-MUC1 anti-TAG-72 scFvM-scFvT-Fc(-), and open triangles show anti-MUC1 anti-TAG-72 scFvM-scFvT-Fc(+). A shows a result with shFc$\gamma$RIIIa(V), and B shows that with shFc$\gamma$RIIIa(F).

Fig. 28 shows binding activity of anti-TAG-72 anti-MUC1 scFvT-scFvM-Fc for shFc$\gamma$RIIIa in the absence of the antigen. The abscissa shows sample concentration, and the ordinate shows absorbance at each sample concentration. Closed circles show anti-TAG-72 anti-MUC1 scFvT-scFvM-Fc(-), and open circles show anti-TAG-72 anti-MUC1 scFvT-scFvM-Fc(+). A shows a result with shFc$\gamma$RIIIa(V), and B shows that with shFc$\gamma$RIIIa(F).

Fig. 29 shows binding activities of scFv$_2$-Fc for shFc$\gamma$RIIIa(V) in the presence of the antigen. Closed triangles show anti-MUC1 anti-TAG-72 scFvM-scFvT-Fc(-), open triangles show anti-MUC1 anti-TAG-72 scFvM-scFvT-Fc(+), closed circles show anti-TAG-72 anti-MUC1 scFvT-scFvM-Fc(-), and open circles show anti-TAG-72 anti-MUC1 scFvT-scFvM-Fc(+). A shows a result in the presence of TAG-72, and B shows that in the presence of MUC1.

Fig. 30 shows ADCC activities of scFv$_2$-Fc for Jurkat cell. The abscissa shows scFv$_2$-Fc concentration, and the ordinate shows ADCC activity at each scFv$_2$-Fc concentration. Closed triangles in A of the drawing show anti-MUC1 anti-TAG-72 scFvM-scFvT-Fc(-), and open triangles in the same show anti-MUC1 anti-TAG-72 scFvM-scFvT-Fc (+), closed circles in B show anti-TAG-72 anti-MUC1 scFvT-scFvM-Fc(-), and open circles show anti-TAG-72 anti-MUC1 scFvT-scFvM-Fc(+).

Fig. 31 shows ADCC activities of scFv$_2$-Fc for T47D cell. The abscissa shows scFv$_2$-Fc concentration, and the ordinate shows ADCC activity at each scFv$_2$-Fc concentration. Closed triangles in A show anti-MUC1 anti-TAG-72 scFvM-scFvT-Fc(-), and open triangles show anti-MUC1 anti-TAG-72 scFvM-scFvT-Fc(+), closed circles in B show anti-TAG-72 anti-MUC1 scFvT-scFvM-Fc(-), and open circles show anti-TAG-72 anti-MUC1 scFvT-scFvM-Fc(+).

Fig. 32 shows ADCC activities of scFv$_2$-Fc for Raji cell. The abscissa shows scFv$_2$-Fc concentration, and the ordinate shows ADCC activity at each scFv$_2$-Fc concentration. Closed triangles in A show anti-MUC1 anti-TAG-72 scFvM-scFvT-Fc(-), and open triangles show anti-MUC1 anti-TAG-72 scFvM-scFvT-Fc(+), closed circles in B show anti-TAG-72 anti-MUC1 scFvT-scFvM-Fc(-), and open circles show anti-TAG-72 anti-MUC1 scFvT-scFvM-Fc(+).

Fig. 33 shows a plasmid pBsIISK(-)/sTNFRII-1.

Fig. 34 shows a plasmid pBsIISK(-)/sTNFRII-2.

Fig. 35 shows a plasmid pBsIISK(-)/sTNFRII-Fc.

Fig. 36 shows a plasmid pKANTEX93/sTNFRII-Fc.

Fig. 37 shows SDS-PAGE electrophoresis patterns of purified sTNFRII-Fc(-) and sTNFRII-Fc(+) under reducing conditions and non-reducing conditions. Staining of protein was carried out using Coomassie Brilliant Blue (CBB).

Fig. 38 shows binding activities of sTNFRII-Fc for anti-TNFRII antibody, measured by ELISA. The abscissa shows sTNFRII-Fc concentration, and the ordinate shows binding activity for anti-TNFRII antibody at each sTNFRII-Fc concentration. Open circles show sTNFRII-Fc(+), and closed circles show sTNFRII-Fc(-).

Fig. 39 shows binding activities of sTNFRII-Fc for shFcγRIIIa. The abscissa shows sTNFRII-Fc concentration, and the ordinate shows binding activity at each sTNFRII-Fc concentration. Open circles show sTNFRII-Fc(+), and closed circles show sTNFRII-Fc(-). A shows a result with shFcγRIIIa(F), and B shows that with shFcγRIIIa(V).

Fig. 40 shows neutralization activities of sTNFRII-Fc for mouse TNF-α. The abscissa shows sTNFRII-Fc concentration, and the ordinate shows TNF-α neutralization activity at each sTNFRII-Fc concentration. Closed circles show a result with sTNFRII-Fc(-), and open circles show that with sTNFRII-Fc(+).

Fig. 41 shows a construction process of a plasmid pKANTEXΔ1-12TNF-α.

Fig. 42 shows analysis of expression of membrane type human TNF-α of TNF-α/EL4 cell and its parent cell line EL4 cell, using a flow cytometer.

Fig. 43 shows ADCC activities of sTNFRII-Fc for TNF-α/EL4 cell. The abscissa shows sTNFRII-Fc concentration, and the ordinate shows ADCC activity (%) at each sTNFRII-Fc concentration. Closed circles show the activity of sTNFRII-Fc(-), and open circles show that of sTNFRII-Fc(+).

Fig. 44 shows a plasmid pBsIISK(-)/LFA-3-Fc.

Fig. 45 shows a plasmid pKANTEX93/LFA-3-Fc.

Fig. 46 shows SDS-PAGE electrophoresis patterns of purified LFA-3-Fc(-) and LFA-3-Fc(+) under reducing conditions and non-reducing conditions. Staining of protein was carried out using Coomassie Brilliant Blue (CBB).

Fig. 47 shows binding activities of LFA-3-Fc for CD2 expressing cell line, measured by fluorescent antibody technique. The abscissa shows LFA-3-Fc concentration, and the ordinate shows average fluorescence intensity at each LFA-3-Fc concentration. Closed circles show LFA-3-Fc(-), and open circles show LFA-3-Fc(+).

Fig. 48 shows binding activities of LFA-3-Fc for shFcγRIIIa. The abscissa shows LFA-3-Fc concentration, and the ordinate shows the binding activity at each LFA-3-Fc concentration. Open circles show the activity of LFA-3-Fc(+), and closed circles show that of LFA-3-Fc(-). A shows a result with shFcγRIIIa(F), and B shows that with shFcγRIIIa(V).

Fig. 49 shows ADCC activities of LFA-3-Fc for Jurkat cell. The abscissa shows LFA-3-Fc concentration, and the ordinate shows ADCC activity (OD490) at each LFA-3-Fc concentration. Closed circles show LFA-3-Fc(-), and open circles show LFA-3-Fc(+).

The present invention is explained below based on Examples. However,. the present invention is not limited thereto.

Examples

Example 1

Construction of CHO/DG44 cell in which both alleles of α1,6-fucosyltransferase (hereinafter referred to as FUT8) on the genome have been disrupted

The CHO/DG44 cell line comprising the deletion of a genome region for both alleles of FUT8 including the translation initiation codons was constructed according to the following steps.

1. Construction of targeting vector pKOFUT8Neo comprising exon 2 of Chinese hamster FUT8 gene

pKOFUT8Neo was constructed in the following manner using targeting vector pKOFUT8Puro comprising exon 2 of Chinese hamster FUT8 gene constructed by the method described in Example 13-1 of WO02/31140, and pKOSelectNeo (manufactured by Lexicon).

pKOSelectNeo (manufactured by Lexicon) was digested with the restriction enzyme *AscI* (manufactured by New England Biolabs) and subjected to agarose gel electrophoresis, and approximately 1.6 Kb *AscI* fragment comprising the neomycin resistance gene expression unit was recovered using GENECLEAN Spin Kit (manufactured by BIO101).

After pKOFUT8Puro was digested with the restriction enzyme *AscI* (manufactured by New England Biolabs), the end of the DNA fragment with alkaline phosphatase derived from *Escherichia coli* C15 (manufactured by Takara Shuzo Co., Ltd.) was dephosphorylated. After the reaction, the DNA fragment was purified by phenol/chloroform extraction and ethanol precipitation.

Sterilized water was added to 0.1 $\mu$g of the pKOSelectNeo-derived *AscI* fragment (approximately 1.6 Kb) and 0.1 $\mu$g of the pKOFUT8Puro-derived *AscI* fragment (approximately 10.1 Kb) obtained above to make up to 5 $\mu$l, and 5 $\mu$l of Ligation High (manufactured by Toyobo Co., Ltd.) was added thereto. The ligation reaction was carried out at 16°C for 30 minutes. *Escherichia coli* DH5$\alpha$ strain was transformed using the resulting reaction mixture, and a plasmid DNA was prepared from each of the obtained ampicillin-resistant clones. The plasmid DNA was subjected to reaction using BigDye Terminator Cycle Sequencing Ready Reaction Kit v2.0 (manufactured by Applied Biosystems) according to the attached instructions, and the nucleotide sequence was analyzed using DNA Sequencer ABI PRISM 377 (manufactured by Applied Biosystems). The thus obtained plasmid pKOFUT8Neo shown in Fig. 1 was used as a targeting vector for the subsequent preparation of FUT8 gene-hemi-knockout cell line.

2. Preparation of hemi-knockout cell line in which one copy of the FUT8 gene on the genome has been disrupted

(1) Obtaining of a cell line in which the targeting vector pKOFUT8Neo has been introduced

The Chinese hamster FUT8 genome region targeting vector pKOFUT8Neo constructed in Example 1-1 was introduced into Chinese hamster ovary-derived CHO/DG44 cells deficient in the dihydrofolate reductase gene (*dhfr*) [*Somataic Cell and Molecular Genetics,* 12, 555 (1986)] in the following manner.

pKOFUT8Neo was digested with the restriction enzyme *SaI*I (manufactured by New England Biolabs) for linearization, and 4 $\mu$g of the linearized pKOFUT8Neo was introduced into $1.6 \times 10^6$ CHO/DG44 cells by electroporation [*Cytotechnology,* 3, 133 (1990)]. The resulting cells were suspended in IMDM-dFBS (10)-HT(1) [IMDM medium (manufactured by Invitrogen) containing 10% dialysis FBS (manufactured by Invitrogen) and 1-fold concentration HT supplement (manufactured by Invitrogen)] and then seeded on a 10-cm dish for adherent cell culture (manufactured by Falcon). After culturing in a 5% $CO_2$ incubator at 37°C for 24 hours, the medium was replaced with 10 ml of IMDM-dFBS(10) (IMDM medium containing 10% dialysis FBS) containing 600 $\mu$g/ml G418 (manufactured by Nacalai Tesque, Inc.). Culturing was carried out in a 5% $CO_2$ incubator at 37°C for 15 days during which the above medium replacement was repeated every 3 to 4 days to obtain G418-resistant clones.

(2) Confirmation of homologous recombination by genomic PCR

Confirmation of the homologous recombination in the G418-resistant clones obtained in the above (1) was carried out by PCR using genomic DNA in the following manner.

The G418-resistant clones on a 96-well plate were subjected to trypsinization, and a 2-fold volume of a frozen medium (20% DMSO, 40% fetal calf serum and 40% IMDM) was added to each well to suspend the cells. One half of the cell suspension in each well was seeded on a flat-bottomed 96-well plate for adherent cells (manufactured by Asahi Techno Glass) to prepare a replica plate, while the other half was stored by cryopreservation as a master plate.

The neomycin-resistant clones on the replica plate were cultured using IMDM-dFBS(10) containing 600 $\mu$g/ml G418 in a 5% $CO_2$ incubator at 37°C for one week, followed by recovery of cells. The genomic DNA of each clone was prepared from the recovered cells according to a known method [*Analytical Biochemistry,* 201, 331 (1992)] and then dissolved overnight in 30 $\mu$l of TE-RNase buffer (pH 8.0) (10 mmol/l Tris-HCL, 1 mmol/l EDTA, 200 $\mu$g/ml RNase A).

Primers used in the genomic PCR were designed as follows. Primers respectively having the sequences represented by SEQ ID NOs:56 and 57, which are contained in the sequence of the FUT8 genome region obtained by the method described in Example 12 of WO03/31140 (SEQ ID NO:55), were employed as forward primers. Primers respectively having the sequences represented by SEQ ID NOs:58 and 59 which specifically bind to the loxP sequence of the targeting vector were employed as reverse primers in the following polymerase chain reaction (PCR). A reaction mixture [25 $\mu$l; DNA polymerase ExTaq (manufactured by Takara Shuzo Co., Ltd.), ExTaq buffer (manufactured by Takara Shuzo Co., Ltd.), 0.2 mmol/l dNTPs, 0.5 $\mu$mol/l each of the above primers (a combination of a forward primer and a reverse primer)] containing 10 μl of each genomic DNA solution prepared above was prepared, and PCR was carried out, after heating at 94°C for 3 minutes, by cycles, one cycle consisting of reaction at 94°C for one minute, reaction at 60°C for one minute and reaction at 72°C for 2 minutes.

After the PCR, the reaction mixture was subjected to 0.8% (w/v) agarose gel electrophoresis, and cell lines with which a specific amplification product (approximately 1.7 Kb) resulting from the homologous recombination was observed were judged to be positive clones.

(3) Confirmation of homologous recombination by genomic Southern blotting

Confirmation of the homologous recombination in the positive clones obtained in the above (2) was carried out by Southern blotting using genomic DNA in the following manner.

From the master plates stored by cryopreservation in the above (2), a 96-well plate containing the positive clones found in (2) was selected. After the plate was incubated in a 5% $CO_2$ incubator at 37°C for 10 minutes, the cells in the wells corresponding to the positive clones were seeded on a flat-bottomed 24-well plate for adherent cells (manufactured by Greiner). After culturing using IMDM-dFBS(10) containing 600 μg/ml G418 in a 5% $CO_2$ incubator at 37°C for one week, the cells were seeded on a flat-bottomed 6-well plate for adherent cells (manufactured by Greiner). The plate was subjected to culturing in a 5% $CO_2$ incubator at 37°C and the cells were recovered. The genomic DNA of each clone was prepared from the recovered cells according to a known method [*Nucleic Acids Research,* 3, 2303 (1976)] and then dissolved overnight in 150 μl of TE-RNase buffer (pH 8.0).

The genomic DNA prepared above (12 μg) was digested with the restriction enzyme *Bam*HI (manufactured by New England Biolabs), and a DNA fragment recovered by ethanol precipitation was dissolved in 20 μl of TE buffer (pH 8.0) (10 mmol/l Tris-HCL, 1 mmol/l EDTA) and then subjected to 0.6% (w/v) agarose gel electrophoresis. After the electrophoresis, the genomic DNA was transferred to a nylon membrane according to a known method [*Proc. Natl. Acad. Sci. USA,* 76, 3683 (1979)], followed by heat treatment of the nylon membrane at 80°C for 2 hours for immobilization.

Separately, a probe used in the Southern blotting was prepared in the following manner. Primers respectively having the sequences represented by SEQ ID NOs:60 and 61, which are contained in the sequence of the FUT8 genome region obtained by the method described in Example 12 of WO03/31140 (SEQ ID NO:55), were prepared and used in the following PCR. A reaction mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers] containing 4.0 ng of pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was recovered using GENECLEAN Spin Kit (manufactured by BIO101). A 5-μl portion of the obtained probe DNA solution was subjected to radiolabeling using [α-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC - 0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 2 shows the results of the analysis of the genomic DNAs of the parent cell line CHO/DG44 and the cell line 50-10-104, which is the positive clone obtained in the above (2), according to the present method. In the cell line CHO/DG44, only approximately 25.5 Kb fragment derived from the wild-type FUT8 allele was detected. On the other hand, in the positive clone, i.e., cell line 50-10-104, approximately 20.0 Kb fragment peculiar to the allele which underwent homologous recombination was detected in addition to approximately 25.5 Kb fragment derived from the wild-type FUT8 allele. The quantitative ratio of these two kinds of fragments was 1:1, whereby it was confirmed that the cell line 50-10-104 was a hemi-knockout clone wherein one copy of the FUT8 allele was disrupted.

3. Preparation of cell line CHO/DG44 in which the FUT8 gene on the genome has been double-knocked out

(1) Preparation of a cell line in which targeting vector pKOFUT8Puro has been introduced

In order to disrupt the other FUT8 allele in the FUT8 gene-hemi-knockout clone obtained in the above 2, the Chinese hamster FUT8 gene exon 2 targeting vector pKOFUT8Puro described in Example 13-1 of WO02/31140 was introduced into the clone in the following manner.

pKOFUT8Puro was digested with the restriction enzyme *Sal*I (manufactured by New England Biolabs) for linearization, and 4 μg of the linearized pKOFUT8Puro was introduced into $1.6 \times 10^6$ cells of the FUT8 gene-hemi-knockout clone by electroporation [*Cytotechnology,* 3, 133 (1990)]. The resulting cells were suspended in IMDM-dFBS (10)-HT(1) and then seeded on a 10-cm dish for adherent cell culture (manufactured by Falcon). After culturing in a 5% $CO_2$ incubator at 37°C for 24 hours, the medium was replaced with 10 ml of IMDM-dFBS(10)-HT(1) containing 15 μg/ml puromycin (manufactured by SIGMA). Culturing was carried out in a 5% $CO_2$ incubator at 37°C for 15 days during which the above medium replacement was repeated every 7 days to obtain puromycin-resistant clones.

(2) Confirmation of homologous recombination by genomic Southern blotting

Confirmation of the homologous recombination in the drug-resistant clones obtained in the above (1) was carried out by Southern blotting using genomic DNA in the following manner.

The puromycin-resistant clones were recovered into a flat-bottomed plate for adherent cells (manufactured by Asahi Techno Glass) according to a known method [*Gene Targeting,* Oxford University Press (1993)], followed by culturing using IMDM-dFBS(10)-HT(1) containing 15 μg/ml puromycin (manufactured by SIGMA) in a 5% $CO_2$ incubator at 37°C for one week.

After the culturing, each clone on the above plate was subjected to trypsinization and the resulting cells were seeded on a flat-bottomed 24-well plate for adherent cells (manufactured by Greiner). After culturing using IMDM-dFBS (10)-HT(1) containing 15 μg/ml puromycin (manufactured by SIGMA) in a 5% $CO_2$ incubator at 37°C for one week, the cells were subjected to trypsinization again and then seeded on a flat-bottomed 6-well plate for adherent cells (manufactured by Greiner). The plate was subjected to culturing in a 5% $CO_2$ incubator at 37°C and the cells were recovered. The genomic DNA of each clone was prepared from the recovered cells according to a known method [Nucleic Acids Research, 3, 2303 (1976)] and then dissolved overnight in 150 μl of TE-RNase buffer (pH 8.0).

The genomic DNA prepared above (12 μg) was digested with the restriction enzyme *Bam*HI (manufactured by New England Biolabs), and a DNA fragment recovered by ethanol precipitation was dissolved in 20 μl of TE buffer (pH 8.0) and then subjected to 0.6% (w/v) agarose gel electrophoresis. After the electrophoresis, the genomic DNA was transferred to a nylon membrane according to a known method *[Proc. Natl. Acad. Sci. USA,* 76, 3683 (1979)]*, followed by heat treatment of the nylon membrane at 80°C for 2 hours for immobilization.

Separately, a probe used in the Southern blotting was prepared in the following manner. Primers respectively having the sequences represented by SEQ ID NOs:62 and 63, which specifically bind to the sequences closer to the 5'-terminal than the FUT8 genome region contained in the targeting vector, were prepared and used in the following PCR. A reaction mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above primers] containing 4.0 ng of the plasmid pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was purified using GENECLEAN Spin Kit (manufactured by BIO101). A 5-μl portion of the obtained probe DNA solution was subjected to radiolabeling using [α-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC - 0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 3 shows the result of the analysis of the genomic DNA of the cell line WK704, which is one of the puromycin-resistant clones obtained from the cell line 50-10-104 by the method described in the above (1), according to the present method. In the cell line WK704, approximately 25.5 Kb fragment derived from the wild-type FUT8 allele was not detected and only approximately 20.0 Kb fragment specific to the allele which underwent homologous recombination (indicated by arrow in the figure) was detected. From this result, it was confirmed that the cell line WK704 was a clone wherein both FUT8 alleles were disrupted.

4. Removal of the drug resistance genes from FUT8 gene-double-knockout cells (1) Introduction of Cre recombinase expression vector

For the purpose of removing the drug resistance genes from the FUT8 gene-double-knockout clone obtained in the above item 3, the Cre recombinase expression vector pBS185 (manufactured by Life Technologies) was introduced into the clone in the following manner.

pBS185 (4 $\mu$g) was introduced into $1.6 \times 10^6$ cells of the FUT8 gene-double-knockout clone by electroporation [*Cytotechnology,* 3, 133 (1990)]. The resulting cells were suspended in 10 ml of IMDM-dFBS(10)-HT(1) and the suspension was diluted 20000-fold with the same medium. The diluted suspension was seeded on seven 10-cm dishes for adherent cell culture (manufactured by Falcon), followed by culturing in a 5% $CO_2$ incubator at 37°C for 10 days to form colonies.

(2) Obtaining of a cell line in which the Cre recombinase expression vector has been introduced

Clones arbitrarily selected from the colonies obtained in the above (1) were recovered into a flat-bottomed plate for adherent cells (manufactured by Asahi Techno Glass) according to a known method [*Gene Targeting,* Oxford University Press (1993)], followed by culturing using IMDM-dFBS(10)-HT(1) in a 5% $CO_2$ incubator at 37°C for one week.

After the culturing, each clone on the above plate was subjected to trypsinization, and a 2-fold volume of a frozen medium (20% DMSO, 40% fetal calf serum and 40% IMDM) was added to each well to suspend the cells. One half of the cell suspension in each well was seeded on a flat-bottomed 96-well plate for adherent cells (manufactured by Asahi Techno Glass) to prepare a replica plate, while the other half was stored by cryopreservation as a master plate.

The cells on the replica plate were cultured using IMDM-dFBS(10)-HT(1) containing 600 $\mu$g/ml G418 and 15 $\mu$g/ml puromycin in a 5% $CO_2$ incubator at 37°C for one week. Positive clones in which the drug resistance genes inserted between loxP sequences has been removed by the expression of Cre recombinase have died in the presence of G418 and puromycin. The positive clones were selected in this manner.

(3) Confirmation of removal of the drug resistance genes by genomic Southern blotting

Confirmation of the removal of the drug resistance genes in the positive clones selected in the above (2) was carried out by genomic Southern blotting in the following manner.

From the master plates stored by cryopreservation in the above (2), a 96-well plate containing the above positive clones was selected. After the plate was incubated in a 5% $CO_2$ incubator at 37°C for 10 minutes, the cells in the wells corresponding to the above clones were seeded on a flat-bottomed 24-well plate for adherent cells (manufactured by Greiner). After culturing using IMDM-dFBS(10)-HT(1) for one week, the cells were subjected to trypsinization and then seeded on a flat-bottomed 6-well plate for adherent cells (manufactured by Greiner). The plate was subjected to culturing in a 5% $CO_2$ incubator at 37°C and the proliferated cells were recovered. The genomic DNA of each clone was prepared from the recovered cells according to a known method [*Nucleic Acids Research,* 3, 2303 (1976)] and then dissolved overnight in 150 $\mu$l of TE-RNase buffer (pH 8.0).

The genomic DNA prepared above (12 $\mu$g) was digested with the restriction enzyme *Nhe*I (manufactured by New England Biolabs), and a DNA fragment recovered by ethanol precipitation was dissolved in 20 $\mu$l of TE buffer (pH 8.0) and then subjected to 0.6% (w/v) agarose gel electrophoresis. After the electrophoresis, the genomic DNA was transferred to a nylon membrane according to a known method [*Proc. Natl. Acad. Sci. USA,* 76, 3683 (1979)], followed by heat treatment of the nylon membrane at 80°C for 2 hours for immobilization.

Separately, a probe used in the Southern blotting was prepared in the following manner. PCR was carried out using primers respectively having the sequences represented by SEQ ID NOs:62 and 63, which specifically bind to the sequences closer to the 5'-terminal than the FUT8 genome region contained in the targeting vector. That is, a reaction mixture [20 $\mu$l; DNA polymerase ExTaq (manufactured by Takara Shuzo Co., Ltd.), ExTaq buffer (manufactured by Takara Shuzo Co., Ltd.), 0.2 mmol/l dNTPs, 0.5 $\mu$mol/l each of the above primers] containing 4.0 ng of the plasmid pFUT8fgE2-2 described in Example 12 of WO02/31140 as a template was prepared, and PCR was carried out, after heating at 94°C for one minute, by 25 cycles, one cycle consisting of reaction at 94°C for 30 seconds, reaction at 55°C for 30 seconds and reaction at 74°C for one minute.

After the PCR, the reaction mixture was subjected to 1.75% (w/v) agarose gel electrophoresis, and approximately 230 bp probe DNA fragment was purified using GENECLEAN Spin Kit (manufactured by BIO101). A 5-$\mu$l portion of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane to which the genomic DNA digestion product had been transferred was put into a roller bottle and 15 ml of a hybridization solution [5 $\times$ SSPE, 50 $\times$ Denhaldt's solution, 0.5% (w/v) SDS, 100 $\mu$g/ml salmon sperm DNA] was added thereto. Prehybridization was carried out at 65°C for 3 hours. Then, the $^{32}$P-labeled probe DNA was heat-denatured and put into the bottle, and hybridization was carried out at 65°C overnight.

After the hybridization, the nylon membrane was immersed in 50 ml of a primary washing solution [2 × SSC - 0.1% (w/v) SDS] and washed by heating at 65°C for 15 minutes. After this washing step was repeated twice, the nylon membrane was immersed in 50 ml of a secondary washing solution [0.2 × SSC - 0.1% (w/v) SDS] and washed by heating at 65°C for 15 minutes. Then, the nylon membrane was exposed to an X-ray film at -80°C for development.

Fig. 4 shows the results of the analysis of the genomic DNAs of the parent cell line CHO/DG44, the cell line 50-10-104 described in the above item 2, the clone WK704 described in the above item 3, and the cell line 4-5-C3, which is one of the drug-sensitive clones obtained from the cell line WK704 by the method described in the above (2), according to the present method. In the cell line CHO/DG44, only approximately 8.0 Kb DNA fragment derived from the wild-type FUT8 allele was detected. In the cell line 50-10-104 and the cell line WK704, approximately 9.5 Kb DNA fragment derived from the allele which underwent homologous recombination was observed. On the other hand, in the cell line 4-5-C3, only approximately 8.0 Kb DNA fragment resulting from the removal of the neomycin resistance gene (approximately 1.6 Kb) and the puromycin resistance gene (approximately 1.5 Kb) from the allele which underwent homologous recombination was detected. From the above results, it was confirmed that the drug resistance genes had been removed by Cre recombinase in the cell line 4-5-C3.

Besides the cell line 4-5-C3, plural FUT8 gene-double-knockout clones in which the drug-resistance gene had been removed (hereinafter referred to as FUT8 gene-double-knockout cells) were obtained.

Example 2

Expression of anti-TAG-72 scFv-Fc by FUT8 gene double knockout cell

1. Preparation of anti-TAG-72 scFv-Fc expression vector

(1) Construction of DNA encoding VH of anti-TAG-72 mouse monoclonal antibody

A DNA encoding the VH of a mouse monoclonal antibody CC49 [*The Journal of Immunology,* 151, 6559 (1993), GenBank Accession number/L14549] capable of specifically recognizing a cancer cell surface antigen TAG-72 was constructed in the following manner.

Firstly, the nucleotide sequence represented by SEQ ID NO:18 was designed. A restriction enzyme recognition sequence for cloning a sequence encoding the VH of CC49 into a cloning vector and an expression vector was inserted into the sequence, a non-translation sequence of 11 bases was inserted into 5'-terminal of the coding region for improving productivity of scFv-Fc, and a nucleotide sequence encoding a linker into the 3'-terminal. Four sequences of synthetic DNA (manufactured by Fasmach) represented by SEQ ID NOs:19, 20, 21 and 22, respectively, were designed by dividing the thus designed nucleotide sequence represented by SEQ ID NO:18 into a total of 4 sequences starting from the 5'-terminal and each having about 130 bases, in such a manner that the sense chain and antisense chain became alternate, and about 20 terminal bases of the nucleotide sequences adjoining each other were complementary for pairing.

A PCR solution [2.5 units KOD DNA Polymerase (manufactured by TOYOBO), 1 × concentration of PCR Buffer # 2 (manufactured by TOYOBO) attached to the KOD DNA Polymerase, 0.2 mM dNTPs, 1 mM magnesium chloride] was prepared by adjusting 2 sequences of synthetic DNA positioning at both termini among the 4 sequences of synthetic DNA to a final concentration of 0.5 μM, and the middle 2 sequences of synthetic DNA to a final concentration of 0.1 μM, and using a DNA thermal cycler GeneAmp PCR System 9700 (manufactured by Applied Biosystems), the solution was heated at 94°C for 5 minutes, and then the reaction was carried out by 25 cycles, one cycle consisting of reaction at 94°C for 30 seconds, reaction at 55°C for 30 seconds and reaction at 74°C for 60 seconds, subsequently allowing the mixture to react at 74°C for 5 minutes. After the PCR, the reaction solution was subjected to agarose gel electrophoresis, and a PCR product of about 450 bp was recovered using QIAquick Gel Extraction Kit (manufactured by QIAGEN). The thus recovered PCR product was digested with a restriction enzyme *Spe*I (manufactured by Takara Shuzo Co., Ltd.) and a restriction enzyme *Eco*RI (manufactured by Takara Shuzo Co., Ltd.), and then the reaction solution was subjected to agarose gel electrophoresis, and a PCR fragment of about 450 bp was recovered using QIAquick Gel Extraction Kit (manufactured by QIAGEN).

On the other hand, a plasmid pBluescriptII SK(-) (manufactured by Stratagene) was digested with restriction enzymes *Eco*RI (manufactured by Takara Shuzo Co., Ltd.) and *Spe*I (manufactured by Takara Shuzo Co., Ltd.) and then subjected to an agarose gel electrophoresis to recover a fragment of about 2.9 kbp.

The PCR fragment of about 450 bp and plasmid pBluescriptII SK(-) derived fragment of about 2.9 kbp obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pBSIISK(-)/CC49VH shown in Fig. 5 was obtained.

(2) Construction of DNA encoding the light chain variable region of an anti-TAG-72 mouse monoclonal antibody

A DNA encoding the VL of a mouse monoclonal antibody CC49 [*The Journal of Immunology,* 151, 6559 (1993), GenBank Accession number/L14553] capable of specifically recognizing a cancer cell surface antigen TAG-72 was constructed in the following manner.

Firstly, the nucleotide sequence represented by SEQ ID NO:23 was designed. A restriction enzyme recognition sequence for cloning a sequence encoding the VL of CC49 into a cloning vector and an expression vector was inserted into the sequence, a nucleotide sequence of a hinge region and a nucleotide sequence of a human IgG1 CH2 region were inserted into 3'-terminal of the coding region, and a nucleotide sequence encoding a linker to VH into the 5'-terminal. Four sequences of synthetic DNA (manufactured by Fasmach) represented by SEQ ID NOs:24, 25, 26 and 27, respectively, were designed by dividing the thus designed nucleotide sequence represented by SEQ ID NO:23 into a total of 4 sequences starting from the 5'-terminal and each having about 150 bases, in such a manner that the sense chain and antisense chain became alternate, and about 20 terminal bases of the nucleotide sequences adjoining each other were complementary for pairing.

A PCR solution [2.5 units KOD DNA Polymerase (manufactured by TOYOBO), 1× concentration of PCR Buffer # 2 (manufactured by TOYOBO) attached to the KOD DNA Polymerase, 0.2 mM dNTPs, 1 mM magnesium chloride] was prepared by adjusting 2 sequences of synthetic DNA positioning at both termini among the 4 sequences of synthetic DNA to a final concentration of 0.5 μM, and the middle 2 sequences of synthetic DNA to a final concentration of 0.1 μM, and using a DNA thermal cycler GeneAmp PCR System 9700 (manufactured by Applied Biosystems), the solution was heated at 94°C for 5 minutes, and then the reaction was carried out by 25 cycles, one cycle consisting of reaction at 94°C for 30 seconds, reaction at 55°C for 30 seconds and reaction at 74°C for 60 seconds, subsequently allowing the mixture to react at 74°C for 5 minutes. After the PCR, the reaction solution was subjected to agarose gel electrophoresis, and a VL PCR product of about 540 bp was recovered using QIAquick Gel Extraction Kit (manufactured by QIAGEN). The thus recovered PCR product was digested with a restriction enzyme *Spe*I (manufactured by Takara Shuzo Co., Ltd.) and a restriction enzyme *Eco*RI (manufactured by Takara Shuzo Co., Ltd.), and then the reaction solution was subjected to agarose gel electrophoresis, and a PCR product derived fragment of about 450 bp was recovered using QIAquick Gel Extraction Kit (manufactured by QIAGEN).

On the other hand, a plasmid pBluescriptII SK(-) (manufactured by Stratagene) was digested with restriction enzymes *Eco*RI (manufactured by Takara Shuzo Co., Ltd.) and *Spe*I (manufactured by Takara Shuzo Co., Ltd.) and then subjected to an agarose gel electrophoresis to recover a fragment of about 2.9 kbp using QIAquick Gel Extraction Kit (manufactured by QIAGEN).

The PCR fragment of about 450 bp and plasmid pBluescriptII SK(-) derived fragment of about 2.9 kbp obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pBSIISK(-)/CC49VL shown in Fig. 6 was obtained.

(3) Construction of anti-TAG-72 scFv-Fc expression vector

An expression vector of anti-TAG-72 scFv-Fc fusion protein was constructed from a vector pKANTEX93 for expression of humanized antibody [*Mol. Immunol.,* 37, 1035 (2000)] and the plasmids pBSIISK(-)/CC49VH obtained in the above-described item (1) and pBSIISK(-)/CC49VL obtained in the above-described (2), in the following manner.

The plasmid pBSIISK(-)/CC49VH obtained in the above-described (1) was digested with a restriction enzyme *Acc*III (manufactured by Takara Shuzo Co., Ltd.) and a restriction enzyme *Eco*RI (manufactured by Takara Shuzo Co., Ltd.), and then the reaction solution was subjected to agarose gel electrophoresis, and an *Eco*RI-*Acc*III fragment of about 450 bp was recovered using QIAquick Gel Extraction Kit (manufactured by QIAGEN).

Also, the plasmid pBSIISK(-)/CC49VL obtained in the above-described (2) was digested with a restriction enzyme *Acc*III (manufactured by Takara Shuzo Co., Ltd.) and a restriction enzyme *Bmg*BI (manufactured by New England Biolabs), and then the reaction solution was subjected to agarose gel electrophoresis, and an *Acc*III-*Bmg*BI fragment of about 540 bp was recovered using QIAquick Gel Extraction Kit (manufactured by QIAGEN).

On the other hand, the vector plasmid pKANTEX93 for expression of humanized antibody was digested with a restriction enzyme *Eco*RI (manufactured by Takara Shuzo Co., Ltd.) and a restriction enzyme *Bmg*BI (manufactured by New England Biolabs) and then subjected to an agarose gel electrophoresis to recover an *Eco*RI-*Bmg*BI fragment of about 9.8 kbp using QIAquick Gel Extraction Kit (manufactured by QIAGEN).

The plasmid pBSIISK(-)/CC49VH derived *Eco*RI-*Acc*III fragment, plasmid pBSIISK(-)/CC49VL derived *Acc*III-*Bmg*BI fragment and plasmid pKANTEX93 derived fragment obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pKANTEX93/CC49scFv-Fc shown in Fig. 7 was obtained.

2. Stable expression in FUT8 gene double knockout cell

Using Ms705 cell as the FUT8 gene double knockout cell described in item 4 of Example 1 and its parent cell line CHO/DG44 cell as the host cells, the anti-TAG-72 scFv-Fc expression vector pKANTEX93/CC49scFv-Fc prepared in the item 1 of this Example was introduced therein, and cells stably producing 2 kinds of TAG-72 scFv-Fc fusion proteins having different structures of sugar chains in the antibody Fc were prepared in the following manner.

An 8-$\mu$g portion of the plasmid pKANTEX93/CC49scFv-Fc was introduced into $1.6 \times 10^6$ cells of the Ms705 cell or CHO/DG44 cell by the electroporation method [*Cytotechnology,* 3, 133 (1990)], and then the cells were suspended in 30 ml of IMDM-(10) [IMDM medium containing 10% of fetal calf serum (FCS) in the case of Ms705 cell, or that of dialyzed fetal bovine serum (dFBS) in the case of CHO/DG44 cell: manufactured by GIBCO-BRL] medium and dispensed at 100 $\mu$l/well into a 96-well microplate (manufactured by Sumitomo Bakelite). After culturing at 37°C for 24 hours in a 5% $CO_2$ incubator, the culturing was continued for 1 to 2 weeks using the IMDM-(10) medium containing G418 in a concentration of 600 $\mu$g/ml. After the culturing, the culture supernatant was recovered from each well, and produced amount of the TAG-72 scFv-Fc fusion protein in the culture supernatant was measured by the ELISA shown in the item 3 of this Example, which is described later.

In order to increase the antibody expression quantity using a dhfr gene amplification system, the transformants of wells where expression of scFv-Fc was found in the culture supernatant were suspended in the IMDM-(10) medium containing 600 $\mu$g/ml of G418 and 50 nM in concentration of methotrexate (hereinafter referred to as "MTX": manufactured by SIGMA) which is an inhibitor of dihydrofolate reductase produced from the dhfr gene, and cultured at 37°C for about 1 week in a 5% $CO_2$ incubator to thereby obtain a transformant showing a resistance to 50 nM of MTX. Next, by increasing the MTX concentration to 100 nM and then to 200 nM, transformants capable of growing in the IMDM-(10) medium containing 600 $\mu$g/ml of G418 and 200 nM of MTX were finally obtained. Mono cell isolation (cloning) of the thus obtained transformants was carried out by limiting dilution.

Finally, a transformant which can grow in the IMDM-dFBS(10) medium containing 500 $\mu$g/ml of G418 and 200 nM of MTX and also can produce the anti-TAG-72 scFv-Fc fusion protein was obtained. The transformant obtained from the parent cell line CHO/DG44 cell was named KC1201, and the transformant obtained from the FUT8 gene double knockout cell was named KC1200.

3. Measurement of anti-TAG-72 scFv-Fc fusion protein concentration in culture supernatant (ELISA)

One $\mu$g/ml of a goat anti-human IgG (H & L) antibody (manufactured by American Qualex) by diluting with phosphate buffered saline (hereinafter referred to as "PBS") was dispensed at 50 $\mu$l/well into a 96-well plate for ELISA use (manufactured by Greiner) and incubated at a room temperature for 1 hour to effect its adsorption. After the incubation and subsequent washing with PBS, PBS containing 1% bovine serum albumin (hereinafter referred to as "BSA": manufactured by Proliant Inc.) (hereinafter referred to as "1% BSA-PBS") was added thereto at 100 $\mu$l/well and incubated at a room temperature for 1 hour to block the remaining active groups. The 1% BSA-PBS was removed, and a culture supernatant as the measuring object was added at each 50 $\mu$l/well and incubated at a room temperature for 2 hours. After the incubation and subsequent washing of each well with PBS containing 0.05% Tween 20 (hereinafter referred to as "Tween-PBS"), a peroxidase-labeled goat anti-human IgG (Fc) antibody solution (manufactured by American Qualex) diluted 500-fold with PBS was added thereto as the secondary antibody at 50 $\mu$l/well and incubated at a room temperature for 1 hour. After washing with Tween-PBS, an ABTS substrate solution [a solution prepared by dissolving 0.55 g of 2,2'-azino-bis(3-ethylbenzothiazoline-6-sulfonic acid) ammonium in 1 liter of 0.1 M citrate buffer (pH 4.2), and adding 1 $\mu$l/ml of hydrogen peroxide just before the use] was added thereto at 50 $\mu$l/well to develop color, and then absorbance at 415 nm (hereinafter referred to as "OD415") was measured.

4. Purification of anti-TAG-72 scFv-Fc fusion protein

The transformants KC1200 and KC1201 capable of expressing the anti-TAG-72 scFv-Fc fusion proteins, obtained in the item 2 of this Example, were respectively suspended in the IMDM-FCS(10) containing 200 nM of MTX to a density of $1\times10^5$ cells/ml and dispensed at 50 ml into 182 cm$^2$ flasks (manufactured by Greiner). Each culture supernatant was discarded when they became confluent by culturing at 37°C for 7 days in a 5% $CO_2$ incubator, and they were washed with 25 ml of PBS, and 30 ml of EXCEL 301 medium (manufactured by JRH Biosciences) was added. After culturing with EXCELL 301 medium at 37°C for 7 days in a 5% $CO_2$ incubator, the cell suspensions were recovered, and respective supernatants were recovered by carrying out 5 minutes of centrifugation under conditions of 3000 rpm at 4°C and then sterilized by filtration using PES Membrane of 0.22 $\mu$m in pore size (manufactured by Iwaki). The two kinds of anti-TAG-72 scFv-Fc fusion protein produced by different transformants were respectively purified from the culture supernatants recovered by the above-described method using a Prosep-A (manufactured by Millipore) column in accordance with the manufacture's instructions. Hereinafter, the purified anti-TAG-72 scFv-Fc fusion proteins are respectively referred to as anti-TAG-72 scFv-Fc(-) produced by KC1200 and anti-TAG-72 scFv-Fc(+) produced by KC1201.

5. Analysis of purified anti-TAG-72 scFv-Fc fusion proteins

Purification degree of the anti-TAG-72 scFv-Fc(-) and anti-TAG-72 scFv-Fc(+) purified in the item 4 of this Example and the ratio of sugar chains in which fucose is not bound to N-acetylglucosamine in the reducing end in the sugar chain, among the total complex type N-glycoside-linked sugar chains attached to antibodies, were confirmed in the following manner.

(1) Evaluation of the purification degree of anti-TAG-72 scFv-Fc(-) and anti-TAG-72 scFv-Fc(+)

An SDS modified polyacrylamide electrophoresis (hereinafter referred to as "SDS-PAGE") of about 3 $\mu$g of each of the purified anti-TAG-72 scFv-Fc fusion proteins was carried out in accordance with the conventionally known method [*Nature,* 227, 680 (1970)].

The results are shown in Fig. 8. Each of the two kinds of purified proteins was detected as a band of about 110 kilo daltons (hereinafter referred to as "kDa") under non-reducing conditions and that of about 55 kDa under reducing conditions. This result coincides with the report stating that molecular weight of the scFv-Fc fusion protein is about 110 kDa under non-reducing conditions, and the molecule is degraded into a composing unit of about 55 kDa under reducing conditions due to cleaving of its intramolecular disulfide bond (hereinafter referred to as "S-S bond") [*Proc. Natl. Acad. Sci. USA,* 36, 61 (1999)], and the electrophoresis patterns bear resemblance in the case of anti-TAG-72 scFv-Fc(-) and anti-TAG-72 scFv-Fc(+) wherein their hosts are different. Based on these, it was suggested that the anti-TAG-72 scFv-Fc(-) and anti-TAG-72 scFv-Fc(+) are expressed as polypeptide chains which coincided with the purpose.

(2) Monosaccharide composition analysis of purified anti-TAG-72 scFv-Fc fusion proteins

Each of the purified samples of anti-TAG-72 scFv-Fc(-) and anti-TAG-72 scFv-Fc(+) obtained in the item 4 of this Example was dried under a reduced pressure using an evaporator, and then mixed with 2.0 to 4.0 M of trifluoroacetic acid solution and subjected to hydrolysis at 100°C for 2 to 4 hours to release neutral sugars and amino sugars from the protein. The trifluoroacetic acid solution was removed using an evaporator, and the residue was re-dissolved in deionized water to carry out the analysis using an analyzer (DX-500) manufactured by Dionex. This was analyzed by an elution program shown in Table 1, using CarboPac PA-1 column and CarboPac PA-1 guard column (manufactured by Dionex), 10 to 20 mM sodium hydroxide-deionized water solution as the eluent, and 500 mM sodium hydroxide-deionized water solution as the washing solution.

Table 1 Elution program for neutral sugar and amino sugar composition analysis

| Time (min.) | 0 | 35 | 35.1 | 45 | 45.1 | 58 |
|---|---|---|---|---|---|---|
| Eluting solution (%) | 100 | 100 | 0 | 0 | 100 | 100 |
| Washing solution (%) | 0 | 0 | 100 | 100 | 0 | 0 |

From the obtained peak areas of neutral and amino sugar components of the elution program, the composition ratio of components (fucose, galactose and mannose) was calculated, regarding the value of N-acetylglucosamine as 4.

The ratio of sugar chains in which fucose is not bound to N-acetylglucosamine in the reducing end in the sugar chain, among the total complex type N-glycoside-linked sugar chains, calculated from the monosaccharide compositional ratio of each proteins, is shown in Table 2. The ratio of the sugar chains in which fucose is not bound to anti-TAG-72 scFv-Fc(+) was 9%. On the other hand, the ratio of the sugar chains in which fucose is not bound was estimated to be almost 100% in the case of anti-TAG-72 scFv-Fc(-), because the peak of fucose was at or below the detection limit.

Based on the above results, it was shown that fucose is not bound to the N-acetylglucosamine in the reducing end in the complex type N-glycoside-linked sugar chain of the anti-TAG-72 scFv-Fc fusion protein.

Table 2 Ratio of sugar chains containing no fucose of anti-TAG-72 scFv-Fc fusion protein

| Protein name | Ratio of sugar chains containing no fucose (%) |
|---|---|
| anti-TAG-72 scFv-Fc(+) | 9% |
| anti-TAG-72 scFv-Fc(+) | ~100% |

Example 3

Evaluation of activity of anti-TAG-72 scFv-Fc fusion proteins

1. Binding activity of anti-TAG-72 scFv-Fc fusion proteins for TAG-72 expression cell (fluorescent antibody technique)

Binding activities of purified samples of the anti-TAG-72 scFv-Fc(-) and anti-TAG-72 scFv-Fc(+) obtained in the item 4 of Example 2 were evaluated by the fluorescent antibody technique using a flow cytometer EPICS-XL (manufactured by Coulter). An anti-IL-5 receptor humanized antibody KM 8404 [*The Journal of Biological Chemistry, 31*, 3466 (2003)] was used as the negative control.

A human T cell lymphoma-derived cell line Jurkat cell (RCB 0806) which is a TAG-72-positive cell was dispensed into a 96-well U-shape plate (manufactured by Falcon) to a density of $2 \times 10^5$ cells per well, an antibody solution prepared by diluting anti-TAG-72 scFv-Fc(-), anti-TAG-72 scFv-Fc(+) or the negative control anti-IL-5 receptor humanized antibody KM 8404 with FACS buffer (PBS containing 0.02% EDTA, 0.05% $NaN_3$ and 0.5% BSA) to a final concentration of 0.016 to 50 $\mu$g/ml was added thereto at 50 $\mu$l/well and incubated for 30 minutes on ice. After washing twice with the FACS buffer, an FITC-labeled anti-human IgG1 antibody (manufactured by Zymed) was diluted 20-fold with the FACS buffer and added thereto at 50 $\mu$l/well. After allowing to react for 30 minutes on ice under shade, the cells were washed 3 times with the FACS buffer and suspended in 500 $\mu$l of PBS, and the fluorescence intensity was measured using the flow cytometer.

The results are shown in Fig. 9. Regarding the anti-TAG-72 scFv-Fc(-) and anti-TAG-72 scFv-Fc(+), the fluorescence intensity was increased concentration-dependently, and their activities to bind to Jurkat cell at each concentration were the same, but the negative control anti-IL-5 receptor humanized antibody KM 8404 did not bind to the Jurkat cell. Based on the above, it was shown that binding of the anti-TAG-72 scFv-Fc(-) and anti-TAG-72 scFv-Fc(+) to the Jurkat cell, which is a TAG-72-positive cell, is a binding specific for the scFv moiety of the fusion protein, and this binding is unrelated to the fucose content in the sugar chain in the anti-TAG-72 scFv-Fc fusion proteins.

2. Binding activity of anti-TAG-72 scFv-Fc to TAG-72 (ELISA)

A human body fluid derived TAG-72 (manufactured by Sigma) was diluted to 1 $\mu$g/ml with PBS, dispensed at 50 $\mu$l/well into a 96-well plate for ELISA use (manufactured by Greiner) and incubated at a room temperature for 1 hour for absorption. After washing with PBS, 1% BSA-PBS was added thereto at 100 $\mu$l/well and incubated at a room temperature for 1 hour to block the remaining active groups. By removing the 1% BSA-PBS, anti-TAG-72 scFv-Fc(-), anti-TAG-72 scFv-Fc(+) or the negative control anti-IL-5 receptor humanized antibody KM 8404 was added thereto at 50 $\mu$l/well in a concentration of 0.0032 $\mu$g/ml to 50 $\mu$g/ml, and incubated at a room temperature for 2 hours. After the incubation, each well was washed with Tween-PBS, and a peroxidase-labeled goat anti-human IgG (Fc) antibody solution (manufactured by American Qualex) diluted 500-fold with PBS was added thereto as the secondary antibody at 50 $\mu$l/well and incubated at a room temperature for 1 hour. After washing with Tween-PBS, ABTS substrate solution was added at 50 $\mu$l/well to develop the color and OD415 was measured.

The results are shown in Fig. 10. It was confirmed that anti-TAG-72 scFv-Fc(-) and anti-TAG-72 scFv-Fc(+) bind to the TAG-72 antigen concentration-dependently, and their binding is almost the same. On the other hand, binding of the negative control anti-IL-5 receptor humanized antibody KM 8404 to TAG-72 was not found. Based on the above, binding of the thus prepared two kinds of anti-TAG-72 scFv-Fc fusion proteins, having different sugar chain structures, to their TAG-72 antigen was a binding specific for the scFv moiety. It was confirmed that binding activities of the two kinds of anti-TAG-72 scFv-Fc fusion proteins to their antigens were almost the same, but the binding of anti-TAG-72 scFv-Fc(-)to its antigen TAG-72 was slightly high in comparison with the binding of anti-TAG-72 scFv-Fc(+)to TAG-72.

3. Binding activity of anti-TAG-72 scFv-Fc to Fcγ receptor IIIa (ELISA)

It is known that there are two alotypes of the Fcγ receptor IIIa, valine type (hereinafter referred to as "FcγRIIIa (V)") and phenylalanine type (hereinafter referred to as "FcγRIIIa(F)"), due to genetic polymorphism at the 176th position amino acid residue counting from the N-terminal methionine, and both of them have different binding activities for the antibody Fc. Binding activities of anti-TAG-72 scFv-Fc(-) and anti-TAG-72 scFv-Fc(+) to FcγRIIIa(V) and FcγRIIIa(F) were measured. The histidine tag-labeled FcγRIIIa(V) and histidine tag-labeled FcγRIIIa(F) used in the measurement were prepare by the method shown in Reference Example described later.

Firstly, the histidine tag-labeled FcγRIIIa(V) or histidine tag-labeled FcγRIIIa(F) diluted to 1 μg/ml with PBS was added at 50 μl/well into a plate to which a mouse anti-histidine tag antibody (manufactured by QIAGEN) had been absorbed, and incubated at a room temperature for 2 hours. After the reaction, each well was washed with Tween-PBS, anti-TAG-72 scFv-Fc(-) or anti-TAG-72 scFv-Fc(+) was added thereto at 50 μl/well in a concentration of 0.0017 μg/ml to 100 μg/ml and incubated at a room temperature for 2 hours. After the incubation, each well was washed with Tween-PBS, and a peroxidase-labeled goat anti-human Ig (H & L) antibody solution (manufactured by American Qualex) diluted 6000-fold with PBS was added thereto as the secondary antibody at 50 μl/well and incubated at a room temperature for 1 hour. After the incubation, each well was washed with Tween-PBS, the ABTS substrate solution was added at 50 μl/well to develop the color and OD415 was measured.

The results are shown in Fig. 11. It was confirmed that anti-TAG-72 scFv-Fc(-) and anti-TAG-72 scFv-Fc(+) bind to the FcγRIIIa concentration-dependently, and it was shown that the binding activity of anti-TAG-72 scFv-Fc(-) for FcγRIIIa was significantly higher than that the binding activity of anti-TAG-72 scFv-Fc(+). This result was the same in the two kinds of FcγRIIIa polymorphism. Also, since it was confirmed that scFv-Fc(-) and scFv-Fc(+) bind to FcγRIIIa, it was shown that the Fc regions of these scFv-Fcs are expressed in the form with binding activity for FcγRIIIa.

4. Binding activity of anti-TAG-72 scFv-Fc fusion proteins in the presence of TAG-72 as the antigen to Fcγ receptor IIIa (ELISA)

Binding activities of anti-TAG-72 scFv-Fc fusion proteins to FcγRIIIa(V) and FcγRIIIa(F) in the presence of TAG-72 antigen were measured. The histidine tag-labeled FcγRIIIa(V) and histidine tag-labeled FcγRIIIa(F) used in the measurement were prepared by the method shown in Reference Example which is described later.

Firstly, anti-TAG-72 scFv-Fc(-) or anti-TAG-72 scFv-Fc(+) was added at 50 μl/well into the plate prepared by the method in the item 2 of this Example, in a concentration of 0.0017 μg/ml to 100 μg/ml, and incubated at a room temperature for 2 hours. After the incubation, each well was washed with Tween-PBS, and the histidine tag-labeled FcγRIIIa(V) or histidine tag-labeled FcγRIIIa(F) diluted to 1 μg/ml with PBS was added at 50 μl/well and incubated at a room temperature for 2 hours. After the incubation, each well was washed with Tween-PBS, and a peroxidase-labeled mouse anti-histidine tag antibody solution (manufactured by QIAGEN) diluted 1000-fold with PBS was added thereto as the secondary antibody at 50 μl/well each and incubated at a room temperature for 1 hour. After washing with Tween-PBS, the ABTS substrate solution was added at 50 μl/well to develop the color and OD415 was measured.

The results are shown in Fig. 12. Anti-TAG-72 scFv-Fc(-) showed the binding activity to FcγRIIIa and its antigen TAG-72 concentration-dependently, but the color development was not found by anti-TAG-72 scFv-Fc(+). This difference was greater than the difference between the binding activity for TAG-72 of anti-TAG-72 scFv-Fc(-) and that of anti-TAG-72 scFv-Fc(+), which was confirmed in the item 2 of this Example, and this indicates that the binding activity to FcγRIIIa of anti-TAG-72 scFv-Fc(-)is higher than that of or anti-TAG-72 scFv-Fc(+) when anti-TAG-72 scFv-Fc(-) or anti-TAG-72 scFv-Fc(+) is bound with the TAG-72 antigen. In addition, it was shown that the binding activity to FcγRIIIa of anti-TAG-72 scFv-Fc(-) is higher than that of anti-TAG-72 scFv-Fc(+), in the presence of TAG-72 antigen independently of the polymorphism of FcγRIIIa, and the difference between the binding activity to FcγRIIIa of anti-TAG-72 scFv-Fc(-) and that of anti-TAG-72 scFv-Fc(+)was significant in the case of the valine type FcγRIIIa(V).

5. Evaluation of cytotoxic activity against TAG-72 expressing cell line (ADCC activity, $^{51}$Cr dissociation method)

In order to evaluate *in vitro* cytotoxicity of the purified anti-TAG-72 scFv-Fc(-) and purified anti-TAG-72 scFv-Fc(+) obtained in the item 4 of Example 2, the ADCC activity against a TAG-72-positive Jurkat cell which is human T cell lymphoma-derived cell line was measured by a $^{51}$Cr dissociation method in the following manner using an effector cell collected from a healthy donor. In addition, a Raji cell (RCB 0806) which is a cell line in which TAG-72 is not expressed was used as the negative control cell line.

(1) Preparation of target cell suspension

Jurkat cell or Raji cell was suspended in RPMI 1640-FCS(10) medium [RPMI 1640 medium (manufactured by GIBCO BRL) containing 10% FCS] to a density of $2 \times 10^6$ cells/ml, and the cells were radio-labeled by adding 3.7 MBq of a radioactive substance $Na_2 {}^{51}CrO_4$ and incubating at 37°C for 1 hour. After the incubation, the cells were washed 3 times by repeating suspension and centrifugation operations using the RPMI 1640-FCS(10) medium, again suspended in RPMI 1640-FCS(10) medium and then incubated at 4°C for 30 minutes on ice to remove free spontaneous dissociation of the radioactive substance. After washing, the suspension was adjusted to $2 \times 10^5$ cells/ml by adding RPMI 1640-FCS (10) medium and used as the target cell suspension.

(2) Preparation of human effector cell suspension

A 50-ml portion of peripheral blood was collected from a healthy donor, and 0.2 ml of heparin sodium (manufactured by Takeda Chemical Industries) was added thereto and gently mixed. A monocyte fraction was separated from the blood using Lymphoprep (manufactured by Daiichi Pure Chemicals) in accordance with its instructions. The cells were washed by centrifuging once with RPMI 1640 medium and once with RPMI 1640-FCS(10) medium, and then adjusted to $2 \times 10^6$ cells/ml by adding RPMI 1640-FCS(10) medium and used as the human effector cell suspension.

(3) Measurement of ADCC activity

The target cell suspension prepared in the above-described (1) was dispensed at 50 μl into each well of a 96-well U-bottom plate (manufactured by Falcon) ($1 \times 10^4$ cells/well). Next, the human effector cell suspension prepared in the above-described (2) was added thereto at 100 μl ($2 \times 10^5$ cells/well, the ratio of the human effector cells to target cells becomes 20:1). Furthermore, anti-TAG-72 scFv-Fc(-) or anti-TAG-72 scFv-Fc(+) was added thereto at a final concentration of 0.000094 to 50 μg/ml while adjusting the total volume to 200 μl and then incubated at 37°C for 4 hours. After the incubation, the reaction suspension was separated into cells and supernatant by centrifugation, and the amount of ${}^{51}Cr$ in the supernatant was measured using a γ-counter. At the same time, it was obtained by the same operation described above using the medium alone instead of the effector cell suspension and antibody solution, and the amount of ${}^{51}Cr$ spontaneously released from the effector cell was obtained using the medium alone instead of the target cell suspension and antibody solution. In addition, the total amount of ${}^{51}Cr$ released from the target cell was calculated by adding 1 N hydrochloric acid solution instead of the antibody solution and human effector cell suspension, carrying out the same operation described in the above, and then measuring the amount of ${}^{51}Cr$ in the supernatant.

$$
\begin{aligned}
\text{ADCC activity (\%)} &= \{(\text{amount of } {}^{51}Cr \text{ at each sample concentration} \\
&\qquad - \text{ amount of } {}^{51}Cr \text{ spontaneously released from effector cell} \\
&\qquad - \text{ amount of } {}^{51}Cr \text{ spontaneously released from target cell}) \\
&/ (\text{total amount of } {}^{51}Cr \text{ released from target cell} \\
&\qquad - \text{ amount of } {}^{51}Cr \text{ spontaneously released from target cell})\} \times 100
\end{aligned}
$$

The results are shown in Fig. 13. As shown in A of the drawing, concentration-dependent ADCC activity against a TAG-72-positive cell Jurkat cell was observed in anti-TAG-72 scFv-Fc(-) or anti-TAG-72 scFv-Fc(+). At each antibody concentration, the ADCC activity of anti-TAG-72 scFv-Fc(-) was higher than the ADCC activity of anti-TAG-72 scFv-Fc (+), and the maximum cytotoxic activity of anti-TAG-72 scFv-Fc(-) was also higher than that of anti-TAG-72 scFv-Fc(+), indicating that 1000-fold higher concentration is necessary for anti-TAG-72 scFv-Fc(+) to exert ADCC activity equivalent to anti-TAG-72 scFv-Fc(-), so that there was a difference equal to or larger than the difference in the binding activity to TAG-72 antigen of anti-TAG-72 scFv-Fc(+) and anti-TAG-72 scFv-Fc(-), which was confirmed in the item 2 of this Example. On the other hand, as shown in B of the drawing, both of the anti-TAG-72 scFv-Fc(-) and anti-TAG-72 scFv-Fc(+) did not show ADCC activity against the TAG-72-negative Raji cell.

Based on the above, regarding the ratio of the Fc fusion protein in which fucose is not bound to the N-acetylglucosamine in the reducing end in the complex type N-glycoside-linked sugar chain of the Fc fusion protein, among the total Fc fusion protein in each Fc fusion protein composition, there was a difference between anti-TAG-72 scFv-Fc(-) and anti-TAG-72 scFv-Fc(+). It was confirmed that this difference in the ratio is the difference in the FcγRIIIa binding activity between anti-TAG-72 scFv-Fc(-) and anti-TAG-72 scFv-Fc(+), and this difference in the FcγRIIIa binding activity corresponds to the difference in ADCC activity.

Example 4

Expression of anti-MUC1 scFv-Fc by FUT8 gene double knockout cell

1. Preparation of anti-MUC1 scFv-Fc expression vector

(1) Construction of a vector for expression of scFv-Fc pNUTS

Using the pKANTEX93/CC49scFv-Fc prepared in the item 1 of Example 2 as a base, a vector for inserting a nucleotide sequence encoding scFv-Fc or scFv$_2$-Fc was constructed in the following manner.

Firstly, the pKANTEX93/CC49scFv-Fc prepared in the item 1 of Example 2 was digested using a restriction enzyme *Bam*HI (manufactured by Takara Shuzo Co., Ltd.) and a restriction enzyme *Spe*I (manufactured by Takara Shuzo Co., Ltd.), and then the termini were blunted using Mung Bean Nuclease (manufactured by Takara Shuzo Co., Ltd.). After the reaction, ligation reaction was carried out by adding Ligation High solution (manufactured by TOYOBO) to the reaction solution, and an *E. coli* XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of each plasmid was analyzed by a DNA sequencer of the same company, ABI PRISM 377 to thereby confirm that the plasmid pKANTEX93/CC49scFv-Fc(B-S-) shown in Fig. 14 was obtained.

Next, the nucleotide sequence represented by SEQ ID NO:77 was designed by the following procedure. Synthetic DNA samples (manufactured by Fasmach) respectively represented by SEQ ID NO:78 and SEQ ID NO:79 which respectively contains a restriction enzyme recognizing sequence (*Eco*RI for 5'-terminal side, *Acc*III for 3'-terminal side) for integrating the sequence into a plasmid, a nucleotide sequence encoding signal sequence (includes a restriction enzyme *Age*I recognizing sequence), and a restriction enzyme recognizing sequence (includes *Bam*HI and *Spe*I from the 5'-terminal side) for integrating maximum of two pairs of VH and VL were prepared. To obtain DNA cassette for cloning the thus designed sequence, annealing reaction of the 2 synthetic DNA samples was carried out in accordance with an established method [*Molecular Cloning, A Laboratory Manual,* Second Edition, Cold Spring Harbor Laboratory Press (1989)], and then the reaction solution was subjected to agarose gel electrophoresis and a DNA fragment of about 80 bp was recovered using QIAquick Gel Extraction Kit (manufactured by QIAGEN).

On the other hand, the above-described plasmid pKANTEX93/CC49scFv-Fc(B-S-) was digested with a restriction enzyme *Eco*RI (manufactured by Takara Shuzo Co., Ltd.) and a restriction enzyme *Bam*HI (manufactured by Takara Shuzo Co., Ltd.), and then subjected to an agarose gel electrophoresis to recover a fragment of about 10.5 kbp using QIAquick Gel Extraction Kit (manufactured by QIAGEN).

Ligation reaction of the DNA fragment of about 80 bp and the fragment derived from the plasmid pKANTEX93/CC49scFv-Fc(B-S-), obtained in the above, was carried out using Ligation High solution (manufactured by TOYOBO), and an *E. coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS shown in Fig. 14 was obtained.

(2) Insertion of DNA encoding VH of anti-MUC1 mouse monoclonal antibody into pNUTS vector

A DNA encoding VH of a mouse monoclonal antibody C595 [*British Journal of Cancer,* 76, 614 (1997)] which specifically recognizes a cancer cell surface antigen MUC1 was inserted into pNUTS vector in the following manner.

The nucleotide sequence represented by SEQ ID NO:80 was designed by the following procedure. Firstly, since nucleotide sequences corresponding to portions of the N-terminus and C-terminus of the amino acid sequence of the VH of anti-MUC1 mouse monoclonal antibody C595 described in *British Journal of Cancer,* 76, 614 (1997) were deleted in the nucleotide sequence of the VH of anti-MUC1 mouse monoclonal antibody C595 of a data base (GenBank Accession number/S77034), they were compensated by referring to the nucleotide sequence of an antibody clone having the same amino acid sequence. Also, the nucleotide sequence of the VH of anti-MUC1 mouse monoclonal antibody C595 described in the reference was partially modified by modifying several amino acid residues thereof in such a manner that it encodes an amino acid sequence of VH suited for scFv. A restriction enzyme *Age*I recognizing sequence for cloning into the expression vector and a signal sequence were added to the 5'-terminal of the thus obtained nucleotide sequence of VH, and a nucleotide sequence encoding a linker and a restriction enzyme *Bam*HI recognizing sequence to the 3'-terminal thereof. Four sequences of synthetic DNA (manufactured by Fasmach) represented by SEQ ID NOs: 81, 82, 83 and 84, respectively, were designed by dividing the thus designed nucleotide sequence represented by SEQ ID NO:80 into a total of 4 nucleotide sequences starting from the 5'-terminal and each having about 120 bases, in such a manner that the sense chain and antisense chain became alternate, and about 20 terminal bases of the nucleotide sequences adjoining each other were complementary for pairing.

A PCR solution [containing 2.5 units of KOD plus DNA Polymerase (manufactured by TOYOBO), 0.2 mM dNTPs, 1 mM magnesium chloride and 1/10 volume of the 10-fold concentration PCR Buffer (manufactured by TOYOBO) attached to the DNA Polymerase] was prepared by adjusting 2 sequences of synthetic DNA positioning at both termini among the 4 sequences of synthetic DNA to a final concentration of 0.5 μM, and the middle 2 sequences of synthetic DNA to a final concentration of 0.1 μM, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 400 bp was recovered using QIAquick Gel Extraction Kit (manufactured by QIAGEN). The thus recovered PCR product was digested with a restriction enzyme *Age*I (manufactured by Nippon Gene) and a restriction enzyme *Bam*HI (manufactured by Takara Shuzo Co., Ltd.), and then the reaction solution was subjected to agarose gel electrophoresis, and a PCR fragment of about 400 bp was recovered using QIAquick Gel Extraction Kit (manufactured by QIAGEN).

On the other hand, the plasmid pNUTS prepared in this item (1) was digested with a restriction enzyme *Age*I (manufactured by Nippon Gene) and a restriction enzyme *Bam*HI (manufactured by Takara Shuzo Co., Ltd.) and then subjected to an agarose gel electrophoresis to recover a fragment of about 10.5 kbp using QIAquick Gel Extraction Kit (manufactured by QIAGEN).

The PCR fragment of about 400 bp and plasmid pNUTS derived fragment obtained in the above were ligated using Ligation High solution (manufactured by TOYOBO), and an *E. coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/HM shown in A of Fig. 15 was obtained.

(3) Construction of anti-MUC1 scFv-Fc expression vector

An expression vector of an anti-MUC1 scFv-Fc fusion protein was constructed in the following manner by inserting a DNA encoding the VL of an anti-MUC1 mouse monoclonal antibody into the pNUTS/HM prepared in the above (2).

The nucleotide sequence represented by SEQ ID NO:85 was designed by the following procedure. Firstly, since nucleotide sequences corresponding to portions of the N-terminus and C-terminus of the amino acid sequence of anti-MUC1 mouse monoclonal antibody C595VL described in a reference [*British Journal of Cancer*, 76, 614 (1997)] were deleted in the nucleotide sequence of the VL of anti-MUC1 mouse monoclonal antibody C595 of a data base (GenBank Accession number/S77032), they were compensated by referring to the nucleotide sequence of an antibody clone having the same amino acid sequence. Also, the nucleotide sequence was partially modified by modifying several amino acid residues of the VL of anti-MUC1 mouse monoclonal antibody C595 described in the above reference, in such a manner that it encodes an amino acid sequence of VL suited for scFv. A restriction enzyme *Bam*HI recognizing sequence and a nucleotide sequence encoding a linker for cloning into an expression vector were added to the 5'-terminal of the thus obtained nucleotide sequence encoding the VL of anti-MUC1 mouse monoclonal antibody C596, and a nucleotide sequence encoding a hinge and a restriction enzyme *Pma*CI recognizing sequence to the 3'-terminal thereof. Four sequences of synthetic DNA (manufactured by Fasmach) represented by SEQ ID NOs:86, 87, 88 and 89, respectively, were designed by dividing the thus designed nucleotide sequence represented by SEQ ID NO:85 into a total of 4 nucleotide sequences starting from the 5'-terminal side, each having about 110 bases, in such a manner that the sense chain and antisense chain became alternate, and about 20 terminal bases of the nucleotide sequences adjoining each other were complementary for paring.

A PCR solution [containing 2.5 units of KOD plus DNA Polymerase (manufactured by TOYOBO), 0.2 mM dNTPs, 1 mM magnesium chloride and 1/10 volume of the 10-fold concentration PCR Buffer (manufactured by TOYOBO) attached to the DNA Polymerase] was prepared by adjusting 2 sequences of synthetic DNA positioning at both termini among the 4 sequences of synthetic DNA to a final concentration of 0.5 μM, and the middle 2 sequences of synthetic DNA to a final concentration of 0.1 1 μM, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 400 bp was recovered using QIAquick Gel Extraction Kit (manufactured by QIAGEN). The thus recovered PCR product was digested with a restriction enzyme *Bam*HI (manufactured by Takara Shuzo Co., Ltd.) and a restriction enzyme *Pma*CI (manufactured by Takara Shuzo Co., Ltd.), and then the reaction solution was subjected to agarose gel electrophoresis, and a PCR fragment of about 400 bp was recovered using QIAquick Gel Extraction Kit (manufactured by QIAGEN).

On the other hand, the plasmid pNUTS/HM prepared in this item (2) was digested with a restriction enzyme *Bam*HI (manufactured by Takara Shuzo Co., Ltd.) and a restriction enzyme *Pma*CI (manufactured by Takara Shuzo Co., Ltd.) and then subjected to an agarose gel electrophoresis to recover a fragment of about 10.5 kbp using QIAquick Gel Extraction Kit (manufactured by QIAGEN).

The PCR fragment of about 400 bp and plasmid pNUTS/HM derived fragment obtained in the above were ligated using Ligation High solution (manufactured by TOYOBO), and an *E. coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/scFvM-Fc shown in Fig. 15 was obtained.

2. Stable expression in FUT8 gene double knockout cell

Using Ms705 cell as the FUT8 gene double knockout cell described in the item 4 of Example 1 and its parent cell line CHO/DG44 cell as the host cells, the anti-MUC1 scFv-Fc expression vector pNUTS/scFvM-Fc prepared in the item 1 of this Example was introduced therein, and transformants stably producing two kinds of anti-MUC1 scFv-Fc fusion proteins having different structures of sugar chains in the antibody Fc were prepared in accordance with method described in the item 2 of Example 2.

Using Ms705 cell as the FUT8 gene double knockout cell described in the item 4 of Example 1 and its parent cell line CHO/DG44 cell as the host cells, and introducing the anti-MUC1 scFv-Fc expression vector pNUTS/scFvM-Fc prepared in the item 1 of this Example therein, cells stably producing two kinds of anti-MUC1 scFv-Fc fusion proteins having different structures of sugar chains in the antibody Fc were prepared in the following manner.

An 8-$\mu$g portion of the plasmid pNUTS/scFvM-Fc was introduced into $1.6 \times 10^6$ cells of the Ms705 cell or CHO/DG44 cell by the electroporation method [*Cytotechnology*, *3*, 133 (1990)], and then the cells were suspended in 20 ml of IMDM-(10) [IMDM medium containing 10% of fetal calf serum (FCS) in the case of Ms705 cell, or that of dialyzed fetal bovine serum (dFBS) in the case of CHO/DG44 cell: manufactured by GIBCO-BRL] medium and dispensed at 100 $\mu$l/well into a 96-well microplate (manufactured by Sumitomo Bakelite). After culturing at 37°C for 24 hours in a 5% $CO_2$ incubator, the culturing was continued for 1 to 2 weeks using the IMDM-(10) medium. The culture supernatant was recovered from each well, and the amount of the anti-MUC1 scFv-Fc fusion protein contained in the culture supernatant was measured by the ELISA described in the item 3 of Example 2.

In order to increase the antibody expression quantity using a dhfr gene amplification system, the transformants of wells where expression of scFv-Fc was found in the culture supernatant were suspended in the IMDM-(10) medium containing 50 nM of MTX which is an inhibitor of the dhfr gene product dihydrofolate reductase, and cultured at 37°C for about 1 week in a 5% $CO_2$ incubator to thereby obtain a transformant showing a resistance to 50 nM of MTX. Next, by increasing the MTX concentration to 100 nM and then to 200 nM, transformants capable of growing in the IMDM-(10) medium containing 200 nM of MTX were finally obtained.

Finally, a transformant which can grow in the IMDM-dFBS(10) medium containing 200 nM in concentration of MTX and also can produce the anti-MUC1 scFv-Fc fusion protein was obtained. The transformant obtained from the parent cell line CHO/DG44 cell was named KM3487, and the transformant obtained from an FUT8 gene double knockout cell, Ms705 cell, was named KM3486.

3. Purification of anti-MUC1 scFv-Fc fusion protein

The transformants KM3486 and KM3487 capable of expressing the anti-MUC1 scFv-Fc fusion proteins, obtained in the item 2 of this Example, were respectively suspended in the IMDM-(10) containing 200 nM of MTX to a density of $1 \times 10^5$ cells/ml and dispensed at 35 ml into 182 cm$^2$ flasks (manufactured by Greiner). Each culture supernatant was discarded when they became confluent by culturing at 37°C for 7 days in a 5% $CO_2$ incubator, and they were washed with 25 ml of PBS, and 30 ml of EXCEL 301 medium (manufactured by JRH Biosciences) was added. After culturing at 37°C for 5 days in a 5% $CO_2$ incubator, the cell suspensions were recovered, and respective supernatants were recovered by carrying out 5 minutes of centrifugation under conditions of 3000 rpm and 4°C and then sterilized by filtration using PES Membrane of 0.22 μm in pore size (manufactured by Iwaki). The two kinds of anti-MUC1 scFv-Fc fusion protein produced by KM3486 and KM3487 were respectively purified from the culture supernatants recovered by the above-described method, using a Prosep-A (manufactured by Millipore) column in accordance with the manufacture's instructions. Hereinafter, the purified anti-MUC1 scFv-Fc fusion protein produced by the transformant KM3486 and the purified anti-MUC1 scFv-Fc fusion protein produced by the transformant KM3487 are referred to as anti-MUC1 scFv-Fc(-) and anti-MUC1 scFv-Fc(+), respectively.

4. Analysis of purified anti-MUC1 scFv-Fc fusion proteins

Purification degree of the anti-MUC1 scFv-Fc(-) and anti-MUC1 scFv-Fc(+) purified in the item 3 of this Example and the ratio of the sugar chains in which fucose is not bound to the N-acetylglucosamine in the reducing end in the sugar chain among the total complex type N-glycoside-linked sugar chains added to the antibodies, were confirmed in the following manner.

(1) Evaluation of the purification degree of anti-MUC1 scFv-Fc(-) and anti-MUC1 scFv-Fc(+)

SDS-PAGE was carried out using about 3 μg of each of the purified anti-MUC1 scFv-Fc fusion proteins in accordance with the item 5(1) of Example 2. The results are shown in Fig. 16. In both of A and B of the drawing, anti-MUC1 scFv-Fc(-) was shown in lane 3, and anti-MUC1 scFv-Fc(+) in lane 4, respectively. These two kinds of purified proteins were detected as a band of about 110 kDa under non-reducing conditions shown in A of the drawing, and that of about 55 kDa under reducing conditions shown in B of the drawing, respectively. Since this result coincided with the result of the item 5(1) of Example 2, it was suggested that the anti-MUC1 scFv-Fc(-) and anti-MUC1 scFv-Fc(+) are expressed as polypeptide chains which coincided with the purpose.

(2) Monosaccharide composition analysis of purified anti-MUC1 scFv-Fc fusion proteins

Monosaccharide composition analysis of the purified samples of anti-MUC1 scFv-Fc(-) and anti-MUC1 scFv-Fc(+) obtained in the item 3 of this Example was carried out in accordance with the method described in the item 5(2) of Example 2.

The ratio of the sugar chains in which fucose is not bound to the N-acetylglucosamine in the reducing end in the sugar chain among the total complex type N-glycoside-linked sugar chains, calculated from the monosaccharide compositional ratio of each protein, is shown in Table 3.

Table 3 Ratio of sugar chains containing no fucose of anti-MUC1 scFv-Fc fusion protein

| Protein name | Ratio of sugar chains containing no fucose (%) |
|---|---|
| anti-MUC1 scFv-Fc(+) | 9% |
| anti-MUC1 scFv-Fc(+) | ~100% |

The ratio of sugar chains in which fucose is not bound was 9% in the case of anti-MUC1 scFv-Fc(+). On the other hand, the ratio of sugar chains in which fucose is not bound was estimated to be almost 100% in the case of anti-MUC1 scFv-Fc(-), because the peak of fucose was at or below the detection limit.

Based on the above results, it was shown that fucose is not bound to the N-acetylglucosamine in the reducing end in the complex type N-glycoside-linked sugar chain of the anti-MUC1 scFv-Fc fusion protein.

Example 5

Evaluation of activity of anti-MUC1 scFv-Fc fusion proteins

1. Binding activity of anti-MUC1 scFv-Fc fusion proteins for MUC1 expression cell (fluorescent antibody technique)

Binding activities of purified samples of the anti-MUC1 scFv-Fc(-) and anti-MUC1 scFv-Fc(+) obtained in the item 3 of Example 4 were evaluated by the fluorescent antibody technique using a flow cytometer EPICS- XL (manufactured by Coulter).

A human breast cancer-derived cell line T-47D cell (ATCC: HTB-133) which is an MUC1-positive cell was dispensed into a 96-well U-shape plate (manufactured by Falcon) to a density of $2 \times 10^5$ cells per well, an antibody solution prepared by diluting anti-MUC1 scFv-Fc(-) or anti-MUC1 scFv-Fc(+) with the FACS buffer to a final concentration of 50

μg/ml was added thereto at 50 μl/well and incubated for 30 minutes on ice. After washing twice with the FACS buffer, an FITC-labeled anti-human IgG1 antibody (manufactured by Zymed) was diluted 20-fold with the FACS buffer and added thereto at 50 μl/well. After incubating for 30 minutes on ice under shade, the cells were washed 3 times with the FACS buffer and suspended in 500 μl of PBS, and the fluorescence intensity was measured using the flow cytometer. In addition, the same operation was carried out on an MUC1-negative cell, Raji cell, as the negative control.

The results are shown in Fig. 17. The anti-MUC1 scFv-Fc(-) and anti-MUC1 scFv-Fc(+) showed binding to the T-47D cell, but did not show binding to the Raji cell. In addition, activities of the anti-MUC1 scFv-Fc(-) and anti-MUC1 scFv-Fc(+) to bind to T-47D cell were equal to each other. Based on the above, it was shown that binding of the anti-MUC1 scFv-Fc(-) and anti-MUC1 scFv-Fc(+) to the T-47D cell, which is an MUC1-positive cell, is a binding specific to the scFv moiety of the Fc fusion protein, and this binding is unrelated to the fucose content in the sugar chain in the anti-MUC1 scFv-Fc fusion proteins.

2. MUC1 binding activity of anti-MUC1 scFv-Fc (ELISA)

A human body fluid derived MUC1 (breast tumor antigen: manufactured by Sigma) was diluted to 100 units/ml with PBS, dispensed at 50 μl/lwell into a 96-well plate for ELISA use (manufactured by Greiner) and incubated at a room temperature for 1 hour for adsorption. After washing with PBS, 1% BSA-PBS was added thereto at 100 μl/well and incubated at a room temperature for 1 hour to block the remaining active groups. After removing 1% BSA-PBS, PBS containing 0.005 units/ml in concentration of Neuraminidase (manufactured by Sigma) and 1 mg/ml in concentration of Pefabloc (manufactured by Roche), respectively, was dispensed at 50 μl/well and incubated at 37°C for 20 minutes to carry out decyalate of MUC1. After the incubation and subsequent washing with PBS, anti-MUC1 scFv-Fc(-) or anti-MUC1 scFv-Fc(+) was added thereto at 50 μl/well in a concentration of 0 to 10 μg/ml and incubated at a room temperature for 2 hours. After the incubation, each well was washed with Tween-PBS, and a peroxidase-labeled goat anti-human IgG (Fc) antibody solution (manufactured by American Qualex) diluted 1000-fold with PBS was added thereto as the secondary antibody at 50 μl/well and incubated at a room temperature for 1 hour. After washing with Tween-PBS, a TMB substrate solution (manufactured by Sigma) was added at 50 μl/well to develop the color, and OD450 was measured. In addition, the same operation was carried out also on a plate to which a human body fluid derived TAG-72 (manufactured by Sigma) was adhered as the negative control.

The results are shown in Fig. 18. As shown in A of the drawing, it was confirmed that anti-MUC1 scFv-Fc(-) and anti-MUC1 scFv-Fc(+) can bind to the MUC1 antigen concentration-dependently, and the binding was almost the same between anti-MUC1 scFv-Fc(-) and anti-MUC1 scFv-Fc(+). On the other hand, as shown in B of the drawing, binding of anti-MUC1 scFv-Fc(-) and anti-MUC1 scFv-Fc(+) to the negative control antigen TAG-72 was not found. Based on the above, binding of the thus prepared two kinds of anti-MUC1 scFv-Fc fusion proteins having different sugar chain structures to their antigen MUC1 was a binding specific to the scFv moiety.

3. Binding activity of anti-MUC1 scFv-Fc to Fcγ receptor IIIa (ELISA)

Binding activities of anti-MUC1 scFv-Fc(-) and anti-MUC1 scFv-Fc(+) to FcγRIIIa(V) or FcγRIIIa(F) were measured in accordance with the method described in the item 3 of Example 3. In this connection, anti-MUC1 scFv-Fc(-) or anti-MUC1 scFv-Fc(+) was added at the time of the reaction in a concentration of 0 to 10 μg/ml. Also, the TMB substrate solution was added to develop the color and OD450 was measured.

The results are shown in Fig. 19. As shown in A of the drawing, it was confirmed that anti-MUC1 scFv-Fc(-) and anti-MUC1 scFv-Fc(+) bind to the FcγRIIIa(V) concentration-dependently, and it was shown that the binding activity of anti-MUC1 scFv-Fc(-) for FcγRIIIa(V) is significantly higher than that the binding activity of anti-MUC1 scFv-Fc(+) for FcγRIIIa(V). As shown in B of the drawing, this was the same also in the case of FcγRIIIa(F). In addition, since binding of the scFv-Fc(-) and scFv-Fc(+) to the FcγRIIIa was confirmed, it was shown that the Fc region of scFv-Fc is expressed in the form with binding activity to FcγRIIIa.

4. Binding activity of anti-MUC1 scFv-Fc fusion proteins to Fcγ receptor IIIa in the presence of MUC1 antigen (ELISA)

Binding activities of anti-MUC1 scFv-Fc fusion proteins to FcγRIIIa(V) in the presence of MUC1 antigen were measured in accordance with the method described in the item 4 of Example 3. In this connection, anti-MUC1 scFv-Fc(-) or anti-MUC1 scFv-Fc(+) was added at the time of the reaction in a concentration of 0 to 10 μg/ml. Also, the TMB substrate solution was added to develop the color and OD450 was measured.

The results are shown in Fig. 20. Anti-MUC1 scFv-Fc(-) showed the binding activity to FcγRIIIa(V) concentration-dependently and MUC1 antigen, but the color development was not found by anti-MUC1 scFv-Fc(+). Based on this, it was confirmed that the binding activity of anti-MUC1 scFv-Fc(-) to FcγRIIIa in the presence of MUC1 antigen is higher than the activity of anti-MUC1 scFv-Fc(+) to FcγRIIIa.

5. Evaluation of cytotoxic activity against MUC1 expressing cell line (ADCC activity, $^{51}$Cr dissociation method)

In order to evaluate *in vitro* cytotoxicity of the purified samples of anti-MUC1 scFv-Fc(-) and anti-MUC1 scFv-Fc(+) obtained in the item 3 of the above-described Example 4, the ADCC activity against an MUC1-positive T-47D cell which is human breast cancer-derived cell line was measured in the following manner in accordance with the method described in the item 5 of Example 3. In addition, a Raji cell which is a cell line in which MUC1 is not expressed was used as the negative control cell line. In this connection, the reaction was carried out by adding anti-MUC1 scFv-Fc(-) or anti-MUC1 scFv-Fc(+) at the time of the reaction in a concentration of 0 to 10 $\mu$g/ml.

The results are shown in Fig. 21. As shown in A of the drawing, concentration-dependent ADCC activity against an MUC1-positive cell T-47D cell was observed in anti-MUC1 scFv-Fc(-) or anti-MUC1 scFv-Fc(+), and the ADCC activity of anti-MUC1 scFv-Fc(-) was higher than the ADCC activity of anti-MUC1 scFv-Fc(+). In addition, the maximum cytotoxic activity was also high in anti-MUC1 scFv-Fc(-) in comparison with anti-MUC1 scFv-Fc(+), indicating that 100-fold higher concentration is necessary for anti-MUC1 scFv-Fc(+) to exert its ADCC activity equivalent to anti-MUC1 scFv-Fc(-). Also, as shown in B of the drawing, anti-MUC1 scFv-Fc(-) and anti-MUC1 scFv-Fc(+) did not show ADCC activity against the MUC1-negative Raji cell.

Based on the above, regarding the ratio of the sugar chains in which fucose is not bound to the N-acetylglucosamine in the reducing end in the sugar chain among the total complex type N-glycoside-linked sugar chains, there was a difference between anti-MUC1 scFv-Fc(-) and anti-MUC1 scFv-Fc(+), and it was confirmed that this difference in the ratio of sugar chain is the difference in the Fc$\gamma$RIIIa binding activity between anti-MUC1 scFv-Fc(-) and anti-MUC1 scFv-Fc(+), and this difference in the Fc$\gamma$RIIIa binding activity corresponds to the difference in ADCC activity.

Example 6

Expression of scFv-Fc fusion proteins having two kinds of scFv by FUT8 gene double knockout cell

Expression vectors of scFv-Fc fusion proteins having two kinds of scFv in which anti-TAG-72 scFv, anti-MUC1 scFv and antibody Fc regions are lined up in order from the N-terminus (hereinafter referred to as anti-MUC1 anti-TAG-72 scFvM-scFvT-Fc or anti-TAG-72 anti-MUC1 scFvT-scFvM-Fc, in the linked order from the N-terminal side) were constructed in the following manner.

1. Construction of anti-MUC1 anti-TAG-72 scFvM-scFvT-Fc expression vector

The anti-MUC1 anti-TAG-72 scFvM-scFvT-Fc expression vector was constructed in the following manner, by inserting the VL moiety of anti-MUC1 scFv and anti-TAG-72 scFv into the plasmid pNUTS/HM prepared in the item 1 (2) of Example 4.

(1) Insertion of the VL moiety of anti-MUC1 scFv into plasmid pNUTS/HM

The nucleotide sequence represented by SEQ ID NO:90 was designed by the following procedure. The nucleotide sequence of a DNA encoding a hinge added to the 3' terminal side and the restriction enzyme *Pma*CI recognizing sequence were removed from the VL sequence designed in the item 1(3) of Example 4, and a nucleotide sequence of a DNA encoding a linker and a restriction enzyme *Spe*I recognizing sequence were added thereto. Four sequences of synthetic DNA (manufactured by Fasmach) represented by SEQ ID NOs:86, 87, 88 and 91, respectively, were designed by dividing the thus designed nucleotide sequence represented by SEQ ID NO:90 into a total of 4 sequences starting from the 5'-terminal side, each having about 110 bases, in such a manner that the sense chain and antisense chain became alternate, and about 20 terminal bases of the nucleotide sequences adjoining each other were complementary for paring.

A PCR solution [containing 2.5 units of KOD plus DNA Polymerase (manufactured by TOYOBO), 0.2 mM dNTPs, 1 mM magnesium chloride and 1/10 volume of 10-fold concentration PCR Buffer (manufactured by TOYOBO) attached to the DNA Polymerase] was prepared by adjusting 2 sequences of synthetic DNA positioning at both termini among the 4 sequences of synthetic DNA to a final concentration of 0.5 $\mu$M, and the middle 2 sequences of synthetic DNA to a final concentration of 0.1 $\mu$M, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 400 bp was recovered using QIAquick Gel Extraction Kit (manufactured by QIAGEN). The thus recovered PCR product was digested with a restriction enzyme *Bam*HI (manufactured by Takara Shuzo Co., Ltd.) and a restriction enzyme *Spe*I (manufactured by Takara Shuzo Co., Ltd.), and then the reaction solution was subjected to agarose gel electrophoresis, and a PCR fragment of about 400 bp was recovered using QIAquick Gel Extraction Kit (manufactured by QIAGEN).

In the meantime, the plasmid pNUTS/HM prepared in the item 1(2) of Example 4 was digested with the restriction enzyme *Bam*HI (manufactured by Takara Shuzo Co., Ltd.) and the restriction enzyme *Spe*I (manufactured by Takara Shuzo Co., Ltd.) and then subjected to an agarose gel electrophoresis to recover a fragment of about 10.5 kbp using QIAquick Gel Extraction Kit (manufactured by QIAGEN).

The PCR fragment of about 400 bp and plasmid pNUTS/HM derived fragment of about 10.5 kbp obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/HMLM shown in Fig. 22 was obtained.

(2) Insertion of anti-TAG-72 scFv into plasmid pNUTS/HMLM

The nucleotide sequence represented by SEQ ID NO:92 was designed by the following procedure. A restriction enzyme *Spe*I recognizing sequence for cloning into a vector and a nucleotide sequence encoding a linker were added at the 5'-terminal side of the DNA sequence of scFv designed in the item 1 of Example 2 containing VH and VL of the anti-TAG-72 antibody CC49 and a linker, and a nucleotide sequence encoding a hinge and a restriction enzyme *Pma*CI recognizing sequence for cloning into a vector into 3'-terminal side thereof. For obtaining the designed cDNA represented by SEQ ID NO:92 by PCR using the expression plasmid pKANTEX93/CC49scFv-Fc prepared in the item 1 of Example 2 as the template, two sequences of synthetic DNA (manufactured by Fasmach) respectively represented by SEQ ID NOs:93 and 94 were prepared.

A PCR solution [containing 2 units of KOD plus DNA Polymerase (manufactured by TOYOBO), 0.2 mM dNTPs, 1 mM magnesium sulfate and 1/10 volume of the 10-fold concentration PCR Buffer (manufactured by TOYOBO) attached to the DNA Polymerase] was prepared by adjusting the plasmid pKANTEX93/CC49scFv-Fc to a final concentration of 10 ng/μl, and the two primers to a final concentration of 0.5 μM, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 400 bp was recovered using QIAquick Gel Extraction Kit (manufactured by QIAGEN). The thus recovered PCR product was digested with a restriction enzyme *Spe*I (manufactured by Takara Shuzo Co., Ltd.) and a restriction enzyme *Pma*CI (manufactured by Takara Shuzo Co., Ltd.), and then the reaction solution was subjected to agarose gel electrophoresis, and a PCR fragment of about 800 bp was recovered using QIAquick Gel Extraction Kit (manufactured by QIAGEN).

In the meantime, the plasmid pNUTS/HMLM prepared in the item (1) was digested with the restriction enzyme *Spe*I (manufactured by Takara Shuzo Co., Ltd.) and the restriction enzyme *Pma*CI (manufactured by Takara Shuzo Co., Ltd.) and then subjected to an agarose gel electrophoresis to recover a fragment of about 11 kbp using QIAquick Gel Extraction Kit (manufactured by QIAGEN).

The PCR fragment of about 800 bp and plasmid pNUTS/HMLM derived fragment of about 11 kbp obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/scFvM-scFvT-Fc shown in Fig. 22 was obtained.

2. Preparation of anti-TAG-72 anti-MUC1 scFvT-scFvM-Fc expression plasmid

The anti-TAG-72 anti-MUC1 scFvT-scFvM-Fc expression plasmid was constructed in the following manner, by inserting the anti-MUC1 scFv and anti-TAG-72 scFv into the plasmid pNUTS prepared in the item 1(1) of Example 4.

(1) Insertion of anti-TAG-72 scFv into expression vector pNUTS

The nucleotide sequence represented by SEQ ID NO:95 was designed by the following procedure. A restriction enzyme *Age*I recognition sequence for cloning into a vector and a signal sequence were added at the 5'-terminal side of the DNA sequence of scFv designed in the item 1 of Example 2 containing VH and VL of the anti-TAG-72 antibody CC49 and a linker, and a nucleotide sequence encoding a linker and a restriction enzyme *Spe*I recognizing sequence for cloning into a vector were added at 3'-terminal side thereof. For obtaining the designed cDNA represented by SEQ ID NO:95 by PCR using the expression vector plasmid pKANTEX93/CC49scFv-Fc prepared in the item 1 of Example 2 as the template, two sequences of synthetic DNA (manufactured by Fasmach) respectively represented by SEQ ID NOs:96 and 97 were prepared.

A PCR solution [containing 2 units of KOD plus DNA Polymerase (manufactured by TOYOBO), 0.2 mM dNTPs, 1 mM magnesium sulfate and 1/10 volume of 10-fold concentration PCR Buffer (manufactured by TOYOBO) attached to the DNA Polymerase] was prepared by adjusting the vector plasmid pKANTEX93/CC49scFv-Fc to a final concentration of 10 ng/μl, and the two primers to a final concentration of 0.5 μM, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 400 bp was recovered using QIAquick Gel Extraction Kit (manufactured by QIAGEN). The thus recovered PCR product was digested with a restriction enzyme *Age*I (manufactured by Nippon Gene) and a restriction enzyme *Spe*I (manufactured by Takara Shuzo Co., Ltd.), and then the reaction solution was subjected to agarose gel electrophoresis, and a PCR fragment of about 800 bp was recovered using QIAquick Gel Extraction Kit (manufactured by QIAGEN).

In the mean time, the plasmid pNUTS prepared in the item 1 of Example 4 was digested with the restriction enzyme *Age*I (manufactured by Nippon Gene) and the restriction enzyme *Spe*I (manufactured by Takara Shuzo Co., Ltd.) and then subjected to an agarose gel electrophoresis to recover a fragment of about 10.5 kbp using QIAquick Gel Extraction Kit (manufactured by QIAGEN).

The PCR fragment of about 800 bp and plasmid pNUTS derived fragment of about 10.5 kbp obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/HTLT shown in Fig. 23 was obtained.

(2) Insertion of anti-MLTC1 scFv into plasmid pNUTS/HTLT

An anti-TAG-72 anti-MUC1 scFvT-scFvM-Fc expression vector was constructed in the following manner.

The nucleotide sequence represented by SEQ ID NO:98 was designed by the following procedure. A restriction enzyme *Spe*I recognition sequence for cloning into a vector and a nucleotide sequence encoding a linker were added at the 5'-terminal side of the DNA sequence of scFv designed in the item 1 of Example 4 containing VH and VL of the anti-MUC1 antibody C595 and a linker, and a nucleotide sequence encoding a hinge and a restriction enzyme *Pma*CI recognition sequence for cloning into a vector was added at the 3'-terminal side thereof. For obtaining the designed cDNA represented by SEQ ID NO:98 by PCR using the expression plasmid pNUTS/scFvM-Fc prepared in the item 1 of Example 4 as the template, two sequences of synthetic DNA (manufactured by Fasmach) respectively represented by SEQ ID NOs:94 and 99 were prepared.

A PCR solution [containing 2 units of KOD plus DNA Polymerase (manufactured by TOYOBO), 0.2 mM dNTPs, 1 mM magnesium chloride and 1/10 volume of the 10-fold concentration PCR Buffer (manufactured by TOYOBO) attached to the DNA Polymerase] was prepared by adjusting the plasmid pNUTS/scFvM-Fc to a final concentration of 10 ng/μl, and the two sequences of primers to a final concentration of 0.5 μM, and using a DNA thermal cycler GeneAmp PCR System 9700 (manufactured by Applied Biosystems), the solution was heated at 94°C for 4 minutes, and then the reaction was carried out by 25 cycles, one cycle consisting of reaction at 94°C for 30 seconds, reaction at 55°C for 30 seconds and reaction at 68°C for 60 seconds. After the PCR, the reaction solution was subjected to agarose gel electrophoresis, and a PCR product of about 800 bp was recovered using QIAquick Gel Extraction Kit (manufactured by QIAGEN). The thus recovered PCR product was digested with a restriction enzyme *Spe*I (manufactured by Takara Shuzo Co., Ltd.) and a restriction enzyme *Pma*CI (manufactured by Takara Shuzo Co., Ltd.), and then the reaction solution was subjected to agarose gel electrophoresis, and a PCR fragment of about 800 bp was recovered using QIAquick Gel Extraction Kit (manufactured by QIAGEN).

In the mean time, the plasmid pNUTS/scFvM-Fc prepared in the item 1 of Example 4 was digested with the restriction enzyme *Spe*I (manufactured by Takara Shuzo Co., Ltd.) and the restriction enzyme *Pma*CI (manufactured by Takara Shuzo Co., Ltd.) and then subjected to an agarose gel electrophoresis to recover a fragment of about 11 kbp using QIAquick Gel Extraction Kit (manufactured by QIAGEN).

The PCR product derived fragment and plasmid pNUTS/scFvM-Fc derived fragment obtained in the above were ligated using Ligation High solution (manufactured by TOYOBO), and an *Escherichia coli* strain XL1-BLUE MRF' (manufactured by Stratagene) was transformed using the reaction solution. Respective plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid pNUTS/scFvT-scFvM-Fc shown in Fig. 23 was obtained.

3. Stable expression in FUT8 gene double knockout cell

Using Ms705 cell as the FUT8 gene double knockout cell described in item 4 of Example 1 and its parent cell line CHO/DG44 cell as the host cells, the anti-MUC1 anti-TAG-72 scFvM-scFvT-Fc fusion protein expression vector pNUTS/scFvM-scFvT-Fc and anti-TAG-72 anti-MUC1 scFvT-scFvM-Fc fusion protein expression vector pNUTS/scFvT-scFvM-Fc prepared in the item 1 of this Example were respectively introduced therein, and transformants producing two kinds of anti-MUC1 anti-TAG-72 scFvM-scFvT-Fc fusion protein having different structures of sugar chains to be bound to the antibody Fc and two kinds of anti-TAG-72 anti-MUC1 scFvT-scFvM-Fc fusion protein having different structures of sugar chains to be bound to the antibody Fc were prepared in accordance with the method described in the item 2 of Example 2.

Transformants which can produce two kinds of anti-MUC1 anti-TAG-72 scFvM-scFvT-Fc fusion protein having different structures of sugar chains to be bound to the antibody Fc and two kinds of anti-TAG-72 anti-MUC1 scFvT-scFvM-Fc fusion protein having different structures of sugar chains to be bound to the antibody Fc which can grow in the IMDM-dFBS(10) medium containing MTX at a final concentration of 200 nM were obtained. Regarding the anti-MUC1 anti-TAG-72 scFvM-scFvT-Fc, the transformant obtained from the parent cell line CHO/DG44 cell was named KM3489, and the transformant obtained from the FUT8 gene double knockout cell was named KM3488, respectively, and regarding the anti-TAG-72 anti-MUC1 scFvT-scFvM-Fc, the transformant obtained from the parent cell line CHO/DG44 cell was named KM3491, and the transformant obtained from the FUT8 gene double knockout cell was named KM3490, respectively.

4. Purification of scFv$_2$-Fc fusion proteins having two kinds of scFv

The scFv$_2$-Fc fusion proteins having two kinds of scFv were respectively purified from the four kinds of the transformants, KM3488, KM3489, KM3490 and KM3491, obtained in the above item 3, in accordance with the method described in the item 4 of Example 2. Hereinafter, the purified scFv$_2$-Fc fusion proteins having two kinds of scFv are referred to as anti-MUC1 anti-TAG-72 scFvM-scFvT-Fc(-) produced by KM3488, anti-MUC1 anti-TAG-72 scFvM-scFvT-Fc(+) produced by KM3489, anti-TAG-72 anti-MUC1 scFvT-scFvM-Fc(-) produced by KM3490, and anti-TAG-72 anti-MUC1 scFvT-scFvM-Fc(+) produced by KM3491, respectively.

5. Analysis of purified scFv$_2$-Fc fusion proteins having two kinds of scFv

Purification degree of the anti-TAG-72 anti-MUC1 scFvM-scFvT-Fc(-), anti-TAG-72 anti-MUC1 scFvM-scFvT-Fc(+), anti-TAG-72 anti-MUC1 scFvT-scFvM-Fc(-) and anti-TAG-72 anti-MUC1 scFvT-scFvM-Fc(+) purified in the above item 4 and the ratio of the sugar chains in which fucose is not bound to N-acetylglucosamine in the reducing end in the sugar chain among the total complex type N-glycoside-linked sugar chains, were confirmed in the following manner.

(1) Evaluation of the purification degree of the purified anti-TAG-72 anti-MUC1 scFv$_2$-Fc fusion proteins

SDS-PAGE was carried out in accordance with the method described in the item 5(1) of Example 2, using about 3 μg of each of the purified anti-TAG-72 anti-MUC1 scFv2-Fc fusion proteins.

The results are shown in Fig. 16. In the drawing, anti-MUC1 anti-TAG-72 scFvM-scFvT-Fc(-) was shown in lane 5, and anti-MUC1 anti-TAG-72 scFvM-scFvT-Fc(+) in lane 6, anti-TAG-72 anti-MUC1 scFvT-scFvM-Fc(-) in lane 7, and anti-TAG-72 anti-MUC1 scFvT-scFvM-Fc(+) in lane 8, respectively. Each of the four kinds of purified proteins was detected as a band of about 160 kDa under non-reducing conditions and that of about 80 kDa under reducing conditions. This result coincides with the molecular weight deduced from the amino acid sequence of purified protein. Based on this, it was suggested that each of the purified anti-TAG-72 anti-MUC1 scFv$_2$-Fc fusion proteins is expressed as a polypeptide chain coincided with the purpose.

(2) Monosaccharide composition analysis of purified scFv$_2$-Fc fusion proteins having two kinds of scFv

Monosaccharide composition analysis of each of the purified samples of anti-TAG-72 anti-MUC1 scFv$_2$-Fc fusion proteins obtained in the above-described item 4 was carried out in accordance with the method described in the item 4(2) of Example 4.

The results are shown in Table 4. The ratio of sugar chains in which fucose is not bound was 9% in the case of anti-MUC1 anti-TAG-72 scFvM-scFvT-Fc(+) and anti-TAG-72 anti-MUC1 scFvT-scFvM-Fc(+). On the other hand, in the case of anti-MUC anti-TAG-72 scFvM-scFvT-Fc(-) and anti-TAG-72 anti-MUC1 scFvT-scFvM-Fc(-), the ratio of sugar chains in which fucose is not bound was estimated to be almost 100%, because the peak of fucose was at or below the detection limit.

Based on the above results, it was shown that fucose is not bound to the N-acetylglucosamine in the reducing end in the complex type N-glycoside-linked sugar chain of the anti-MUC1 anti-TAG-72 scFvM-scFvT-Fc(-) and anti-TAG-72 anti-MUC1 scFvT-scFvM-Fc(-).

Table 4 Ratio of sugar chains containing no fucose of scFv-Fc fusion proteins having two kinds of scFv

| Protein name | Ratio of sugar chains containing no fucose (%) |
|---|---|
| anti-MUC1 anti-TAG-72 scFvM-scFvT-Fc(+) | 9% |
| anti-MUC1 anti-TAG-72 scFvM-scFvT-Fc(-) | ~100% |
| anti-TAG-72 anti-MUC1 scFvT-scFvM-Fc(+) | 9% |
| anti-TAG-72 anti-MUC1 scFvT-scFvM-Fc(-) | ~100% |

Example 7

Evaluation of activity of scFv$_2$-Fc fusion proteins having two kinds of scFv

1. Binding activities of scFv$_2$-Fc fusion proteins having two kinds of scFv for TAG-72 expression cell and MUC1 expression cell (fluorescent antibody technique)

Binding activities of the purified samples of anti-TAG-72 anti-MUC1 scFvM-scFvT-Fc(-), anti-TAG-72 anti-MUC1 scFvM-scFvT-Fc(+), anti-TAG-72 anti-MUC1 scFvT-scFvM-Fc(-) and anti-TAG-72 anti-MUC1 scFvT-scFvM-Fc(+) obtained in the item 4 of Example 6 were evaluated by the fluorescent antibody technique in accordance with the method described in the item 1 of Example 3 using a flow cytometer EPICS-XL (manufactured by Coulter). In this case, a human T cell lymphoma-derived cell line, Jurkat cell which is a TAG-72-positive and MUC1-negative cell, was used as the TAG-72 expression cell, and a human breast cancer-derived cell line, T-47D cell which is an MUC1-positive and TAG-72-negative cell, as the MUC1 expression cell, respectively. In addition, the same operation was also carried out using Raji cell, which is a TAG-72-negative and MUC1-negative cell, as the negative control. In this connection, anti-TAG-72 anti-MUC1 scFv$_2$-Fc was used in the reaction in a concentration of 75 μg/ml.

The results are shown in Fig. 24. Anti-MUC1 anti-TAG-72 scFvM-scFvT-Fc(-) and anti-MUC1 anti-TAG-72 scFvM-scFvT-Fc(+) showed their binding to Jurkat cell and T-47D cell, but did not show the binding to Raji cell. In addition, binding activities of anti-MUC1 anti-TAG-72 scFvM-scFvT-Fc(-) and anti-MUC1 anti-TAG-72 scFvM-scFvT-Fc (+) for Jurkat cell and T-47D cell were similar to each other.

In the same manner, anti-TAG-72 anti-MUC1 scFvT-scFvM-Fc(-) and anti-TAG-72 anti-MUC1 scFvT-scFvM-Fc(+) showed their binding to Jurkat cell and T-47D cell, but did not show the binding to Raji cell. In addition, binding activities of anti-TAG-72 anti-MUC1 scFvT-scFvM-Fc(-) and anti-TAG-72 anti-MUC1 scFvT-scFvM-Fc(+) to Jurkat cell and T-47D cell were similar to each other.

Based on the above, it was shown that the binding of anti-TAG-72 anti-MUC1 scFvM-scFvT-Fc(-) and anti-TAG-72 anti-MUC1 scFvM-scFvT-Fc(+) to the TAG-72-positive Jurkat cell and the binding of the same to the MUC1-positive T-47D and the binding of anti-TAG-72 anti-MUC1 scFvT-scFvM-Fc(-) and anti-TAG-72 anti-MUC1 scFvT-scFvM-Fc(+) to the TAG-72-positive Jurkat cell and the binding of the same to the MUC1-positive T-47D are a binding specific to respective scFv moieties of the scFv$_2$-Fc fusion proteins having two kinds of scFv, and this binding is unrelated to the fucose content in the sugar chain of the scFvT-scFvM-Fc fusion proteins having two kinds of scFv.

2. TAG-72 or MUC1 binding activity of scFv$_2$-Fc having two kinds of scFv (ELISA)

Binding activities of the purified samples of anti-TAG-72 anti-MUC1 scFvM-scFvT-Fc(-), anti-TAG-72 anti-MUC1 scFvM-scFvT-Fc(+), anti-TAG-72 anti-MUC1 scFvT-scFvM-Fc(-) and anti-TAG-72 anti-MUC1 scFvT-scFvM-Fc (+) obtained in the item 4 of Example 6 to TAG-72 or MUC1 were evaluated by the ELISA in accordance with the method described in the item 2 or item 3 of Example 3. In this case, this was carried out by adjusting respective scFv$_2$-Fc having two kinds of scFv to a final concentration of 0 to 15 μg/ml. In addition, the TMB substrate solution (manufactured by Sigma) was used as the color developing substrate, and OD450 was measured.

Results of the binding activities of scFv$_2$-Fc having two kinds of scFv to TAG-72 are shown in Fig. 25. As shown in A of the drawing, it was confirmed that anti-MUC1 anti-TAG-72 scFvM-scFvT-Fc(-) and anti-MUC1 anti-TAG-72 scFvM-scFvT-Fc(+) concentration-dependently bind to their antigen TAG-72, and the bindings are almost the same. In the same manner, as shown in B of the drawing, it was confirmed that anti-TAG-72 anti-MUC1 scFvT-scFvM-Fc(-) and anti-TAG-72 anti-MUC1 scFvT-scFvM-Fc(+) concentration-dependently bind to their antigen TAG-72, and the bindings are almost the same.

In addition, results of the binding activities of scFv$_2$-Fc having two kinds of scFv for MUC1 are shown in Fig. 26. As shown in A of the drawing, it was confirmed that anti-MUC1 anti-TAG-72 scFvM-scFvT-Fc(-) and anti-MUC1 anti-TAG-72 scFvM-scFvT-Fc(+) concentration-dependently bind to their antigen MUC1, and the bindings are almost the same. In the same manner, as shown in B of the drawing, it was confirmed that anti-TAG-72 anti-MUC1 scFvT-scFvM-Fc(-) and anti-TAG-72 anti-MUC1 scFvT-scFvM-Fc(+) concentration-dependently bind to their antigen MUC1, and the bindings are almost the same.

Based on the above, binding of the scFv$_2$-Fc having two kinds of scFv obtained in the item 4 of Example 6 for their antigen TAG-72 or MUC1 was a binding specific for respective scFv moieties despite its sugar chain structures' differences.

3. Fcγ receptor IIIa binding activity of scFv$_2$-Fc having two kinds of scFv (ELISA)

Activities of the scFv$_2$-Fc having two kinds of scFv obtained in the item 4 of Example 6 to bind to FcγRIIIa(V) or FcγRIIIa(F) were measured in accordance with the method described in the item 3 of Example 3. In this case, the various scFv$_2$-Fc having two kinds of scFv was added at the time of the reaction in a concentration of 0 to 15 μg/ml. In addition, the color was developed using the TMB substrate solution, and OD450 was measured.

The results are respectively shown in Fig. 27 and Fig. 28. As shown in A of Fig. 27, it was confirmed that anti-MUC1 anti-TAG-72 scFvM-scFvT-Fc(-) and anti-MUC1 anti-TAG-72 scFvM-scFvT-Fc(+) bind concentration dependently to FcγRIIIa(V), and it was shown that the binding activity of anti-MUC1 anti-TAG-72 scFvM-scFvT-Fc(-) for FcγRIIIa(V) is higher than the binding activity of anti-MUC1 anti-TAG-72 scFvM-scFvT-Fc(+) for FcγRIIIa(V). This result was the same also in the case of FcγRIIIa(F) as shown in B of Fig. 27.

In addition, as shown in A of Fig. 28, it was able to confirm that anti-TAG-72 anti-MUC1 scFvT-scFvM-Fc(-) and anti-TAG-72 anti-MUC1 scFvT-scFvM-Fc(+) bind concentration dependently to FcγRIIIa(V), and it was shown that the binding activity of anti-TAG-72 anti-MUC1 scFvT-scFvM-Fc(-) for FcγRIIIa(V) is higher than the binding activity of anti-TAG-72 anti-MUC1 scFvT-scFvM-Fc(+) for FcγRIIIa(V). This result was the same also in the case of FcγRIIIa(F) as shown in B of Fig. 28.

Since binding was confirmed between respective scFv$_2$-Fc having two kinds of scFv obtained in the item 4 of Example 6 and the FcγRIIIa, it was shown that these Fc regions are expressed in the form with binding activity for FcγIIIa. In addition, since difference between the results of the binding activities for FcγIIIa due to the difference in the sugar chain structures of Fc regions were the same as the difference between the binding activities of the scFv-Fc having only one kind of scFv described in the item 3 of Example 3 or the item 3 of Example 5 for FcγIIIa due to difference in the Fc region sugar chain structures, the binding activity of the Fc region for FcγIIIa is maintained when made into a form of Fc fusion protein having two kinds ofscFv, and it was also the same that the binding activity is higher in the Fc region in which a fucose free sugar chain is bound than in the Fc region in which a fucose-added sugar chain is bound.

4. Fcγ receptor IIIa binding activity of scFv$_2$-Fc having two kinds of scFv in the presence of TAG-72 or MUC1 (ELISA)

Activities of the anti-TAG-72 anti-MUC1 scFv2-Fc fusion proteins to bind to FcγRIIIa(V) in the presence of TAG-72 or MUC1 antigen, were measured in accordance with the method described in the item 4 of Example 3. In this case, the color was developed using the TMB substrate solution (manufactured by Sigma), and OD450 was measured.

The results are shown in Fig. 29. Under each conditions in the presence of TAG-72 or in the presence of MUC1, concentration-dependent binding activities for FcγRIIIa(V) and the antigen TAG-72 were found in the anti-MUC1 anti-TAG-72 scFvM-scFvT-Fc(-) and anti-TAG-72 anti-MUC1 scFvT-scFvM-Fc(-) in which a fucose free sugar chain is bound, but the binding activities were not found in the anti-MUC1 anti-TAG-72 scFvM-scFvT-Fc(+) and anti-TAG-72 anti-MUC1 scFvT-scFvM-Fc(+)in which a fucose-added sugar chain is bound.

In addition, in the case of the anti-TAG-72 anti-MUC1 scFvM-scFvT-Fc(-) and anti-TAG-72 anti-MUC1 scFvT-scFvM-Fc(-), anti-TAG-72 anti-MUC1 scFvM-scFvT-Fc(-) was possessed of higher binding activity for FcγRIIIa(V). This result suggests that, when the number of scFv to be fused with the Fc region is two or more, strength of the binding of the Fc region to FcγRIIIa(V) changes depending on the order of scFv to be fused.

Based on the above, since difference between the binding activities for FcγIIIa due to difference in the sugar chain structures of Fc regions in the presence of the antigen were the same as the difference between binding activities of the scFv-Fc having only one kind of scFv described in the item 4 of Example 3 or the item 4 of Example 5 for FcγIIIa due to difference in the Fc region sugar chain structures from, the binding activity of the Fc region for FcγIIIa is maintained when made into a form of Fc fusion protein having two kinds of scFv, and it was also the same that the binding activity is higher in the Fc region in which a fucose free sugar chain is bound than in the Fc region in which a fucose-added sugar chain is bound.

From the results of the above-described item 3 and item 4, it was revealed that the binding activity of the Fc region for FcγIIIa is higher in the Fc region in which a fucose free sugar chain is bound than in the Fc region in which a fucose-added sugar chain is bound, regardless of the presence or absence of the antigen and regardless of the number of scFv to be fused with the Fc region.

5. Evaluation of cytotoxic activity of scFv$_2$-Fc having two kinds of scFv upon TAG-72 expressing cell line or MUC1 expressing cell line (ADCC activity, $^{51}$Cr dissociation method)

In order to evaluate *in vitro* cytotoxicity of the purified samples of the scFv$_2$-Fc having two kinds of scFv obtained in the item 4 of the above-described Example 6, the ADCC activity against a TAG-72-positive and MUC1-positive cell, human ovarian cancer-derived cell line OVCAR-3, a TAG-72-positive cell, human T cell-derived lymphoma cell line Jurkat, and an MUC1-positive cell, human ovarian cancer-derived cell line T-47D was measured in accordance with the method described in the item 5 of Example 3, using an effector cell collected from a healthy donor. In addition, a Raji cell which is a TAG-72-negative and MUC1-negative cell line was used as the negative control cell line. In this connection, the scFv$_2$-Fc having two kinds of scFv was added at the time of the reaction in a concentration of 0 to 15 μg/ml.

The results are shown in Fig. 30, Fig. 31 and Fig. 32.

As shown in A of Fig. 30, concentration-dependent ADCC activity for the TAG-72-positive cell, Jurkat cell, was observed in anti-MUC1 anti-TAG-72 scFvM-scFvT-Fc(-) or anti-MUC1 anti-TAG-72 scFvM-scFvT-Fc(+). In addition, the maximum cytotoxic activity was also high in anti-MUC1 anti-TAG-72 scFvM-scFvT-Fc(-) in comparison with anti-MUC1 anti-TAG-72 scFvM-scFvT-Fc(+). This result indicates that 1000 times higher concentration is necessary for anti-MUC1 anti-TAG-72 scFvM-scFvT-Fc(+) to show its ADCC activity equivalent to anti-MUC1 anti-TAG-72 scFvM-scFvT-Fc(-).

As shown in A of Fig. 31, the results were also the same when the MUC1-positive cell, T-47D cell, was used as the target cell.

Also, as shown in B of Fig. 30 or B of Fig. 31, concentration-dependent ADCC activity against the TAG-72-positive, Jurkat cell, or the MUC1-positive, T-47D cell, was observed in anti-TAG-72 anti-MUC1 scFvT-scFvM-Fc(-) or anti-TAG-72 anti-MUC1 scFvT-scFvM-Fc(+). In addition, in each target cell, the maximum cytotoxic activity was also high in anti-TAG-72 anti-MUC1 scFvT-scFvM-Fc(-) in comparison with anti-TAG-72 anti-MUC1 scFvT-scFvM-Fc(+), indicating that 1000 times higher concentration is necessary for anti-TAG-72 anti-MUC1 scFvT-scFvM-Fc(+) to show its ADCC activity equivalent to anti-TAG-72 anti-MUC1 scFvT-scFvM-Fc(-).

On the other hand, as shown in Fig. 32, ADCC activity against the TAG-72-negative and MUC1-negative Raji cell was not found in each of the scFv2-Fc having two kinds of scFv.

Based on the above, regarding the ratio of the Fc fusion protein in which fucose is not bound to N-acetylglucosamine in the reducing end in the complex type N-glycoside-linked sugar chain bound to the Fc fusion protein, among the total Fc fusion protein in each Fc fusion protein composition, there was a difference between anti-TAG-72 anti-MUC1 scFvM-scFvT-Fc(-) and anti-TAG-72 anti-MUC1 scFvM-scFvT-Fc(+) and between anti-TAG-72 anti-MUC1 scFvT-scFvM-Fc(-) and anti-TAG-72 anti-MUC1 scFvT-scFvM-Fc(+), and it was confirmed that this difference in the ratio is the difference in the FcγRIIIa binding activity between anti-TAG-72 anti-MUC1 scFvM-scFvT-Fc(-) and anti-TAG-72 anti-MUC1 scFvM-scFvT-Fc(+) or between anti-TAG-72 anti-MUC1 scFvT-scFvM-Fc(-) and anti-TAG-72 anti-MUC1 scFvT-scFvM-Fc(+), and this difference in the FcγRIIIa binding activity corresponds to the difference in ADCC activity. In addition, it was confirmed that, regarding the ADCC activities of the Fc fusion proteins having such bi-specificities, the cytotoxic activities are induced respectively and each independently for the two kinds of antigens TAG-72 and MUC1.

Example 8

Preparation of soluble type TNF-α receptor II-Fc fusion protein (sTNFRII-Fc)

1. Preparation of sTNFRII-Fc expression vector

(1) Construction of DNA encoding sTNFRII

A cDNA encoding the sTNFRII-Fc fusion protein described in USP 5605690 was constructed by the PCR method in the following manner. In this case, the cDNA was prepared into two cDNA sequences of the first half part represented by SEQ ID NO:28 and the latter half part represented by SEQ ID NO:29, by dividing it with the restriction enzyme *Blp*I site existing in the cDNA sequence encoding sTNFRII.

Firstly, a non-translation region of 87 bases and an sTNFRII secretory signal sequence were inserted into the 5'-terminal of the first half part of the sequence encoding sTNFRII of the sequence represented by SEQ ID NO:28. In addition, binding nucleotide sequences of primers for amplification use at the time of the PCR, also including restriction enzyme recognition sequences for cloning into a cloning vector and an expression vector, were added to the 5'-terminal and 3'-terminal of the sequence. Four sequences of synthetic DNA (manufactured by Fasmach) represented by SEQ ID NOs:30, 31, 32 and 33, respectively, were designed by dividing the thus designed nucleotide sequence represented by SEQ ID NO:28 into a total of 4 sequences starting from the 5'-terminal side and each having about 150 bases, in such a manner that the sense chain and antisense chain became alternate, and about 20 terminal bases of the nucleotide sequences adjoining each other were complementary for pairing.

The PCR was carried out by adding each oligonucleotide to a reaction solution containing 0.2 mM dNTPs and 1 mM magnesium chloride, to a final concentration of 0.1 μM, and adjusting the reaction solution to a total of 50 μl by using 0.4 μM of M13 primer RV (manufactured by Takara Shuzo Co., Ltd.), 0.4 μM of M13 primer M3 (manufactured by GENSET) and 2.5 units KOD Polymerase (manufactured by TOYOBO). The reaction was carried out by 25 cycles, one cycle consisting of reaction at 94°C for 30 seconds, reaction at 55°C for 30 seconds and reaction at 74°C for 60 seconds, and subsequent one cycle of reaction at 74°C for 5 minutes. After the PCR, the reaction solution was purified by QIA quick PCR purification kit (manufactured by QIAGEN), digested with a restriction enzyme *Kpn*I (manufactured by New England Biolabs), and then the reaction solution was subjected to agarose gel electrophoresis, and a PCR fragment of about 0.49 kb was recovered using QIAquick Gel Extraction Kit (manufactured by QIAGEN).

In the meantime, a plasmid pBluescript II SK(-) (manufactured by Stratagene) was digested with the restriction enzyme *Kpn*I (manufactured by New England Biolabs) and a restriction enzyme *Hin*dIII (manufactured by New England Biolabs) and then subjected to an agarose gel electrophoresis to recover a *Kpn*I-*Hin*dIII fragment of about 2.9 kb using QIAquick Gel Extraction Kit (manufactured by QIAGEN).

Next, the PCR fragment of about 0.49 kb of the first half part of sTNFRII and plasmid pBluescript II SK(-)-derived *Kpn*I-*Hin*dIII fragment, obtained in the above, were ligated using Ligation High solution (manufactured by TOYOBO) in accordance with the manufacture's instructions, and an *Escherichia coli* strain DH5a (manufactured by TOYOBO) was transformed using the reaction solution. Respective plasmid DNA samples were prepared from the thus obtained transformant clones and incubated using BigDye Terminator Cycle Sequencing Ready Reaction Kit ver. 3 (manufactured by Applied Biosystems) in accordance with the manufacture's instructions, and then nucleotide sequence of the PCR fragment inserted into each plasmid was analyzed using a DNA sequencer of the same company, ABI PRISM 377 to thereby confirm that the plasmid pBsIISK(-)/sTNFRII-1 shown in Fig. 33 having the desired nucleotide sequence was obtained.

Next, in the nucleotide sequence represented by SEQ ID NO:29, parts of the hinge and CH2 regions of human Fc were inserted into the latter half part of the sequence encoding sTNFRII and its 3'-terminal. In addition, binding nucleotide sequences of primers for amplification use at the time of the PCR, also including restriction enzyme recognition sequences for cloning into a cloning vector and an expression vector, were added to the 5'-terminal and 3'-terminal of the sequence. Four sequences of synthetic oligonucleotides (manufactured by Fasmach) represented by SEQ ID NOs: 34, 35, 36 and 37 were designed by dividing the thus designed nucleotide sequence represented by SEQ ID NO:29 into a total of 4 sequences starting from the 5'-terminal side and each having about 150 bases, in such a manner that the sense chain and antisense chain became alternate, and about 20 terminal bases of the nucleotide sequences adjoining each other were complementary for pairing.

The PCR was carried out by adding each oligonucleotide to a reaction solution containing 0.2 mM dNTPs and 1 mM magnesium chloride, to a final concentration of 0.1 μM, and adjusting the reaction solution to a total of 50 μl by using 0.4 μM of M13 primer RV (manufactured by Takara Shuzo Co., Ltd.), 0.4 μM of M13 primer M3 (manufactured by GENSET) and 2.5 units KOD polymerase (manufactured by TOYOBO). The reaction was carried out by 25 cycles, one cycle consisting of reaction at 94°C for 30 seconds, reaction at 55°C for 30 seconds and reaction at 74°C for 60 seconds, and subsequent 1 cycle of reaction at 74°C for 5 minutes. After the PCR, the reaction solution was purified by QIA quick PCR purification kit (manufactured by QIAGEN), digested with a restriction enzyme *Kpn*I (manufactured by New England Biolabs) and a restriction enzyme *Hin*dIII (manufactured by New England Biolabs), and then the reaction solution was subjected to agarose gel electrophoresis, and a PCR fragment of about 0.5 kb was recovered using QIAquick Gel Extraction Kit (manufactured by QIAGEN).

Next, the PCR fragment of about 0.5 kb of the latter half part of sTNFRII, obtained in the above, and the plasmid pBluescript II SK(-)-derived *Kpn*I-*Hin*dIII fragment obtained in the above were ligated using Ligation High solution (manufactured by TOYOBO), and an *Escherichia coli* strain DH5a (manufactured by TOYOBO) was transformed using the reaction solution. Respective plasmid DNA samples were prepared from the transformant clones and incubated using BigDye Terminator Cycle Sequencing Ready Reaction Kit ver. 3 (manufactured by Applied Biosystems) in accordance with the manufacture's instructions, and then nucleotide sequence of the PCR fragment inserted into each plasmid was analyzed using a DNA sequencer of the same company, ABI PRISM 377 to thereby confirm that the plasmid pBsIISK(-)/sTNFRII-2 shown in Fig. 34 having the desired nucleotide sequence was obtained.

(2) Construction of DNA encoding sTNFRII-Fc

A vector pKANTEX93 for expression of humanized antibody was digested with a restriction enzyme *Apa*I (manufactured by Takara Shuzo Co., Ltd.) and a restriction enzyme *Bam*HI (manufactured by New England Biolabs), and then the reaction solution was fractionated using an agarose gel electrophoresis. An *Apa*I-*Bam*HI fragment of about 1.0 kbp containing human IgG1 subclass H chain constant region (hCγ1) was recovered using QIAquick Gel Extraction Kit (manufactured by QIAGEN). In the same manner, a plasmid pBluescript II SK(-) (manufactured by Stratagene) was also digested with the restriction enzyme *Apa*I (manufactured by Takara Shuzo Co., Ltd.) and restriction enzyme *Bam*HI (manufactured by New England Biolabs), and then an *Apa*I-*Bam*HI fragment of about 2.9 kbp was recovered. The pKANTEX93-derived *Apa*I-*Bam*HI fragment of about 1.0 kbp and pBluescript II SK(-)-derived *Apa*I-*Bam*HI fragment of about 2.9 kbp were ligated using the solution I of TAKARA DNA Ligation Kit Ver. 2 (manufactured by Takara Shuzo Co., Ltd.), and the *Escherichia coli* strain DH5α (manufactured by TOYOBO) was transformed using the reaction solution to construct a plasmid pBsIISK(-)/hCγ1.

The plasmid pBsIISK(-)/sTNFRII-1 obtained in the above-described (1) was digested with a restriction enzyme *Kpn*I (manufactured by New England Biolabs) and a restriction enzyme *Blp*I (manufactured by New England Biolabs), and then the reaction solution was subjected to agarose gel electrophoresis, and a *Kpn*I-*Blp*I fragment of about 0.48 kb was recovered using QIAquick Gel Extraction Kit (manufactured by QIAGEN).

Also, the plasmid pBsIISK(-)/sTNFRII-2 obtained in the above-described (1) was digested with the restriction enzyme *Blp*I (manufactured by New England Biolabs) and a restriction enzyme *Sty*I (manufactured by New England Biolabs), and then the reaction solution was subjected to agarose gel electrophoresis, and a *Blp*I-*Sty*I fragment of about 0.49 kb was recovered using QIAquick Gel Extraction Kit (manufactured by QIAGEN).

In the meantime, the plasmid pBsIISK(-)/hCγ1 was digested with the restriction enzyme *Kpn*I (manufactured by New England Biolabs) and restriction enzyme *Sty*I (manufactured by New England Biolabs), and then the reaction solution was subjected to agarose gel electrophoresis, and a *Kpn*I-*Sty*I fragment of about 3.5 kbp was recovered using QIAquick Gel Extraction Kit (manufactured by QIAGEN).

The plasmid pBsIISK(-)/sTNFRII-1-derived *Kpn*I-*Blp*I fragment of about 0.48 kb, plasmid pBsIISK(-)/sTNFRII-2-derived *Blp*I-*Sty*I fragment of about 0.49 kb and plasmid pBsIISK(-)/hCγI-derived *Sty*I-*Kpn*I fragment of about 3.5 kbp, obtained in the above were ligated using Ligation High solution (manufactured by TOYOBO), and the *Escherichia coli* strain DH5a (manufactured by TOYOBO) was transformed using the reaction solution. Respective plasmid DNA samples were prepared from the thus obtained transformant clones and incubated using BigDye Terminator Cycle Sequencing Ready Reaction Kit ver. 3 (manufactured by Applied Biosystems) in accordance with the manufacture's instructions, and then nucleotide sequence of the fragment inserted into each plasmid was analyzed using a DNA sequencer of the same company, ABI PRISM 377 to thereby confirm that the plasmid pBsIISK(-)/sTNFRII-Fc shown in Fig. 35 was obtained.

(3) Construction of sTNFRII-Fc fusion protein expression vector

An sTNFRII-Fc fusion protein expression vector pKANTEX93/sTNFRII-Fc was constructed in the following manner using a vector pKANTEX93 for expression of humanized antibody and the plasmid pBsIISK(-)/sTNFRII-Fc obtained in the item (2).

The plasmid pBsIISK(-)/sTNFRII-Fc obtained in the item (2) was digested with a restriction enzyme *Eco*RI (manufactured by Takara Shuzo Co., Ltd.) and a restriction enzyme *Bam*HI (manufactured by New England Biolabs), and then the reaction solution was subjected to agarose gel electrophoresis, and an *Eco*RI-*Bam*HI fragment of about 1.6 kbp was recovered using QIAquick Gel Extraction Kit (manufactured by QIAGEN).

On the other hand, the vector plasmid pKANTEX93 for expression of humanized antibody was digested with the restriction enzymes *Eco*RI (manufactured by Takara Shuzo Co., Ltd.) and *Bam*HI (manufactured by New England Biolabs) and then subjected to an agarose gel electrophoresis to recover an *Eco*RI-*Bam*HI fragment of about 9.3 kbp using QIAquick Gel Extraction Kit (manufactured by QIAGEN).

Next, the pBsIISK(-)/sTNFRII-Fc-derived *Eco*RI-*Bam*HI fragment of about 1.6 kbp and the plasmid pKANTEX93-derived fragment of about 9.3 kbp obtained in the above were ligated using Ligation High solution (manufactured by TOYOBO), the *Escherichia coli* strain DH5a (manufactured by TOYOBO) was transformed using the reaction solution, respective plasmid DNA samples were prepared from the transformant clones and incubated using BigDye Terminator Cycle Sequencing Ready Reaction Kit ver. 3 (manufactured by Applied Biosystems) in accordance with the manufacture's instructions, and then nucleotide sequence of the fragment inserted into each plasmid was analyzed using a DNA sequencer of the same company, ABI PRISM 377 to thereby confirm that the plasmid pKANTEX93/sTNFRII-Fc shown in Fig. 36 was obtained.

2. Stable expression in FUT8 gene double knockout cell

Using Ms705 cell as the FUT8 gene double knockout cell described in the item 4 of Example 1 and its parent cell line CHO/DG44 cell as the host cells, the sTNFRII-Fc fusion protein expression vector pKANTEX93/sTNFRII-Fc prepared in the item 1 of this Example was introduced therein, and a cell stably producing the sTNFRII-Fc fusion protein was prepared by the method described in the item 2 of Example 2.

Finally, a transformant which can grow in the IMDM-dFBS(10) medium containing 600 $\mu$g/ml of G418 and 200 nM of MTX and also can produce the sTNFRII-Fc fusion protein was obtained. The transformant obtained from the FUT8 gene double knockout cell was named KC1194.

3. Purification of sTNFRII-Fc fusion proteins

The sTNFRII-Fc fusion proteins were purified from the sTNFRII-Fc fusion protein producing cells prepared in the item 2 of this Example by the method described in the item 4 of Example 2. Hereinafter, the purified sTNFRII-Fc fusion proteins are referred to as sTNFRII-Fc(-) produced by KC1194 and sTNFRII-Fc(+) produced by the parent cell line CHO/DG44 cell, respectively.

4. Analysis of purified sTNFRII-Fc fusion proteins

Purification degree of the sTNFRII-Fc(-) and sTNFRII-Fc(+) purified in the item 3 of this Example and the fucose content in the sugar chain added to the Fc region were confirmed in the following manner.

(1) Evaluation of the purification degree of sTNFRII-Fc(-) and sTNFRII-Fc(+)

SDS-PAGE was carried out using about 3 $\mu$g of each of the purified sTNFRII-Fc fusion proteins purified in the item 3 of this Example in accordance with the method described in the item 5(1) of Example 2.

The results are shown in Fig. 37. Each of the two kinds of purified proteins was detected as a band of about 140 kDa under non-reducing conditions and that of about 70 kDa under reducing conditions. This result coincides with the report stating that molecular weight of the sTNFRII-Fc fusion protein is about 140 kDa under non-reducing conditions, and the molecule is degraded into a composing unit of about 70 kDa under reducing conditions due to cleaving of its intramolecular S-S bond [*Proc. Natl. Acad. Sci. USA,* 36, 61 (1999)], and the electrophoresis patterns bear resemblance to each other in the case of sTNFRII-Fc(-) and sTNFRII-Fc(+) wherein their hosts are different, so that it was suggested that the sTNFRII-Fc(-) and sTNFRII-Fc(+) are expressed as polypeptide chains which coincide with the purpose.

(2) Monosaccharide composition analysis of purified sTNFRII-Fc fusion proteins

Monosaccharide composition analysis of the purified samples of sTNFRII-Fc(-) and sTNFRII-Fc(+) obtained in the item 3 of this Example was carried out in accordance with the method described in the item 5(2) of Example 2. However, since it is known that the binding site in the complex-type N-glycoside-linked sugar chain is present at two positions in the sTNFRII, and several O-glycoside binding type sugar chain binding sites are also present therein, fragments of Fc region were purified from each of the purified sTNFRII-Fc fusion proteins, and the monosaccharide composition analysis was carried out using the fragments.

A 500-$\mu$g portion of each of the purified sTNFRII-Fc fusion proteins and 5 $\mu$g of lysyl endopeptidase were suspended in 50 mmol/l Tris buffer at pH 8.5 and incubated at 37°C for 1 hour after adjusting the total volume to 5 ml. Just after the incubation, the fragments of Fc region were purified using MabSelect (manufactured by Pharmacia) column in accordance with the instructions.

The results are shown in Table 5. The ratio of the sugar chains in which fucose is not bound was 7% in the case of sTNFRII-Fc(+). On the other hand, the ratio of the sugar chains in which fucose is not bound was estimated to be almost 100% in the case of sTNFRII-Fc(-), because the peak of fucose was at or below the detection limit.

Based on the above results, it was shown that fucose is not bound to the N-acetylglucosamine in the reducing end in the complex type N-glycoside-linked sugar chain of sTNFRII-Fc(-).

Table 5 Ratio of sugar chains containing no fucose of sTNFRII-Fc fusion protein

| Protein name | Ratio of sugar chains containing no fucose (%) |
| --- | --- |
| sTNFRII-Fc(+) | 7% |
| sTNFRII-Fc(-) | ~100% |

Example 9

Evaluation of activity of sTNFRII-Fc fusion proteins

1. Reactivity of sTNFRII-Fc fusion proteins for anti-TNFRII antibody (ELISA)

An anti-TNFRII antibody (manufactured by R & D) was diluted to 1 µg/ml with PBS, dispensed at 50 µl/well into a 96-well plate for ELISA use (manufactured by Greiner) and incubated at 4°C overnight to effect its adhesion. After washing with PBS, 1% BSA-PBS was added thereto at 100 µl/well and incubated at a room temperature for 1 hour to block the remaining active groups. By removing 1% BSA-PBS, sTNFRII-Fc(-) or sTNFRII-Fc(+) was added thereto at 50 µl/well and incubated at a room temperature for 2 hours. After the reaction, each well was washed with Tween-PBS, and a peroxidase-labeled goat anti-human IgG (Fc) antibody solution (manufactured by American Qualex) diluted 500-fold with PBS was added thereto as the secondary antibody solution at 50 µl/well and incubated at a room temperature for 1 hour. After washing with Tween-PBS, the color was developed by adding the ABTS substrate solution at 50 µl/well, and OD415 was measured.

The results are shown in Fig. 38. It was confirmed that sTNFRII-Fc(-) and sTNFRII-Fc(+) bind to the anti-TNFRII antibody concentration-dependently, and their binding is almost the same, and it was shown that this is a binding specific for the TNFRII moiety of thus prepared two kinds of sTNFRII-Fc(-) and sTNFRII-Fc(+) having different sugar chain structures, and this binding is unrelated to the fucose content in the sugar chain to be added to the Fc of sTNFRII-Fc fusion proteins.

2. Binding activities of sTNFRII-Fc fusion proteins to Fcγ receptor IIIa (ELISA)

This was carried out in the same manner as in the method described in the item 3 of Example 3. However, concentration range of the measured sTNFRII-Fc fusion proteins was started from 100 nmol/l.

The results are shown in Fig. 39. A concentration-dependent binding was confirmed on sTNFRII-Fc(-) and sTNFRII-Fc(+), and the binding activity of sTNFRII-Fc(-) to FcγRIIIa was higher than the binding activity of sTNFRII-Fc(+), which was the same regardless of the polymorphism of the two kinds of FcγRIIIa. In addition, since binding was confirmed between sTNFRII-Fc(-) or sTNFRII-Fc(+) and FcγRIIIa, it was shown that the Fc region of sTNFRII-Fc has the normal three-dimensional structure which can bind to FcγRIIIa.

3. Neutralization activity of sTNFRII-Fc fusion proteins to TNF-α

Measurement of the neutralization activity was carried out using a TNF-α-sensitive cell, L929 cell [*J. Natl. Cancer Inst.,* 9, 229 (1948)]. L929 cells cultured using MEM-FBS(10) medium (MEM medium containing 10% PBS and 10 µg/ml of gentamicin) were treated with trypsin-EDTA (manufactured by GIBCO-BRL), and then recovered, adjusted to $3 \times 10^5$ cells/ml MEM-PBS(10) medium and dispensed at 100 µl/well into a 96-well flat bottom plate. After culturing at 37°C for 24 hours in a 5% $CO_2$ incubator, the medium was removed in such a manner that the cells were not sucked. To each well were added 100 µl of MEM-PBS(10) medium, 50 µl of 0.05 ng/ml of mouse TNF-α (manufactured by R & D), 50 µl of 5-fold concentration of each final concentration of sTNFRII-Fc(-) or sTNFRII-Fc(+) and 50 µl of 2.5 µg/ml of actinomycin D (manufactured by MBL) to give a total volume of 250 µl/well, and the culturing was continued for additional 24 hours. The medium was removed in such a manner that the cells were not sucked, PBS(-) was added at 100 µl/well and the PBS(-) was again removed in such a manner that the cells were not sucked, and then the cells were air-dried for 10 minutes or more. A solution of 0.05% Crystal Violet (manufactured by Wako Pure Chemical Industries) was added at 50 µl/well and incubated for 10 minutes or more. By adding methanol (manufactured by Nakalai Tesque) at 150 µl/well, absorbance at 590 nm was measured using a plate reader. By defining the cells at the time of not adding TNF-α in the presence of actinomycin D as 100%, and the cells at the time of adding the same as 0%, the neutralization activity to TNF-α at each concentration was calculated.

The results are shown in Fig. 40. Since sTNFRII-Fc(-) and sTNFRII-Fc(+) concentration-dependently inhibited the activity of mouse TNF-α, and the neutralization activities were almost the same, it was confirmed that there is no difference in the neutralization activities to TNF-α caused by the sugar chains of the prepared two kinds of sTNFRII-Fc(-) and sTNFRII-Fc(+) having different sugar chain structures.

4. ADCC activities of sTNFRII-Fc fusion proteins (lactate dehydrogenase method)

ADCC activities of sTNFRII-Fc fusion proteins upon a membrane type human TNF-α expressing mouse T cell tumor strain EL4 (ATCC TIB-39) (hereinafter referred to as "TNF-α/EL4") were measured in the following manner using a peripheral blood monocyte fraction collected from a healthy donor as the effector cell.

(1) Preparation of TNF-α/EL4

(1-1) Preparation of human lymph node-derived single-stranded cDNA

A single-stranded cDNA was synthesized from 1 μg of human lymph node-derived poly A+ RNA (manufactured by BD Biosciences Clontech) using SuperScript™ First-Strand Synthesis System for RT-PCR (manufactured by Invitrogen) in accordance with the manufacture's instructions. After the synthesis, the final solution volume was adjusted to 1 ml, and a 5-fold diluted solution of this was used in the following reaction.

(1-2) Preparation of cDNA encoding membrane type human TNF-α

When a TNF-α in which 12 residues of the 77th to 88th positions having a recognition site by a protease are deleted from the full length sequence of human TNF-α is expressed in NIH 3T3 cell, the TNF-α is not cleaved by the protease, so that the human TNF-α is expressed on the cell membrane [*Cell,* 63, 251 (1990)]. Accordingly, a cDNA encoding a human TNF-α in which 12 residues of the 77th to 88th positions were deleted was constructed in the following manner.

Using 5 μl of the cDNA solution prepared in the above item (1-1) as the template and adding 0.4 μM in final concentration of the synthetic DNA samples respectively represented by SEQ ID NO:100 and SEQ ID NO:101 as the primers, a PCR solution [1 unit of KOD-Plus-DNA Polymerase, 0.2 mM dNTPs, 1 mM magnesium sulfate, 1 × concentration of KOD-Plus-DNA Polymerase Buffer (all manufactured by TOYOBO)] was prepared, and using a DNA thermal cycler GeneAmp PCR System 9700 (manufactured by Applied Biosystems), the solution was heated at 94°C for 4 minutes, and then the reaction was carried out by 30 cycles, one cycle consisting of reaction at 94°C for 30 seconds, reaction at 55°C for 30 seconds and reaction at 68°C for 60 seconds. By this reaction, a moiety of about 440 bp of the C-terminal side of human TNF-α is amplified. After the PCR, the reaction solution was purified using QIAquick PCR Purification Kit (manufactured by QIAGEN), digested with a restriction enzyme *Eco*RI (manufactured by Takara Shuzo Co., Ltd.) and a restriction enzyme *Bam*HI (manufactured by Takara Shuzo Co., Ltd.) and then subjected to an agarose gel electrophoresis, and a PCR fragment of about 440 bp was recovered using QIAquick Gel Extraction Kit (manufactured by QIAGEN).

The plasmid pBluescript II SK(-) (manufactured by Stratagene) was digested with a restriction enzyme *Afl*III (manufactured by New England Biolabs), smooth-ended using a modification enzyme Mung Bean Nuclease (manufactured by Takara Shuzo Co., Ltd.) and then subjected to an autonomous ligation reaction using Ligation High (manufactured by TOYOBO). The *Escherichia coli* strain DH5α (manufactured by TOYOBO) was transformed using the reaction solution, and a plasmid DNA was prepared from the thus obtained transformant clones to thereby obtain a plasmid pBSAflIII(-) from which the *Afl*III recognition site was deleted.

The thus obtained plasmid pBSAflIII(-) was digested with the restriction enzyme *Eco*RI (manufactured by Takara Shuzo Co., Ltd.) and the restriction enzyme *Bam*HI (manufactured by Takara Shuzo Co., Ltd.) and then subjected to an agarose gel electrophoresis, and an *Eco*RI-*Bam*HI fragment of about 2.9 kbp was recovered in the same manner.

The PCR fragment of about 440 bp and plasmid pBSAflIII(-)-derived *Eco*RI-*Bam*HI fragment of about 2.9 kbp obtained in the above were ligated using Ligation High (manufactured by TOYOBO), and the *Escherichia coli* strain DH5α (manufactured by TOYOBO) was transformed using the reaction solution. Plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the plasmid ΔTNF-αpBSAflIII(-) of interest was obtained.

Next, an N-terminal sequence of about 300 bp of the human TNF-α was divided into a total of 4 nucleotide sequences starting from the 5'-end side, by designing in such a manner that the sense chain and antisense chain became alternate, and the sequences adjoining each other were arranged in such a manner that about 20 terminal bases thereof were overlapped and they can therefore be paired. By further adding restriction enzyme recognition sequences for cloning use to the termini of SEQ ID NO:102 and SEQ ID NO:105, 4 sequences of synthetic DNA (manufactured by Fasmach) of SEQ ID NOs:102, 103, 104 and 105 were synthesized.

Using 0.1 μM in final concentration of each oligonucleotide, and further adding 0.5 μM in final concentration of the synthetic oligonucleotides of SEQ ID NO:106 and SEQ ID NO: 107 as the amplification primers, a PCR solution [2 units of KOD-Plus-DNA Polymerase, 0.2 mM dNTPs, 1 mM magnesium sulfate, 1 × concentration of KOD-Plus-DNA Polymerase Buffer (all manufactured by TOYOBO)] was prepared, and using a DNA thermal cycler GeneAmp PCR System 9700 (manufactured by Applied Biosystems), the reaction was carried out by 25 cycles, one cycle consisting of reaction at 94°C for 30 seconds, reaction at 55°C for 30 seconds and reaction at 74°C for 60 seconds, subsequently carrying out 1 cycle of reaction at 74°C for 5 minutes. After the PCR, the reaction solution was purified using QIAquick PCR Purification Kit (manufactured by QIAGEN), digested with a restriction enzyme *Eco*RI (manufactured by Takara Shuzo Co., Ltd.) and a restriction enzyme *Afl*III (manufactured by New England Biolabs) and then subjected to an agarose gel electrophoresis, and a PCR fragment of about 300 bp was recovered using QIAquick Gel Extraction Kit (manufactured by QIAGEN).

The plasmid ΔTNF-αpBSAflIII(-) obtained in the above was digested with the restriction enzyme *Eco*RI (manufactured by Takara Shuzo Co., Ltd.) and restriction enzyme *Afl*III (manufactured by New England Biolabs) and then subjected to an agarose gel electrophoresis to recover an *Eco*RI-*Afl*III fragment of about 3.2 kbp.

The PCR fragment of about 300 bp and plasmid ΔTNF-αpBSAflIII(-)-derived *Eco*RI-*Afl*III fragment of about 3.2 kbp obtained in the above were ligated using Ligation High (manufactured by TOYOBO), and the *Escherichia coli* strain DH5α (manufactured by TOYOBO) was transformed using the reaction solution. Plasmid DNA samples were prepared from the thus obtained transformant clones and incubated using BigDye Terminator Cycle Sequencing Ready Reaction Kit v3.0 (manufactured by Applied Biosystems) in accordance with the manufacture's instructions, and then nucleotide sequence of the cDNA inserted into each plasmid was analyzed using a DNA sequencer of the same company, ABI PRISM 377 to thereby confirm that the desired plasmid Δ1-12TNF-αpBS containing a cDNA encoding the membrane type human TNF-α was obtained. The cDNA sequence of the constructed membrane type human TNF-α is represented by SEQ ID NO:108, and a deduced amino acid sequence of the membrane type human TNF-α in SEQ ID NO: 109, respectively.

(1-3) Construction of membrane type human TNF-α expression vector

The plasmid Δ1-12TNF-αpBS obtained in the above item (1-2) was digested with the restriction enzymes *Eco*RI (manufactured by Takara Shuzo Co., Ltd.) and *Bam*HI (manufactured by Takara Shuzo Co., Ltd.) and then subjected to an agarose gel electrophoresis to recover an *Eco*RI-*Bam*HI fragment of about 0.72 kbp in the same manner as described in the above.

The plasmid Δ1-12TNF-αpBS-derived *Eco*RI-*Bam*HI fragment of about 0.72 kbp obtained in the above and the *Eco*RI-*Bam*HI fragment of about 9.3 kbp derived from the vector pKANTEX93 for expression of humanized antibody, prepared in the item 1(3) of Example 8, were ligated using Ligation High (manufactured by TOYOBO), and the *Escherichia coli* strain DH5α (manufactured by TOYOBO) was transformed using the reaction solution. As a result of preparing plasmid DNA samples from the thus obtained transformant clones, it was confirmed that the membrane type human TNF-α expression vector pKANTEXΔ1-12TNF-α shown in Fig. 41 was obtained.

(1-4) Preparation of TNF-α/EL4

An 8-μg portion of the plasmid pKANTEXΔ1-12TNF-α obtained in the above item (1-3) was introduced into $3 \times 10^6$ cells of the EL4 cell by the electroporation method [*Cytotechnology, 3,* 133 (1990)], and then the cells were suspended in 60 ml of RPMI1640(10) medium [RPMI1640 medium containing 10% FCS, (manufactured by Invitrogen)] and dispensed at 200 μl/well into a 96-well microplate (manufactured by Sumitomo Bakelite). After culturing at 37°C for 24 hours in a 5% $CO_2$ incubator, the culturing was continued for 1 to 2 weeks using the RPMI1640(10) medium containing G418 in a concentration of 0.5 mg/ml. After the culturing, expression of the membrane type human TNF-α was examined in the following manner using drug-resistance strains whose growth was confirmed.

Each of the drug-resistant strains or the parent cell line EL4 cell was suspended in 1% BSA-PBS containing 40-fold diluted human immunoglobulin solution (manufactured by Welfide) and 5-fold diluted FITC-labeled anti-human TNF-α antibody solution (manufactured by R & D), to a density of $5 \times 10^5$ cells/50 μl, dispensed into a 96-well U-shape bottom plate and incubated at 4°C for 30 minutes under shade. After the incubation, the cells were washed twice with 1% BSA-PBS, suspended in 1 ml of PBS and then analyzed using a flow cytometer.

The results are shown in Fig. 42. As shown in Fig. 42, expression of the membrane type human TNF-α was confirmed in the drug-resistant strain 7. On the other hand, expression of the membrane type human TNF-α was not found in the parent cell line EL4 cell.

(2) Preparation of effector cell suspension

A 50-ml portion of peripheral blood was collected from a healthy person, and 0.3 ml of heparin sodium (manufactured by Shimizu Pharmaceutical) was added thereto and gently mixed. A monocyte layer was separated from this using Lymphoprep (manufactured by Axis-Shield) in accordance with the instructions. The cells were centrifuged and washed twice with RPMI1640F(-)-FCS(5) medium [RPMI1640 medium (manufactured by Invitrogen) containing 5% FCS and 1% penicillin-streptomycin (manufactured by Invitrogen) but not containing Phenol Red], and then adjusted to $5 \times 10^6$ cells/ml by adding RPMI1640F(-)-FCS(5) medium and was used as the effector cell suspension.

(3) Preparation of target cell suspension

The TNF-α/EL4 (drug-resistant clone 7) prepared in the above was suspended in RPMI1640F(-)-FCS(5) to a density of $2 \times 10^5$ cells/ml and used as the target cell suspension.

(4) Measurement of ADCC activity

The effector cell suspension prepared in the above-described (2) was dispensed at 50 μl into each well of a 96-well U-bottom plate (manufactured by Falcon) ($2.5 \times 10^5$ cells/well). Next, the target cell suspension prepared in the above-described (3) was dispensed at 50 μl ($1 \times 10^4$ cells/well), and was added so that the ratio of the effector cells to target cells becomes 25:1. Furthermore, each of the various sTFNRII-Fc fusion proteins prepared in the item 2 of this Example was added thereto to a final concentration of 0.00001 to 1 μg/ml while adjusting the total volume to 150 μl and then, after centrifugation (700 rpm, 5 minutes), was incubated at 37°C for 4 hours. After the incubation, the reaction suspension was separated into cells and supernatant by centrifugation (1200 rpm, 5 minutes), and the supernatant was dispensed at 50 μl into a 96-well flat bottom plate. The substrate reaction solution attached to the CytoTox96-Non-Radioactive Cytotoxicity Assay (manufactured by Promega) was added at 50 μl/well to the dispensed supernatant and incubated at a room temperature for 30 minutes under shade. After the incubation, absorbance at OD 490 nm was measured by adding the Stop solution attached thereto at 50 μl/well, and the amount of lactate dehydrogenase (hereinafter referred to as "LDH") in the supernatant was measured. The specific LDH releasing activity of sTNFRII-Fc fusion protein was calculated by subtracting the value of well containing the target cell and effector cell alone from each measured value. Regarding the total LDH content of the target cell, the value of a well to which 1/10 volume of the Lysis buffer attached thereto was added at the time of the reaction was measured, and regarding the amount of LDH spontaneously released from the target cell, the value of a well in which the reaction was carried out using the medium alone was measured. The ADCC activity (%) was calculated from these values in accordance with the following formula.

$$
\begin{aligned}
&\text{ADCC activity (\%)} \\
&= \{\text{measured value of specific LDH at each sample concentration} \\
&\quad /(\text{measured value of total LDH} \\
&\quad - \text{measured value of spontaneously released LDH})\} \\
&\quad \times 100
\end{aligned}
$$

The results are shown in Fig. 43. The sTNFRII-Fc(-) showed concentration-dependent ADCC activity against TNF-α/EL4. On the other hand, only very low ADCC activity was found in sTNFRII-Fc(+) within the measured concentration range. The above results show a possibility that ADCC activity of Fc fusion proteins can be markedly increased by removing fucose of N-acetylglucosamine in the reducing end of the complex type N-glycoside-linked sugar chain of the antibody Fc region.

Example 10

Preparation of CD2 binding LFA-3 domain-Fc fusion protein (LFA-3-Fc):

1. Preparation of LFA-3-Fc fusion protein expression vector

(1) Construction of a DNA encoding CD2 binding LFA-3 domain

A cDNA encoding the Fc fusion protein of CD2 binding LFA-3 domain described in USP 5914111 was constructed in the following manner.
In the nucleotide sequence represented by SEQ ID NO:38, a non-translation region of 9 bases and a secretion signal sequence of LFA-3 were integrated into 5'-terminal of the sequence encoding the CD2 binding LFA-3 domain. A human Fc hinge (5 residues were deleted from the N-terminus) and a part of the $CH_2$ region were integrated into the 3'-terminal. In addition, binding nucleotide sequences of primers for amplification use at the time of PCR, also including restriction enzyme recognition sequences for cloning into a cloning vector and an expression vector, were added to the 5'-terminal and 3'-terminal of the sequence. Four sequences of synthetic oligonucleotides (manufactured by Fasmach) of SEQ ID NOs:39, 40, 41 and 42, respectively, were designed by dividing the thus designed nucleotide sequence represented by SEQ ID NO:38 into a total of 4 nucleotide sequences starting from the 5'-terminal side and each having approximately from 120 to 140 bases in such a manner that the sense chain and antisense chain became alternate, and about 20 terminal bases of the sequences adjoining each other were complementary for pairing.

PCR was carried out by adding each oligonucleotide to a reaction solution containing 0.2 mM dNTPs and 1 mM magnesium chloride to a final concentration of 0.1 $\mu$M, and adjusting the reaction solution to a total of 50 $\mu$M by using 0.4 $\mu$M of M13 primer RV (manufactured by Takara Shuzo Co., Ltd.), 0.4 $\mu$M M13 primer of M3 (manufactured by GENSET) and 2.5 units of KOD polymerase (manufactured by TOYOBO). The reaction was carried out by 25 cycles, one cycle consisting of reaction at 94°C for 30 seconds, reaction at 55°C for 30 seconds and reaction at 74°C for 60 seconds, and subsequent 1 cycle of reaction at 74°C for 5 minutes. The reaction solution was purified using QIA quick PCR purification kit (manufactured by QIAGEN), digested with a restriction enzyme *Kpn*I (manufactured by New England Biolabs) and a restriction enzyme *Alw*NI (manufactured by New England Biolabs) and then subjected to an agarose gel electrophoresis, and a *Kpn*I-*Alw*NI fragment of about 0.42 kb was recovered using QIAquick Gel Extraction Kit (manufactured by QIAGEN).

Next, the plasmid pBsIISK(-)/hCγ1 prepared in the item 1(2) of Example 8 was digested with the restriction enzyme *Alw*NI (manufactured by New England Biolabs) and restriction enzyme *Afl*III (manufactured by New England Biolabs) and then subjected to an agarose gel electrophoresis to recover an *Alw*NI-*Afl*III fragment of about 1.1 kb using QIAquick Gel Extraction Kit (manufactured by QIAGEN). On the other hand, the same plasmid was digested with the restriction enzyme *Afl*III (manufactured by New England Biolabs) and restriction enzyme *Kpn*I (manufactured by New England Biolabs) and then subjected to an agarose gel electrophoresis to recover an *Afl*III-*Kpn*I fragment of about 2.5 kb using QIAquick Gel Extraction Kit (manufactured by QIAGEN).

The PCR derived *Kpn*I-*Alw*NI fragment of about 0.42 kb, plasmid pBsIISK(-)/hCγ1 derived *Alw*NI-*Afl*III fragment of about 2.5 kb and plasmid pBsIISK(-)/hCγl derived *Afl*III-*Kpn*I fragment obtained in the above were ligated using Ligation High solution (manufactured by TOYOBO) and in accordance with the manufacture's instructions. The *Escherichia coli* strain DH5a (manufactured by TOYOBO) was transformed using the recombinant plasmid DNA solution obtained in this manner, each plasmid DNA samples were prepared from the resulting transformant clones and incubated using BigDye Terminator Cycle Sequencing Ready Reaction Kit ver. 3 (manufactured by Applied Biosystems) in accordance with the manufacture's instructions, and then nucleotide sequence of the PCR fragment inserted into each plasmid was analyzed using a DNA sequencer of the same company, ABI PRISM 377 to thereby confirm that the plasmid pBsIISK(-)/LFA-3-Fc shown in Fig. 44 having the desired nucleotide sequence was obtained.

(2) Construction of LFA-3-Fc fusion protein expression vector

An LFA-3-Fc fusion protein expression vector pKANTEX93/LFA-3-Fc was constructed in the following manner using the vector pKANTEX93 for expression of humanized antibody and the plasmid pBsIISK(-)/LFA-3-Fc obtained in the item (1).

The plasmid pBsIISK(-)/LFA-3-Fc obtained in the item (1) was digested with a restriction enzyme *Eco*RI (manufactured by Takara Shuzo Co., Ltd.) and a restriction enzyme *Xcm*I (manufactured by New England Biolabs) and then the reaction solution was subjected to agarose gel electrophoresis to recover an *Eco*RI-*Xcm*I fragment of about 0.1 kb using QIAquick Gel Extraction Kit (manufactured by QIAGEN).

Next, the vector pKANTEX93 for expression of humanized antibody was digested with the restriction enzymes *Xcm*I (manufactured by New England Biolabs) and *Bam*HI (manufactured by New England Biolabs) and then the reaction solution was subjected to agarose gel electrophoresis to recover an *Xcm*I-*Bam*HI fragment of about 1 kb using QIAquick Gel Extraction Kit (manufactured by QIAGEN). The same plasmid was digested with the restriction enzymes *Bam*HI (manufactured by New England Biolabs) and *Eco*RI (manufactured by Takara Shuzo Co., Ltd.) and then the reaction solution was subjected to agarose gel electrophoresis to recover a *Bam*HI-*Eco*RI fragment of about 9.3 kb using QIAquick Gel Extraction Kit (manufactured by QIAGEN).

Next, the pBsIISK(-)/LFA-3-Fc derived *Eco*RI-*Xcm*I fragment and plasmid pKANTEX93 derived *Xcm*I-*Bam*HI fragment obtained in the above were ligated using Ligation High solution (manufactured by TOYOBO) and in accordance with the manufacture's instructions. The *Escherichia coli* strain DH5a (manufactured by TOYOBO) was transformed using the recombinant plasmid DNA solution obtained in this manner, and each of plasmid DNA samples was prepared from the resulting transformant clones to thereby analyze the nucleotide sequences by a DNA sequencer of the same company, ABI PRISM 377 using BigDye Terminator Cycle Sequencing Ready Reaction Kit ver. 3 (manufactured by Applied Biosystems). As a result of the analysis, it was confirmed that the plasmid pKANTEX93/LFA-3-Fc shown in Fig. 45 having the desired nucleotide sequence was obtained.

2. Stable expression in FUT8 gene double knockout cell

Using the FUT8 gene double knockout cell described in the item 4 of Example 1 and its parent cell line CHO/DG44 cell as the host cells, the LFA-3-Fc fusion protein expression vector pKANTEX93/LFA-3-Fc prepared in the item 1 of this Example was introduced therein, and a cell stably producing the LFA-3-Fc fusion protein was prepared in accordance with the method described in the item 2 of Example 2. However, gene amplification using dihydrofolate reductase gene was not performed.

Finally, a transformant which can grow in the IMDM-dFBS(10) medium containing 600 μg/ml of G418 and also can produce the LFA-3-Fc fusion protein was obtained. The transformant obtained from the FUT8 gene double knockout cell was named KC1198.

3. Purification of CD2 binding LFA-3 domain-Fc fusion proteins

The LFA-3-Fc fusion protein producing cells prepared in the item 2 of the above-described Example 6 were cultured at a 1000 ml scale using EXCELL 301 medium (manufactured by JRH). LFA-3-Fc fusion proteins were purified from the culture supernatants in accordance with the method described in the item 4 of Example 2. Hereinafter, the purified LFA-3-Fc fusion proteins are referred to as LFA-3-Fc(+) produced by the parent cell line CHO/DG44 cell and LFA-3-Fc(-) produced by KC 1198, respectively.

4. Analysis of purified LFA-3-Fc fusion proteins

Purification degree of the LFA-3-Fc(-) and LFA-3-Fc(+) purified in the item 3 of this Example and the fucose content in the sugar chain added to the Fc region were confirmed in the following manner.

(1) Evaluation of the purification degree of LFA-3-Fc(-) and LFA-3-Fc(+)

The SDS-PAGE was carried out using about 2 μg of each of the purified LFA-3-Fc fusion proteins in accordance with the method described in the item 5(1) of Example 2. The results are shown in Fig. 46. Each of the two kinds of purified proteins was detected as a band of about 115 kDa under non-reducing conditions and that of about 60 kDa under reducing conditions. This result coincides with the report stating that molecular weight of the LFA-3-Fc fusion protein is about 11 5 kDa under non-reducing conditions, and the molecule is degraded into a composing unit of about 60 kDa under reducing conditions due to cleaving of its intramolecular S-S bond [*Proc. Natl. Acad. Sci. USA,* 36, 61 (1999)], and the electrophoresis patterns bear resemblance in the case of the two kinds of LFA-3-Fc(-) and LFA-3-Fc (+) wherein their hosts are different, so that it was suggested that the LFA-3-Fc(-) and LFA-3-Fc(+) are expressed as the polypeptide chains encoded by the expression vector.

(2) Monosaccharide composition analysis of purified LFA-3-Fc fusion proteins

Monosaccharide composition analysis of the purified samples of LFA-3-Fc(-) and LFA-3-Fc(+) obtained in the item 3 of this Example was carried out in accordance with the method described in the item 5(2) of Example 2. However, since it is known that the binding site in the complex type N-glycoside-linked sugar chain is present at three positions in the CD2 binding LFA-3 domain (USP 5614111, fragments of Fc region were purified from each of the purified LFA-3-Fc fusion proteins, and the monosaccharide composition analysis was carried out using the fragments.

A 500-μg portion of each of the purified LFA-3-Fc fusion proteins and 5 μg of lysyl endopeptidase were suspended in 50 mmol/l Tris buffer at pH 8.5 and incubated at 37°C for 1 hour after adjusting the total volume to 5 ml. Just after the reaction, the Fc fragments were purified using MabSelect (manufactured by Pharmacia) column in accordance with the instructions.

The results are shown in Table 6. The ratio of the sugar chains in which fucose is not bound was 7% in the case of LFA-3-Fc(+). On the other hand, the ratio of the sugar chains in which fucose is not bound was estimated to be almost 100% in the case of LFA-3-Fc(-), because the peak of fucose was at or below the detection limit.

Based on the above results, it was shown that fucose is not bound to the N-acetylglucosamine in the reducing end in the complex type N-glycoside-linked sugar chain of LFA-3-Fc(-).

Table 6 Ratio of sugar chains containing no fucose of LFA-3-Fc fusion protein

| Protein name | Ratio of sugar chains containing no fucose (%) |
|---|---|
| LFA-3-Fc(+) | 7% |
| FA-3-Fc(-) | ~100% |

Example 11

Evaluation of activity of CD2 binding LFA-3 domain-Fc fusion proteins

1. Binding ability to CD2 molecule on the membrane surface (fluorescent antibody technique)

Reactivity for the CD2 having the binding activity to the LFA-3-Fc fusion proteins LFA-3-Fc(-) and LFA-3-Fc (+), prepared in the item 3 of Example 6, was examined by the fluorescent antibody technique in the following manner. A CCRF-CEM cell (ATCC CCL-119) was used as the CD2 expressing cell line.

The CCRF-CEM cell was dispensed into a 96-well U-shape plate at a density of $2\times10^5$ cells per well, and each of the solutions prepared by optionally diluting LFA-3-Fc(-) and LFA-3-Fc(+) with FACS buffer starting from 435 nmol/l by a conventionally known method [*Kohso Kohtai* Hoh *(Enzyme Antibody Method)*: published by Gakusai Kikaku (1985)] was added at 100 $\mu$l/well and incubated on ice for 30 minutes. After washing twice with the FACS buffer, a solution prepared by diluting an PE-labeled anti-human IgG (Fc$\gamma$) antibody (manufactured by Beckman Coulter) 50-fold with the FACS buffer was added thereto at 50 $\mu$l/well. After the reaction on ice for 30 minutes under shade, the cells were washed three times with the FACS buffer, and the fluorescence intensity was measured using a flow cytometer.

As shown in Fig. 47, the binding activities of LFA-3-Fc(-) and LFA-3-Fc(+)to CD2 were almost the same. Though it has been reported that influence of sugar chain modification upon the binding of CD2 and LFA-3 is considerable [*Trends in Glycoscience and Glycotechnology,* 11, 1 (1999)], it was shown that the LFA-3-Fc(-) molecule in which fucose is not bound does not exert influence upon the binding activity.

2. Binding activities to Fc$\gamma$RIIIa

This was carried out in the same manner as in the method described in the item 3 of Example 3. However, concentration range of the measured LFA-3-Fc fusion proteins was started from 33 nmol/l.

The results are shown in Fig. 48. It was confirmed that LFA-3-Fc(-) and sLFA-3-Fc(+) concentration-dependently bind to Fc$\gamma$RIIIa, and the activity of LFA-3-Fc(-) to bind to Fc$\gamma$RIIIa was higher than that of LFA-3-Fc(+). This result was the same result of two kinds of the Fc$\gamma$RIIIa polymorphism.

Since differences in the binding activity for Fc$\gamma$RIIIa and the fucose content of the sugar chain which binds to the Fc region are found between LFA-3-Fc(-) and LFA-3-Fc(+), it clearly shows that the difference in the binding activity for Fc$\gamma$RIIIa is originates from the difference in the fucose content of the sugar chain which binds to the Fc region. On the other hand, since the fucose content of the LFA-3 region does not exert influence upon the binding to CD2, it clearly shows that the effector activity mediated by the antibody Fc region can be artificially controlled by removing the fucose of N-acetylglucosamine in the reducing end in the complex type N-glycoside-linked sugar chain which binds to the antibody Fc region.

3. ADCC activities of CD2 binding LFA-3 domain-Fc fusion proteins

ADCC activities of CD2 binding LFA-3 domain-Fc fusion proteins upon a CD2-positive human T cell lymphoma cell line Jurkat were measured in the following manner using a peripheral blood monocyte fraction collected from a healthy donor as the effector cell.

(1) Preparation of effector cell suspension

A 50-ml portion of peripheral blood was collected from a healthy person, and 0.3 ml of heparin sodium (manufactured by Shimizu Pharmaceutical) was added thereto and gently mixed. A monocyte layer was separated from this using Lymphoprep (manufactured by Axis-Shield) and in accordance with the instructions. The cells were centrifuged and washed twice with RPMI1640F(-)-FCS(5) medium [RPMI1640 medium (manufactured by Invitrogen) containing 5% FCS and 1% penicillin-streptomycin (manufactured by Invitrogen) but not containing Phenol Red], and then adjusted to $5\times10^6$ cells/ml by adding RPMI1640F(-)-FCS(5) medium and was used as the effector cell suspension.

(2) Preparation of target cell suspension

The Jurkat cell was suspended in RPMI1640F(-)-FCS(5) medium to a density of $2\times10^5$ cells/ml and used as the target cell suspension.

(3) Measurement of ADCC activity

The effector cell suspension prepared in the item (1) was dispensed at 50 μl into each well of a 96-well U-bottom plate (manufactured by Falcon) ($2.5 \times 10^5$ cells/well). Next, the target cell suspension prepared in the item (2) was dispensed at 50 μl ($1 \times 10^4$ cells/well) and was added so that the ratio of the effector cells to target cells becomes 25:1. Furthermore, each of the various LFA-3-Fc fusion proteins prepared in the item 2 of this Example was added thereto to a final concentration of 0.00001 to 10 μg/ml while adjusting the total volume to 150 μl and then, after centrifugation (700 rpm, 5 minutes), was incubated at 37°C for 4 hours. After the incubation, the reaction solution was separated into cells and supernatant by centrifugation (1200 rpm, 5 minutes), and the supernatant was dispensed at 50 μl into a 96-well flat bottom plate. The substrate reaction solution attached to the CytoTox96-Non-Radioactive Cytotoxicity Assay (manufactured by Promega) was added at 50 μl/well to the dispensed supernatant and incubated at a room temperature for 30 minutes under shade. After the incubation, absorbance at OD 490 nm was measured by adding the Stop solution attached thereto at 50 μl/well, and the amount of lactate dehydrogenase in the supernatant was measured and used as the cytotoxic activity. The specific cytotoxic activity of LFA-3-Fc fusion protein was calculated by subtracting the value of well containing the target cell and effector cell alone from each measured value.

The results are shown in Fig. 49. The LFA-3-Fc(-) and LFA-3-Fc(+) showed concentration-dependent ADCC activity against Jurkat cell, and the activity was about 100 times higher in LFA-3-Fc(-). The above results show a possibility that ADCC activity of Fc fusion proteins can be increased by removing fucose of N-acetylglucosamine in the reducing end of the complex type N-glycoside-linked sugar chain of the antibody Fc region.

Reference Example

Preparation of soluble human FcγRIIIa protein

1. Construction of a soluble human FcγRIIIa protein expression vector

(1) Preparation of human peripheral blood monocyte cDNA

From a healthy donor, 30 ml of vein blood was collected and was gently mixed with heparin sodium (manufactured by Takeda Pharmaceutical). From the mixture, the monocyte layer was separated using Lymphoprep (manufactured by Daiichi Pure Chemicals) according to the manufacture's instructions. It was centrifuged with PRMI1640 medium once and PRMI1640-FCS(10) medium once and then $2 \times 10^6$ cells/ml of peripheral blood monocyte suspension suspended in PRMI1640-FBS(10) was prepared. After 5 ml of the peripheral blood monocyte suspension was centrifuged at a room temperature and at 800 rpm for 5 minutes, the supernatant was discarded and the residue was suspended in 5 ml of PBS. After centrifugation at a room temperature and at 800 rpm for 5 minutes, the supernatant was discarded and total RNA was extracted by QIAamp RNA Blood Mini Kit (manufactured by QIAGEN) in accordance with the attached manufacture's instructions.

A single-stranded cDNA was synthesized by reverse transcription reaction to 2 μg of the obtained total RNA, using oligo(dT) as primers and using SUPERSCRITP™ Preamplification System for First Strand cDNA Synthesis (manufactured by Life Technologies) according to the attached manufacture's instructions.

(2) Obtaining of cDNA encoding human FcγRIIIa protein

A cDNA of a human FcγRIIIa protein (hereinafter referred to as "hFcγRIIIa") was prepared as follows.
First, a specific forward primer containing a translation initiation codon (represented by SEQ ID NO:44) and a specific reverse primer containing a translation termination codon (represented by SEQ ID NO:45) were designed from the nucleotide sequence of hFcγRIIIa cDNA [*J. Exp. Med.,* 170, 481 (1989)].
Next, using a DNA polymerase ExTaq (manufactured by Takara Shuzo Co., Ltd.), 50 μl of a reaction solution [1x concentration ExTaq buffer (manufactured by Takara Shuzo Co., Ltd.), 0.2 mM dNTPs, 1 μM of the above gene-specific primers (SEQ ID NOs:44 and 45)] containing 5 μl of 20-fold diluted solution of the human peripheral blood monocyte-derived cDNA solution prepared in the item (1) was prepared, and PCR was carried out. The PCR was carried out by 35 cycles, one cycle consisting of a reaction at 94°C for 30 seconds, at 56°C for 30 seconds and at 72°C for 60 seconds.
After the PCR, the reaction solution was purified by using QIAquick PCR Purification Kit (manufactured by QIAGEN) and dissolved in 20 μl of sterile water. The products were digested with restriction enzymes *Eco*RI (manufactured by Takara Shuzo Co., Ltd.) and *Bam*HI (manufactured by Takara Shuzo Co., Ltd.) and subjected to agarose gel electrophoresis to recover about 800 bp of a PCR-derived fragment.
In the meantime, 2.5 μg of a plasmid pBluescript II SK(-) (manufactured by Stratagene) was digested with restriction enzymes *Eco*RI (manufactured by Takara Shuzo Co., Ltd.) and *Bam*HI (manufactured by Takara Shuzo Co., Ltd.), and digested products were subjected to agarose gel electrophoresis to recover a fragment of about 2.9 kbp.

The human peripheral blood monocyte cDNA-derived fragment of about 800 bp of and the plasmid pBluescript II SK(-)-derived fragment of about 2.9 kbp obtained in the above were ligated by using DNA Ligation Kit Ver. 2.0 (manufactured by Takara Shuzo Co., Ltd.). The *Escherichia coli* strain DH5α (manufactured by TOYOBO) was transformed by using the reaction solution, each plasmid DNA was prepared from the resulting transformant clones, the reaction was carried out by using BigDye Terminator Cycle Sequencing FS Ready Reaction Kit (manufactured by Applied Biosystems) according to the attached manufacture's instructions, and then the nucleotide sequence of the cDNA inserted into each plasmid was determined by using a DNA Sequencer of the same company, ABI PRISM 377. It was confirmed that all of the inserted cDNAs whose sequences were determined by this method encodes a full ORF sequence of the cDNA of hFcγRIIIa. As a result, cDNAs encoding two types of hFcγRIIIa were obtained. One is a sequence represented by SEQ ID NO:46, and pBSFcγRIIIa5-3 was obtained as a plasmid containing the sequence. The amino acid sequence corresponding to the nucleotide sequence represented by SEQ ID NO:46 is represented by SEQ ID NO:47. Another is a sequence represented by SEQ ID NO:48, and pBSFcγRIII a5-3 was obtained as a plasmid containing the sequence. The amino acid sequence corresponding to the nucleotide sequence represented by SEQ ID NO:48 is represented by SEQ ID NO:49. The difference between nucleotide sequences represented by SEQ ID NO:46 and SEQ ID NO:48 is that nucleotide at position 538 shows T and G, respectively. As a result, in the corresponding amino acid sequences, the position 176 in the sequence is Phe and Val, respectively. Herein, hFcγRIIIa of the amino acid sequence represented by SEQ ID NO:47 is named hFcγRIIIa(F), and hFcγRIIIa of the amino acid sequence represented by SEQ ID NO:49 is named hFcγRIIIa(V).

(3) Obtaining of a cDNA encoding soluble hFcγRIIIa(F)

A cDNA encoding soluble hFcγRIIIa(F) having the extracellular region of hFcγRIIIa(F) (positions 1 to 193 in SEQ ID NO:47) and a His-tag sequence at the C-terminal (hereinafter referred to as "shFcγRIIIa(F)") was constructed as follows.

First, a primer FcgR3-1 (represented by SEQ ID NO:50) specific for the extracellular region was designed from the nucleotide sequence of cDNA of hFcγRIIIa(F) represented by SEQ ID NO:46.

Next, using a DNA polymerase ExTaq (manufactured by Takara Shuzo Co., Ltd.), 50 μl of a reaction solution [1x concentration ExTaq buffer (manufactured by Takara Shuzo Co., Ltd.), 0.2 mM dNTPs, 1 μM of the primer FcgR3-1, 1 μM of the primer M13M4 (manufactured by Takara Shuzo Co., Ltd.)] containing the plasmid pBSFcγRIIIa5-3 prepared in the item (2), as the template, of was prepared, and PCR was carried out. The PCR was carried out by 35 cycles, one cycle consisting of a reaction at 94°C for 30 seconds, at 56°C for 30 seconds and at 72°C for 60 seconds as one cycle. After the PCR, the reaction solution was purified by using QIAquick PCR Purification Kit (manufactured by QIAGEN) and dissolved in 20 μl of sterile water. The products were digested with restriction enzymes *Pst*I (manufactured by Takara Shuzo Co., Ltd.) and *Bam*HI (manufactured by Takara Shuzo Co., Ltd.) and subjected to agarose gel electrophoresis to recover about 110 bp of a specific amplification fragment.

On the other hand, the plasmid pBSFcγRIIIa5-3 was digested with restriction enzymes *Pst*I (manufactured by Takara Shuzo Co., Ltd.) and *Bam*HI (manufactured by Takara Shuzo Co., Ltd.), and the digested products were subjected to agarose gel electrophoresis to recover a fragment of about 3.5 kbp.

The hFcγRIIIa(F) cDNA-derived specific amplification fragment of about 110 bp and the plasmid pBSFcγRIIIa5-3-derived fragment of about 3.5 kbp obtained in the above were ligated by using DNA Ligation Kit Ver. 2.0 (manufactured by Takara Shuzo Co., Ltd.). The *Escherichia coli* strain DH5α (manufactured by TOYOBO) was transformed by using the reaction solution. Each plasmid DNA was prepared from the resulting transformant clones, the reaction was carried out by using BigDye Terminator Cycle Sequencing FS Ready Reaction Kit (manufactured by Parkin Elmer) according to the attached manufacture's instructions, and the nucleotide sequence of the cDNA inserted into each plasmid was analyzed by using DNA Sequencer 377 (manufactured by Parkin Elmer) to thereby confirm that pBSFcγRIIIa(F)+His3 was obtained.

The thus determined full length cDNA sequence for shFcγRIIIa(F) is represented by SEQ ID NO:51, and its corresponding amino acid sequence containing a signal sequence is represented by SEQ ID NO:52. In SEQ ID NO:52, the amino acid residue at position 176 from the N-terminal methionine was phenylalanine.

(4) Obtaining of a cDNA encoding soluble hFcγRIIIa(V)

A cDNA encoding soluble hFcγRIIIa(V) having the extracellular region of hFcγRIIIa(V) (positions 1 to 193 in SEQ ID NO:49) and a His-tag sequence at the C-terminal [hereinafter referred to as "shFcγRIIIa(V)"] was constructed as follows.

After digesting the plasmid pBSFcγRIIIa3 obtained in the item (2) with a restriction enzyme *Alw*NI (manufactured by New England Biolabs), the digested product was subjected to agarose gel electrophoresis to recover a fragment of about 2.7 kbp containing the 5'-terminal side of hFcγRIIIa(V).

After digesting the plasmid pBSFcγRIIIa+His3 obtained in the item (3) with a restriction enzyme *Alw*NI (manufactured by New England Biolabs), the digested product was subjected to agarose gel electrophoresis to recover a fragment of about 920 bp containing the 3'-terminal side of hFcγRIIIa and His-tag sequence.

The plasmid pBSFcγRIIIa3-derived fragment of about 2.7 kbp and the plasmid pBSFcγRIIIa(F)+His3-derived fragment of about 920 bp obtained in the above were ligated by using DNA Ligation Kit Ver. 2.0 (manufactured by Takara Shuzo Co., Ltd.). The *Escherichia coli* strain DH5α (manufactured by TOYOBO) was transformed by using the reaction solution. Each plasmid DNA was prepared from the resulting transformant clones, the reaction was carried out by using BigDye Terminator Cycle Sequencing FS Ready Reaction Kit (manufactured by Parkin Elmer) according to the attached manufacture's instructions, and the nucleotide sequence of the cDNA inserted into each plasmid was analyzed by using DNA Sequencer 377 (manufactured by Parkin Elmer) to thereby confirm that pBSFcγRIIIa+His2 was obtained.

The thus determined full length cDNA sequence for shFcγRIIIa(F) is represented by SEQ ID NO:53, and its corresponding amino acid sequence containing a signal sequence is represented by SEQ ID NO:54. In SEQ ID NO:54, the amino acid residue at position 176 from the N-terminal methionine was valine.

(5) Construction of shFcγRIIIa(F) and shFcγRIIIa(V) expression vector

shFcγRIIIa(F) or shFcγRIIIa(V) expression vector was constructed as follows.

After each of the plasmids pBSFcγRIIIa+His3 and pBSFcγRIIIa+His2 obtained in the items (3) and (4) was digested with restriction enzymes *Eco*RI (manufactured by Takara Shuzo Co., Ltd.) and *Bam*HI (manufactured by Takara Shuzo Co., Ltd.), the digested products were subjected to agarose gel electrophoresis to recover each of fragments of about 620 bp.

On the other hand, the plasmid pKANTEX93 was digested with restriction enzymes *Eco*RI (manufactured by Takara Shuzo Co., Ltd.) and *Bam*HI (manufactured by Takara Shuzo Co., Ltd.), and digested products were subjected to agarose gel electrophoresis to recover a fragment of about 10.7 kbp.

Each of about 620 bp of the fragments containing shFcγRIIIa(F) cDNA and shFcγRIIIa(V) cDNA obtained above was ligated with about 10.7 kbp of the plasmid pKANTEX93-derived fragment by using DNA Ligation Kit Ver. 2.0 (manufactured by Takara Shuzo Co., Ltd.). The *Escherichia coli* strain DH5α (manufactured by TOYOBO) was transformed by using the reaction solution. Each plasmid DNA was prepared from each of the resulting transformant clones, the reaction was carried out by using BigDye Terminator Cycle Sequencing FS Ready Reaction Kit (manufactured by Parkin Elmer) in accordance with the manufacture's instructions, and the nucleotide sequence of the cDNA inserted into each plasmid was analyzed by using DNA Sequencer 377 (manufactured by Parkin Elmer) to confirm that the desired expression vector pKANTEXFcγRIIIa(F)-His containing the shFcγRIII(F) cDNA and the expression vector pKANTEXFcγRIIIa(V)-His containing the shFcγRIII(V) cDNA were obtained.

2. Preparation of cell stably producing shFcγRIIIa

Two kinds of cells stably producing shFcγRIIIa were prepared by introducing the shFcγRIIIa expression vector pKANTEXFcγRIIIa(F)-His or pKANTEXFcγRIIIa(V)-His constructed in the item 1 of this Reference Example into rat myeloma YB2/0 cell [ATCC CRL-1662, *J. Cell. Biol.,* 93, 576 (1982)],

pKANTEXFcγRIIIa-His was digested with a restriction enzyme *Aat*II to obtain a linear fragment, 10 μg of each thereof was introduced into $4 \times 10^6$ YB2/0 cells by electroporation [*Cytotechnology*, 3, 133 (1990)], and the resulting cells were suspended in 40 ml of Hybridoma-SFM-FBS(10) [Hybridoma-SFM medium containing 10% FBS (manufactured by Life Technology)] and dispensed at 200 μl/well into a 96-well culture plate (manufactured by Sumitomo Bakelite). After culturing at 37°C for 24 hours in a 5% $CO_2$ incubator, G418 was added to give a concentration of 1.0 mg/ml, followed by culturing for 1 to 2 weeks. Culture supernatants were recovered from wells in which colonies of transformants showing G418 resistance were formed and their growth was confirmed, and the expression amount of shFcγRIIIa in the supernatants was measured by the ELISA described in the item 5 of this Reference Example.

Regarding the transformants in wells in which expression of the shFcγRIIIa was confirmed in the culture supernatants, in order to increase the production amount of the shFcγRIIIa by using a *dhfr* gene amplification system, each of them was suspended in the Hybridoma-SFM-FBS(10) medium containing 1.0 mg/ml G418 and 50 nmol/l DHFR inhibitor MTX (manufactured by SIGMA) to give a density of 1 to $2 \times 10^5$ cells/ml and dispensed at 2 ml into each well of a 24 well plate (manufactured by Greiner). After culturing at 37°C for 1 to 2 weeks in a 5% $CO_2$ incubator, transformants showing 50 nM MTX resistance were induced. An expression amount of shFcγRIIIa in culture supernatants in wells where growth of transformants was observed was measured by the ELISA described in the item 5 of this Reference Example. Regarding the transformants in wells in which expression of the shFcγRIIIa was found in culture supernatants, the MTX concentration was increased to 100 nM and then to 200 nM sequentially by a method similar to the above to thereby finally obtain a transformant capable of growing in the Hybridoma-SFM-FBS(10) medium containing 1.0 mg/ml G418 and 200 nM MTX and also of highly producing shFcγRIIIa. Regarding the obtained transformants, cloning was carried out twice by limiting dilution. shFcγRIIIa(F)-producing transformant clone KC1107 and shFcγRIIIa(V)-producing transformant clone KC1111 were obtained.

4. Purification of shFcγRIIIa

The shFcγRIIIa(F)-producing transformant clone KC1107 and shFcγRIIIa-producing transformant clone KC1111 obtained in the item 2 of this Reference Example was suspended in Hybridoma-SFM-GF(5) [Hybridoma-SFM medium (manufactured by LIFE TECHNOLOGIES) containing 5% Daigo's GF21 (manufactured by Wako Pure Chemical Industries)] containing 1.0 mg/mL of G418 and 200 nmol/L of MTX to give a density of $3 \times 10^5$ cells/ml and dispensed at 50 ml into 182 cm$^2$ flasks (manufactured by Greiner). After culturing at 37°C for 4 days in a 5% CO$_2$ incubator, the culture supernatants were recovered. shFcγRIIIa(F) and shFcγRIIIa(V) were purified from the culture supernatants by using Ni-NTA agarose (manufactured by QIAGEN) column according to the attached manufacture's instructions.

5. Detection of shFcγRIIIa(F) and shFcγRIIIa(V) (ELISA)

shFcγRIIIa(F) and shFcγRIIIa(V) in culture supernatants or purified shFcγRIIIa(F) and shFcγRIIIa(V) were detected or determined by the ELISA shown below.

A solution of a mouse antibody against His-tag, Tetra·His Antibody (manufactured by QIAGEN), which was adjusted to 5 μg/ml with PBS, was dispensed at 50 μl/well into each well of a 96-well plate for ELISA (manufactured by Greiner) and incubated at 4°C for 12 hours or more. After the incubation, 1% BSA-PBS was added at 100 μl/well and incubated at a room temperature for 1 hour to block the remaining active groups. After 1% BSA-PBS was discarded, culture supernatant of the transformant or each of various diluted solutions of purified shFcγRIIIa(F) or shFcγRIIIa(V) was added at 50 μl/well and incubated at a room temperature for 1 hour. After the reaction and subsequent washing of each well with Tween-PBS, a biotin-labeled mouse anti-human CD16 antibody solution (manufactured by PharMingen) diluted 50-fold with 1% BSA-PBS was added at 50 μl/well and incubated at a room temperature for 1 hour. After the incubation and subsequent washing with Tween-PBS, a peroxidase-labeled Avidin D solution (manufactured by Vector) diluted 4,000-fold with 1% BSA-PBS was added at 50 μl/well and incubated at a room temperature for 1 hour. After the incubation and subsequent washing with Tween-PBS, the ABTS substrate solution was added at 50 μl/well to develop color, and 5 minutes thereafter, the reaction was stopped by adding 5% SDS solution at 50 μl/well. Thereafter, OD415 was measured.

Free Text of Sequence Listing

SEQ ID NO: 17 - Explanation of artificial sequence: Amino acid sequence of single stranded antibody
SEQ ID NO:18 - Explanation of artificial sequence: Synthetic DNA
SEQ ID NO:19 - Explanation of artificial sequence: Synthetic DNA
SEQ ID NO:20 - Explanation of artificial sequence: Synthetic DNA
SEQ ID NO:21 - Explanation of artificial sequence: Synthetic DNA
SEQ ID NO:22 - Explanation of artificial sequence: Synthetic DNA
SEQ ID NO:23 - Explanation of artificial sequence: Synthetic DNA
SEQ ID NO:24 - Explanation of artificial sequence: Synthetic DNA
SEQ ID NO:25 - Explanation of artificial sequence: Synthetic DNA
SEQ ID NO:26 - Explanation of artificial sequence: Synthetic DNA
SEQ ID NO:27 - Explanation of artificial sequence: Synthetic DNA
SEQ ID NO:28 - Explanation of artificial sequence: Synthetic DNA
SEQ ID NO:29 - Explanation of artificial sequence: Synthetic DNA
SEQ ID NO:30 - Explanation of artificial sequence: Synthetic DNA
SEQ ID NO:31 - Explanation of artificial sequence: Synthetic DNA
SEQ ID NO:32 - Explanation of artificial sequence: Synthetic DNA
SEQ ID NO:33 - Explanation of artificial sequence: Synthetic DNA
SEQ ID NO:34 - Explanation of artificial sequence: Synthetic DNA
SEQ ID NO:36 - Explanation of artificial sequence: Synthetic DNA
SEQ ID NO:37 - Explanation of artificial sequence: Synthetic DNA
SEQ ID NO:38 - Explanation of artificial sequence: Synthetic DNA

SEQ ID NO:39 - Explanation of artificial sequence: Synthetic DNA
SEQ ID NO:40 - Explanation of artificial sequence: Synthetic DNA
SEQ ID NO:41 - Explanation of artificial sequence: Synthetic DNA
SEQ ID NO:42 - Explanation of artificial sequence: Synthetic DNA
SEQ ID NO:43 - Explanation of artificial sequence: Synthetic DNA
SEQ ID NO:44 - Explanation of artificial sequence: Synthetic DNA
SEQ ID NO:45 - Explanation of artificial sequence: Synthetic DNA
SEQ ID NO:48 - Explanation of artificial sequence: Synthetic DNA
SEQ ID NO:49 - Explanation of artificial sequence: Synthetic DNA
SEQ ID NO:50 - Explanation of artificial sequence: Synthetic DNA
SEQ ID NO:56 - Explanation of artificial sequence: Synthetic DNA
SEQ ID NO:57 - Explanation of artificial sequence: Synthetic DNA
SEQ ID NO:58 - Explanation of artificial sequence: Synthetic DNA
SEQ ID NO:59 - Explanation of artificial sequence: Synthetic DNA
SEQ ID NO:60 - Explanation of artificial sequence: Synthetic DNA
SEQ ID NO:61 - Explanation of artificial sequence: Synthetic DNA
SEQ ID NO:62 - Explanation of artificial sequence: Synthetic DNA
SEQ ID NO:63 - Explanation of artificial sequence: Synthetic DNA
SEQ ID NO:74 - Explanation of artificial sequence: Amino acid sequence of single stranded antibody
SEQ ID NO:75 - Explanation of artificial sequence: Amino acid sequence of bispecific single stranded antibody
SEQ ID NO:76 - Explanation of artificial sequence: Amino acid sequence of bispecific single stranded antibody
SEQ ID NO:77 - Explanation of artificial sequence: Synthetic DNA
SEQ ID NO:78 - Explanation of artificial sequence: Synthetic DNA
SEQ ID NO:79 - Explanation of artificial sequence: Synthetic DNA
SEQ ID NO:81 - Explanation of artificial sequence: Synthetic DNA
SEQ ID NO:82 - Explanation of artificial sequence: Synthetic DNA
SEQ ID NO:83 - Explanation of artificial sequence: Synthetic DNA
SEQ ID NO:84 - Explanation of artificial sequence: Synthetic DNA
SEQ ID NO:85 - Explanation of artificial sequence: Synthetic DNA
SEQ ID NO:86 - Explanation of artificial sequence: Synthetic DNA
SEQ ID NO:87 - Explanation of artificial sequence: Synthetic DNA
SEQ ID NO:88 - Explanation of artificial sequence: Synthetic DNA
SEQ ID NO:89 - Explanation of artificial sequence: Synthetic DNA
SEQ ID NO:90 - Explanation of artificial sequence: Synthetic DNA
SEQ ID NO:91 - Explanation of artificial sequence: Synthetic DNA
SEQ ID NO:92 - Explanation of artificial sequence: Synthetic DNA
SEQ ID NO:93 - Explanation of artificial sequence: Synthetic DNA
SEQ ID NO:94 - Explanation of artificial sequence: Synthetic DNA
SEQ ID NO:95 - Explanation of artificial sequence: Synthetic DNA
SEQ ID NO:96 - Explanation of artificial sequence: Synthetic DNA
SEQ ID NO:97 - Explanation of artificial sequence: Synthetic DNA
SEQ ID NO:98 - Explanation of artificial sequence: Synthetic DNA
SEQ ID NO:99 - Explanation of artificial sequence: Synthetic DNA
SEQ ID NO:100 - Explanation of artificial sequence: Synthetic DNA
SEQ ID NO:101 - Explanation of artificial sequence: Synthetic DNA
SEQ ID NO:102 - Explanation of artificial sequence: Synthetic DNA
SEQ ID NO:103 - Explanation of artificial sequence: Synthetic DNA
SEQ ID NO:104 - Explanation of artificial sequence: Synthetic DNA
SEQ ID NO:105 - Explanation of artificial sequence: Synthetic DNA
SEQ ID NO:106 - Explanation of artificial sequence: Synthetic DNA
SEQ ID NO:107 - Explanation of artificial sequence: Synthetic DNA

**Claims**

1. A pharmaceutical fusion protein composition comprising a fusion protein molecule of a binding protein and an antibody Fc region having complex type N-glycoside-linked sugar chains, wherein the complex type N-glycoside-linked sugar chains have a structure in which fucose is not bound to N-acetylglucosamine in the reducing end in the sugar chains.

2. The fusion protein composition according to claim 1, wherein the complex type N-glycoside-linked sugar chains are sugar chains in which 1-position of fucose is not bound to 6-position of N-acetylglucosamine in the reducing end through α-bond in the sugar chains.

3. The fusion protein composition according to claim 1 or 2, wherein the antibody Fc region is an IgG class of a human antibody.

4. The fusion protein composition according to claim 3, wherein the antibody Fc region is an IgG1 class of a human antibody.

5. The fusion protein composition according to claim 4, wherein the antibody fusion protein composition comprises an IgG1 class heavy chain constant region domain 2 (CH$_2$) of a human antibody.

6. The fusion protein composition according to claim 5, wherein the fusion protein composition comprises a hinge region, a heavy chain constant region domain 2 (CH$_2$) and a heavy chain constant region domain 3 (CH$_3$) of a human IgG1 class antibody.

7. The fusion protein composition according to any one of claims 1 to 6, wherein the binding protein comprises at least one protein selected from the group consisting of a binding fragment of an antibody, a soluble receptor and a ligand protein.

8. The fusion protein composition according to claim 7, wherein the binding fragment of an antibody comprises at least one polypeptide comprising an antibody heavy chain variable region (VH) and an antibody light chain variable region (VL).

9. The fusion protein composition according to claim 8, wherein the polypeptide comprising an antibody heavy chain variable region (VH) and an antibody light chain variable region (VL) is a single-chain antibody.

10. The fusion protein composition according to claim 7, wherein the binding fragment of an antibody is a single-chain antibody.

11. The fusion protein composition according to claim 7, wherein the binding fragment of an antibody comprises a polypeptide comprising two kinds of antibody heavy chain variable regions (VH) and two kinds of antibody light chain variable regions (VL).

12. The fusion protein composition according to claim 11, wherein the polypeptide comprising antibody heavy chain variable regions (VH) and light chain variable regions (VL) is a single-chain antibody.

13. The fusion protein composition according to claim 7, wherein the binding fragment of an antibody is a bispecific single-chain antibody.

14. The fusion protein composition according to claim 7, wherein the soluble receptor is a soluble TNF (tumor necrosis factor) receptor II.

15. The fusion protein composition according to claim 15, wherein the soluble receptor comprises the amino acid sequence represented by SEQ ID NO:64.

16. The fusion protein composition according to claim 14 or 15, wherein the fusion protein is produced by FERM BP-8499.

17. The fusion protein composition according to claim 7, wherein the ligand protein is LFA-3 (leukocyte function antigen-3).

18. The fusion protein composition according to claim 16, wherein the ligand protein comprises the amino acid sequence represented by SEQ ID NO:65.

19. The fusion protein composition according to claim 17 or 18, wherein the fusion protein is produced by FERM BP-8500.

**20.** The fusion protein composition according to any one of claims 1 to 19, wherein the fusion protein composition is a dimer.

**21.** A transformant obtainable by introducing a DNA encoding the fusion protein according to any one of claims 1 to 20 into a host cell.

**22.** The transformant according to claim 21, wherein the host cell is a cell in which a genome is modified so that an enzyme relating to synthesis of an intracellular sugar nucleotide, GDP-fucose or an enzyme relating to a modification of a sugar chain in which 1-position of fucose is bound to 6-position of N-acetylglucosamine in the reducing end through $\alpha$-bond in the complex type N-glycoside-linked sugar chain is inactivated.

**23.** The transformant according to claim 22, wherein the host cell is a cell in which all of alleles on a genome encoding an enzyme relating to synthesis of an intracellular sugar nucleotide, GDP-fucose or an enzyme relating to a modification of a sugar chain in which 1-position of fucose is bound to 6-position of N-acetylglucosamine in the reducing end through $\alpha$-bond in the complex type N-glycoside-linked sugar chain are knocked out.

**24.** The transformant according to claim 22 or 23, wherein the enzyme relating to synthesis of an intracellular sugar nucleotide, GDP-fucose, is an enzyme selected from the group consisting of GDP-mannose 4,6-dehydratase (GMD) and GDP-4-keto-6-deoxy-D-mannose 3,5-epimerase (Fx).

**25.** The transformant according to claim 24, wherein the GDP-mannose 4,6-dehydratase is a protein encoded by a DNA selected from the following (a) or (b):

(a) a DNA comprising the nucleotide sequence represented by SEQ ID NO:1;
(b) a DNA which hybridizes with a DNA consisting of the nucleotide sequence represented by SEQ ID NO:1 under stringent conditions and which encodes a protein having GDP-mannose 4,6-dehydratase activity.

**26.** The transformant according to claim 24, wherein the GDP-mannose 4,6-dehydratase is a protein selected from the group consisting of the following (a), (b) and (c):

(a) a protein comprising the amino acid sequence represented by SEQ ID NO:2;
(b) a protein consisting of an amino acid sequence wherein one or more amino acid(s) is/are deleted, substituted, inserted and/or added in the amino acid sequence represented by SEQ ID NO:2 and having GDP-mannose 4,6-dehydtratase activity;
(c) a protein consisting of an amino acid sequence which has 80% or more homology to the amino acid sequence represented by SEQ ID NO:2 and having GDP-mannose 4,6-dehydratase activity.

**27.** The transformant according to claim 24, wherein the GDP-4-keto-6-deoxy-D-mannose 3,5-epimerase is a protein encoded by a DNA selected from the following (a) or (b):

(a) a DNA comprising the nucleotide sequence represented by SEQ ID NO:3;
(b) a DNA which hybridizes with a DNA consisting of the nucleotide sequence represented by SEQ ID NO:3 under stringent conditions and which encodes a protein having GDP-4-keto-6-deoxy-D-mannose 3,5-epimerase activity.

**28.** The transformant according to claim 24, wherein the GDP-4-keto-6-deoxy-D-mannose 3,5-epimerase activity is a protein selected from the group consisting of the following (a) to (c):

(a) a protein comprising the amino acid sequence represented by SEQ ID NO:4;
(b) a protein consisting of an amino acid sequence wherein one or more amino acid(s) is/are deleted, substituted, inserted and/or added in the amino acid sequence represented by SEQ ID NO:4 and having GDP-4-keto-6-deoxy-D-mannose 3,5-epimerase activity;
(c) a protein consisting of an amino acid sequence which has 80% or more homology to the amino acid sequence represented by SEQ ID NO:4 and having G GDP-4-keto-6-deoxy-D-mannose 3,5-epimerase activity.

**29.** The transformant according to claim 22 or 23, wherein the enzyme relating to a modification of a sugar chain in which 1-position of fucose is bound to 6-position of N-acetylglucosamine in the reducing end through $\alpha$-bond in the complex type N-glycoside-linked sugar chain is $\alpha$1,6-fucosyltransferase.

**30.** The transformant according to claim 29, wherein the α1,6-fucosyltransferase is a protein encoded by a DNA selected from the group consisting of the following (a) to (d):

(a) a DNA comprising the nucleotide sequence represented by SEQ ID NO:5;
(b) a DNA comprising the nucleotide sequence represented by SEQ ID NO:6;
(c) a DNA which hybridizes with a DNA consisting of the nucleotide sequence represented by SEQ ID NO:5 under stringent conditions and which encodes a protein having α1,6-fucosyltransferase activity;
(d) a DNA which hybridizes with a DNA consisting of the nucleotide sequence represented by SEQ ID NO:6 under stringent conditions and which encodes a protein having α1,6-fucosyltransferase activity.

**31.** The transformant according to claim 29, wherein the α1,6-fucosyltransferase is a protein selected from the group consisting of the following (a) to (f):

(a) a protein comprising the amino acid sequence represented by SEQ ID NO:7;
(b) a protein comprising the amino acid sequence represented by SEQ ID NO:8;
(c) a protein consisting of an amino acid sequence wherein one or more amino acid(s) is/are deleted, substituted, inserted and/or added in the amino acid sequence represented by SEQ ID NO:7 and having α1,6-fucosyltransferase activity;
(d) a protein consisting of an amino acid sequence wherein one or more amino acid(s) is/are deleted, substituted, inserted and/or added in the amino acid sequence represented by SEQ ID NO:8 and having α1,6-fucosyltransferase activity;
(e) a protein consisting of an amino acid sequence which has 80% or more homology to the amino acid sequence represented by SEQ ID NO:7 and having α1,6-fucosyltransferase activity;
(f) a protein consisting of an amino acid sequence which has 80% or more homology to the amino acid sequence represented by SEQ ID NO:8 and having α1,6-fucosyltransferase activity.

**32.** The transformant according to any one of claims 21 to 31, wherein the host cell is a cell selected from the group consisting of the following (a) to (h):

(a) a CHO cell derived from Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-Ag14 cell;
(e) a BHK cell derived from Syrian hamster kidney tissue;
(f) a human leukemia cell line Namalwa cell;
(g) an embryonic stem cell;
(h) a fertilized egg cell.

**33.** The transformant according to any one of claims 21 to 32, wherein the transformant is FERM BP-8499.

**34.** The transformant according to any one of claims 21 to 32, wherein the transformant is FERM BP-8500.

**35.** A process for producing the fusion protein composition according to any one of claims 1 to 20, which comprises culturing the transformant according to any one of claims 21 to 34 in a medium to form and accumulate the fusion protein composition in the culture, and recovering and purifying the antibody composition from the culture.

**36.** The antibody fusion protein composition according to any one of claims 1 to 20, which is obtainable by the process according to claim 35.

**37.** A medicament comprising the fusion protein composition according to any one of claims 1 to 20 and 36 as an active ingredient.

**38.** An agent for preventing or treating tumor, inflammatory diseases or autoimmune diseases, comprising the fusion protein composition as an active ingredient according to any one of claims 1 to 20 and 36.

**39.** The agent for preventing or treating the diseases according to claim 38, wherein the tumor is blood tumor or cancer.

**INTERNATIONAL SEARCH REPORT**

| | International application No. |
|---|---|
| | PCT/JP2004/015325 |

**A. CLASSIFICATION OF SUBJECT MATTER**
Int.Cl⁷ C07K19/00, C17K16/00, C12N15/62, C12N5/10, C12P21/08,
A61K38/17, A61P35/00, A61P37/00, A61P29/00

According to International Patent Classification (IPC) or to both national classification and IPC

**B. FIELDS SEARCHED**

Minimum documentation searched (classification system followed by classification symbols)
Int.Cl⁷ C07K19/00, C17K16/00, C12N15/62, C12N5/10, C12P21/08,
A61K38/17, A61P35/00, A61P37/00, A61P29/00

Documentation searched other than minimum documentation to the extent that such documents are included in the fields searched

Electronic data base consulted during the international search (name of data base and, where practicable, search terms used)
REGISTRY(STN), CA(STN), WPI(DIALOG), BIOSIS(DIALOG), JSTPlus(JOIS),
GenBank/EMBL/DDBJ/GeneSeq, SwissProt/PIR/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 02/31140 A1 (Kyowa Hakko Kogyo Co., Ltd.), 18 April, 2002 (18.04.02), & AU 200194198 A & US 2003/0115614 A1 & EP 1331266 A1 & BR 200114475 A & KR 2003081312 A & MX 2003002974 A1 | 1-39 |
| P,X | YAMANE-OHNUKI, N. et al., Establishment of FUT8 knockout Chinese hamster ovary cells: an ideal host cell line for producing completely defucosylated antibodies with enhanced antibody-dependent cellular cytotoxicity., Biotechnol. Bioeng., 05 September, 2004 (05.09.04), Vol.87, No.5, pages 614 to 622 | 1-39 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 January, 2005 (13.01.05) | 01 February, 2005 (01.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2004/015325 |

C (Continuation).   DOCUMENTS CONSIDERED TO BE RELEVANT

| Category* | Citation of document, with indication, where appropriate, of the relevant passages | Relevant to claim No. |
| --- | --- | --- |
| P,X | WO 03/85107 A1  (Kyowa Hakko Kogyo Co., Ltd.), 16 October, 2003 (16.10.03), & AU 2003236022 A1      & US 2004/0110704 A1 | 1-39 |
| P,X | WO 03/85118 A1  (Kyowa Hakko Kogyo Co., Ltd.), 16 October, 2003 (16.10.03), & AU 2003236015 A1      & US 2004/0132140 A1 | 1-39 |

Form PCT/ISA/210 (continuation of second sheet) (January 2004)